# EUROPEAN PATENT APPLICATION

(11) **EP 3 581 569 A1**
(43) Date of publication of application: **18.12.2019**
(21) Application number: 18751703.2
(22) Date of filing: 07.02.2018
(51) Int. Cl.: C07D 401/14, A61K 31/4545, A61K 31/48, A61K 31/5377, A61K 38/00, A61K 45/00, A61P 1/00, A61P 5/00, A61P 43/00, C07D 405/14

(54) **COMPOUND HAVING SOMATOSTATIN RECEPTOR AGONISTIC ACTIVITY AND PHARMACEUTICAL USE THEREOF**

(30) Priority: 08.02.2017 JP 2017021363
(71) Applicant: ONO Pharmaceutical Co., Ltd., Osaka-shi, Osaka 541-8526 (JP)
(72) Inventor: ISHIDA, Akiharu, Mishima-gun Osaka 618-8585 (JP); YOSHIDA, Atsushi, Mishima-gun Osaka 618-8585 (JP); MIYATA, Hidenori, Mishima-gun Osaka 618-8585 (JP); SHONO, Tomoyuki, Mishima-gun Osaka 618-8585 (JP)
(74) Representative: Grünecker Patent- und Rechtsanwälte PartG mbB
(86) International application number: PCT/JP2018/004111
(87) International publication number: WO 2018/147300

(57) **Abstract**

The present invention provides a somatostatin receptor subtype 2 agonist. The compound disclosed herein represented by the general formula (I): 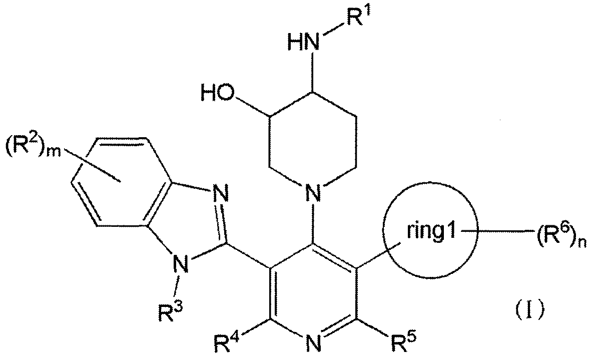 wherein all symbols have the same meanings as the definitions described in the specification;
or a salt thereof is a low-molecular compound having strong agonistic activity for somatostatin receptor subtype 2 and can be orally administered. Therefore, the present compound can be easily administered and can alleviate pain accompanied by the therapy of patients. The present compound also has sufficiently weak hERG inhibitory activity and/or phospholipidosis action relative to the SSTR2 agonistic activity, and thus can suppress or alleviate side effects resulting from the activity/action.

SEQUENCE LISTING

<110> ONO PHARMACEUTICAL CO., LTD.

<120> COMPOUNDS HAVING SOMATOSTATIN RECEPTOR AGONISTIC ACTIVITY AND
PHARMACEUTICAL USE THEREOF

<130> P17-228WO

<150> JP 2017-021363
<151> 2017-02-08

<160> 2

<170> PatentIn Ver. 3.5.1

<210> 1
<211> 34
<212> DNA
<213> Artificial Sequence

<220>
<223> Forward primer hSSTR2_F1_XhoI

<400> 1
caccctcgag gacatggcgg atgagccact caat                                34

<210> 2
<211> 34
<212> DNA
<213> Artificial Sequence

<220>
<223> Reverse primer hSSTR2_R1_EcoRI

<400> 2
ccttgaattc gatactggtt tggaggtctc catt                                34

## Description

### [Technical Field]

The present invention relates to a compound represented by the general formula (I) described hereinbelow having a somatostatin receptor, particularly, somatostatin receptor subtype 2 agonistic activity, or a salt thereof, and pharmaceutical use thereof.

### [Background Art]

Acromegaly is a hormonal disorder resulting from excess secretion of growth hormones from a pituitary gland caused by pituitary adenoma and the like. The affected patients have hypertrophy of heads, bones in hands and feet and soft tissues. The prevalence of acromegaly is about 60 patients per 1 million people, which is not necessarily high. However, the disease impacts the lives of the patients due to aberrations in parts of the body and is a serious disease having an increased risk of mortality because of cardiac diseases which occur in one-third of the patients.

Patients with acromegaly are currently treated by, in addition to surgical excision of adenoma secreting growth hormone and radiotherapy, drug therapy for exogenously administering an analogue of somatostatin, a hormone which suppresses secretion of growth hormone. Somatostatin analogues include octreotide acetate (Sandostatin®) by Novartis Pharmaceuticals and lanreotide acetate (Somatuline®) by Ipsen Pharma SAS, of which usefulness has been recognised and assured. Meanwhile the drugs are peptide drugs and thus require administration by injection, and it is reported that intramuscular injection of the sustained-release formulation thereof once in a few weeks is accompanied by significant pain. In order to solve the problem, it is believed to be the best choice to obtain a non-peptidic, orally administrable low-molecular compound rather than a peptide drug that requires injection.

Meanwhile, it has been revealed that there are 5 somatostatin receptor subtypes, SSTR1 to SSTR5, and it is reported that octreotide acetate and lanreotide acetate bind to somatostatin receptor subtype 2 (SSTR2) with high affinity. It has also been reported that the drugs bind to somatostatin receptor subtype 3 (SSTR3) and somatostatin receptor subtype 5 (SSTR5) with moderate affinity and do not bind to somatostatin receptor subtype 1 (SSTR1) or somatostatin receptor subtype 4 (SSTR4).

As the difference in affinity of octreotide acetate and lanreotide acetate towards the receptor subtypes has been scientifically revealed, a few non-peptidic, low-molecular somatostatin receptor agonists have been synthesised.

For example, PTL 1 discloses that the compound represented by the general formula (A): 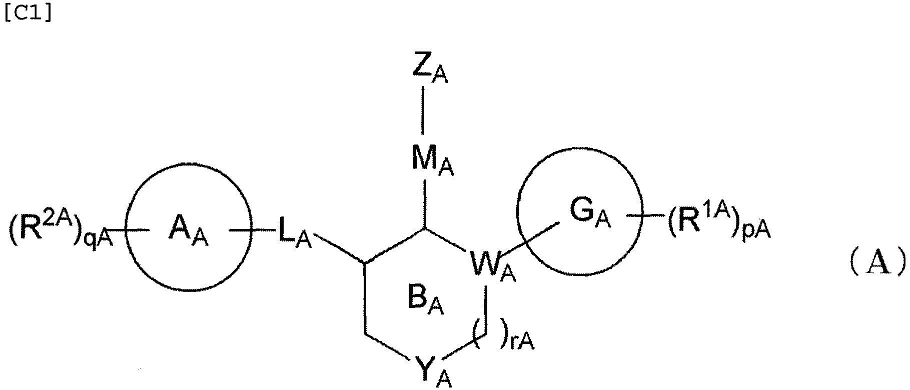
wherein R^{1A} represents (1) a halogen atom, (2) a C1-4 alkyl which may be substituted with a substituent, (3) a C1-4 alkoxy or the like;
pA represents an integer of 0 to 3;
when pA is 2 or more, a plurality of R^{1A} may be the same or different;
R^{2A} represents (1) a halogen atom, (2) -OR^{3A}, (3) -COOR^{5A}, (4) a C1-4 alkyl which may be substituted or the like;
R^{3A} represents a C1-4 alkyl or the like;
R^{5A} represents a C1-4 alkyl or the like;
qA represents an integer of 0 to 3;
when qA is 2 or more, a plurality of R^{2A} may be the same or different;
the ring AA represents a 5- to 10-membered monocyclic or bicyclic heterocycle or the like;
the ring G_{A} represents a benzene, pyridine, pyrimidine, pyrazole, thiazole, furan ring or the like;
WA and Y_{A} respectively and independently represent a nitrogen atom or carbon atom;
rA represents 0 or 1;
the ring B_{A} represents, when at least one of W_{A} and Y_{A} represents a nitrogen atom and rA represents 0 or 1, a pyridine ring or the like;
L_{A} represents a bond or the like;
M_{A} represents a bond or the like;
Z_{A} represents a 5- to 10-membered monocyclic or bicyclic nitrogen-containing heterocycle which may be substituted with a substituent selected from the group consisting of (a) a halogen atom, (b) -NR^{53A}R^{54A}, (c) -OR^{55A}, (d) a C1-4 alkyl which may be substituted with -NR^{56A}R^{57A} and/or -OR^{58A} and (e) an oxo, or the like; and
R^{53A} to R^{58A} respectively and independently represent a hydrogen atom, a C1-4 alkyl, a C1-4 haloalkyl, oxetanyl or the like,
a salt thereof, an N-oxide thereof, a solvate thereof or a prodrug thereof is a SSTR2 selective agonist and is useful for prophylaxis and/or therapy of acromegaly, or a gastrointestinal symptom accompanying gastrointestinal obstruction.

PTL 2 discloses that the compound represented by the general formula (B): 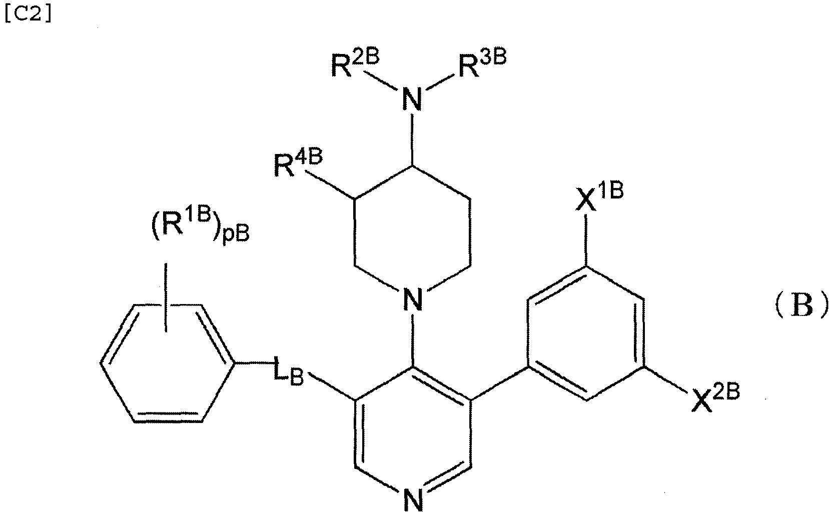
wherein R^{1B} represents (1) a halogen atom, (2) a cyano group, (3) a C1-4 alkyl, (4) a C1-4 alkoxy or the like;
pB represents an integer of 0 to 2;
when pB is 2, a plurality of R^{1B} may be the same or different;
R^{2B} and R^{3B} respectively and independently represent a hydrogen atom or a C1-4 alkyl;
R^{4B} represents a hydrogen atom;
or R^{2B} and R^{4B} together with an atom to which R^{2B} and R^{4B} are bound may form a 5- to 8-membered nitrogen-containing saturated heterocycle;
L_{B} represents (1) a bond, (2) -CR^{AB}=CR^{BB}→, or (3) -C(=O) -NR^{DB}→ (in each group, the arrow indicates the binding site to the pyridine ring);
R^{AB}, R^{BB} and R^{DB} respectively and independently represent a hydrogen atom or a C1-4 alkyl; and
X^{1B} and X^{2B} respectively and independently represent a halogen atom,
a salt thereof, an N-oxide thereof or a solvate thereof or a prodrug of the foregoing is a SSTR2 selective agonist and is useful for prophylaxis and/or therapy of acromegaly, or a gastrointestinal symptom accompanying gastrointestinal obstruction.

However, none of the background art documents disclose the compound described hereinbelow in the present invention or a salt thereof or pharmaceutical use thereof.

### [Citation List]

### [Patent Literature]

[PTL 1] WO 2014/007228
[PTL 2] WO 2015/046482

### [Summary of Invention]

### [Technical Problem]

Medicaments which are taken over a long period of time such as those for therapy of acromegaly are desired to have as little side effects as possible. If a medicament has, in addition to the main effect thereof, hERG inhibitory activity or phospholipidosis action, QT prolongation or severe side effects in organs where the agent accumulates may result. The compound represented by the general formula (A), a salt thereof, an N-oxide thereof, a solvate thereof or a prodrug thereof disclosed in PTL 1 is a non-peptidic low-molecular SSTR2 selective agonist, has hERG inhibitory activity that is dissociated from SSTR2 agonistic activity and has weak phospholipidosis action in vitro. An object of the present invention is to provide a low-molecular compound that has sufficiently low actions that may cause side effects relative to SSTR2 agonistic activity such as having stronger SSTR2 agonistic activity and having hERG inhibitory activity that is more sufficiently dissociated or having further weaker phospholipidosis action.

### [Solution to Problem]

The inventors of the present invention have carried out extensive studies in order to achieve the above object, and as a result, found that the compound represented by the general formula (I) described hereinafter, or a salt thereof can achieve the above object. The inventors have carried out further researches and completed the present invention.

Thus the present invention relates to:
[1] a compound represented by the general formula (I): 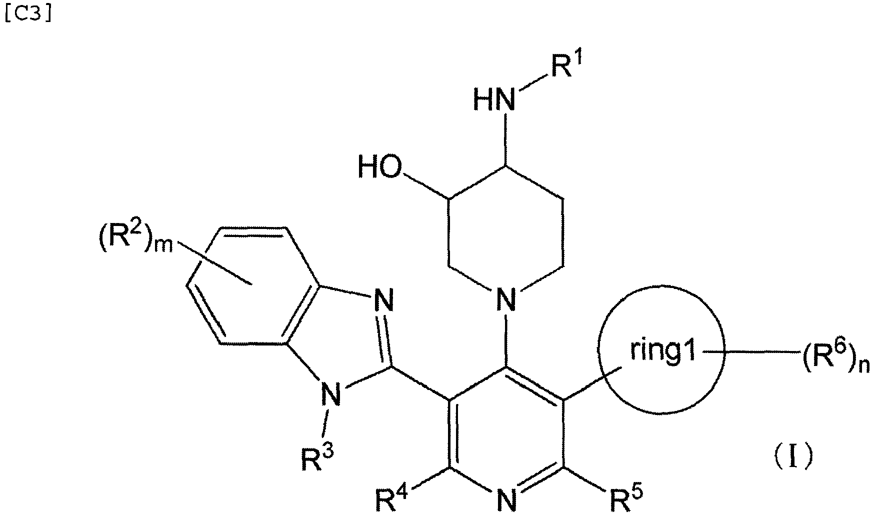 wherein R¹ represents 3-oxetanyl, 3,3,3-trifluoropropyl, ethyl or a hydrogen atom; R² represents a C1-4 alkyl, a C1-4 alkoxy, a halogen, nitrile or a C1-4 alkoxycarbonyl; R³ represents a C1-4 alkyl or a hydrogen atom; R⁴ represents a hydrogen atom, a C1-4 alkyl, a C5-6 monocyclic carbocycle which may be substituted with 1 to 3 R⁷ groups or a 5- to 6-membered monocyclic heterocycle which may be substituted with 1 to 3 R⁸ groups; R⁷ represents a halogen or a C1-4 alkyl; R⁸ represents a halogen or a C1-4 alkyl; when the C5-6 monocyclic carbocycle is substituted with 2 or more R⁷ groups, a plurality of R⁷ may be the same or different; when the 5- to 6-membered monocyclic heterocycle is substituted with 2 or more R⁸ groups , a plurality of R⁸ may be the same or different; R⁵ represents a C1-4 alkyl or a hydrogen atom; ring1 represents a C5-6 monocyclic carbocycle or a 5- to 6-membered monocyclic heterocycle; R⁶ represents a C1-4 alkyl, a C1-4 alkoxy or a halogen; m represents an integer of 0 to 3; n represents an integer of 0 to 3; when m is 2 or more, a plurality of R² may be the same or different; and when n is 2 or more, a plurality of R⁶ may be the same or different, or a salt thereof (excluding rac-(3R,4S)-4-amino-1-[3-(3,5-dimethoxyphenyl)-5-(4,6-dimethyl-1H-benzimidazol-2-yl)-4-pyridinyl]-3-piperidinol, rac-(3R,4S)-4-amino-1-[3-(6-fluoro-1H-benzimidazol-2-yl)-5-(3-fluoro-5-methoxyphenyl)-4-pyridinyl]-3-piperidinol and rac-(3R,4S)-4-amino-1-[3-(6-chloro-1H-benzimidazol-2-yl)-5-(3-fluoro-5-methoxyphenyl)-4-pyridinyl]-3-piperidinol);
[2] the compound according to [1], wherein the compound represented by the general formula (I) is a compound represented by the general formula (IB): 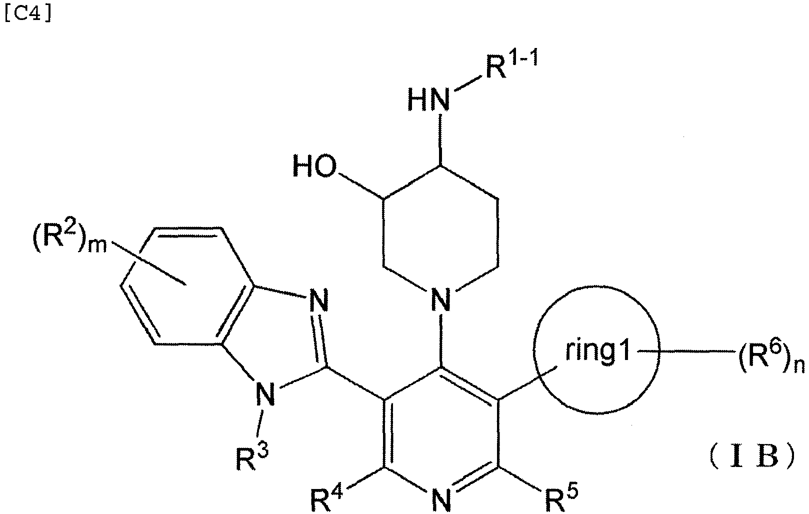 wherein R¹⁻¹ represents 3-oxetanyl, 3,3,3-trifluoropropyl or ethyl; and other symbols have the same meanings as described in [1],
   or a salt thereof;
[3] the compound according to [1] or [2], wherein the compound represented by the general formula (IB) is a compound represented by the general formula (IB-1): 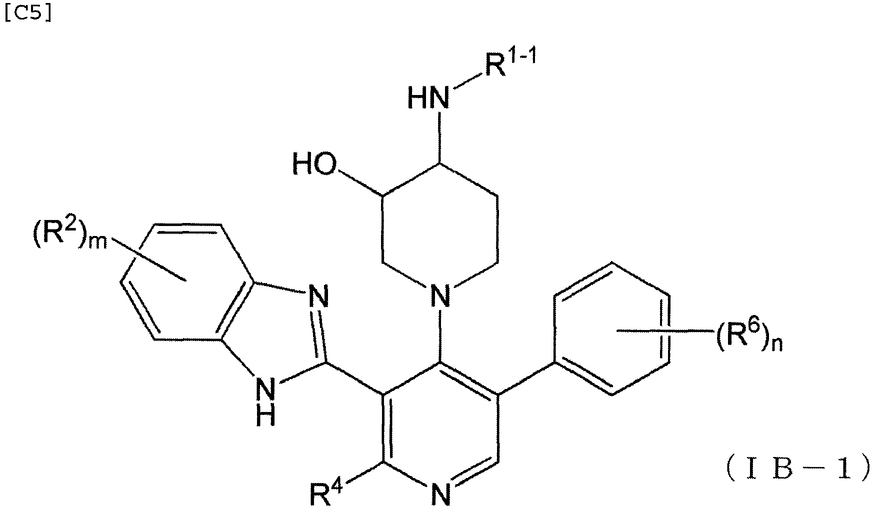 wherein all symbols have the same meanings as described in [1] or [2],
   or a salt thereof;
[4] the compound according to any of [1] to [3], wherein R² is a C1-4 alkoxy, a halogen or a C1-4 alkoxycarbonyl; and R⁶ is a C1-4 alkoxy or a halogen,
   or a salt thereof;
[5] the compound according to any of [1] to [4], wherein R⁶ is a halogen; and n is 2,
   or a salt thereof;
[6] the compound according to [2], wherein the compound is:
   (1) (3S,4R)-1-[3-(5,6-difluoro-1H-benzimidazol-2-yl)-5-(3,5-difluorophenyl)-4-pyridinyl]-4-(3-oxetanylamino)-3-piperidinol;
   (2) (3S,4R)-1-[3-(3-chloro-5-fluorophenyl)-5-(5,6-difluoro-1H-benzimidazol-2-yl)-4-pyridinyl]-4-[(3,3,3-trifluoropropyl)amino]-3-piperidinol;
   (3) (3S,4R)-1-[3-(5,6-difluoro-1H-benzimidazol-2-yl)-5-(2,5-difluorophenyl)-4-pyridinyl]-4-(ethylamino)-3-piperidinol;
   (4) (3S,4R)-1-[3-(5,6-difluoro-1H-benzimidazol-2-yl)-5-(3-fluoro-5-methoxyphenyl)-4-pyridinyl]-4-[(3,3,3-trifluoropropyl)amino]-3-piperidinol; or
   (5) (3S,4R)-1-[3-(3,5-difluorophenyl)-5-(6-fluoro-5-methoxy-1H-benzimidazol-2-yl)-4-pyridinyl]-4-[(3,3,3-trifluoropropyl)amino]-3-piperidinol,
   or a salt thereof;
[7] the compound according to [1], wherein R⁴ is a C1-4 alkyl, a C5-6 monocyclic carbocycle which may be substituted with 1 to 3 R⁷ groups or a 5- to 6-membered monocyclic heterocycle which may be substituted with 1 to 3 R⁸ groups, or a salt thereof;
[8] the compound according to [7], wherein the compound represented by the general formula (I) is a compound represented by the general formula (I-1): 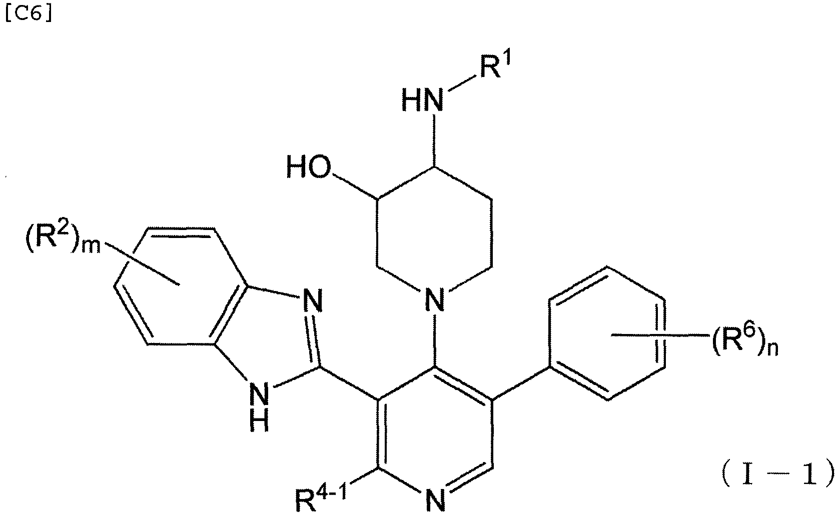 wherein R⁴⁻¹ represents a C1-4 alkyl, a C5-6 monocyclic carbocycle which may be substituted with 1 to 3 R⁷ groups or a 5- to 6-membered monocyclic heterocycle which may be substituted with 1 to 3 R⁸ groups; and other symbols have the same meanings as described in [1],
   or a salt thereof;
[9] the compound according to any of [7] or [8], wherein R² is a C1-4 alkoxy, a halogen or a C1-4 alkoxycarbonyl; and R⁶ is a C1-4 alkoxy or a halogen,
   or a salt thereof;
[10] the compound according to any of [7] to [9], wherein R⁶ is a halogen; and n is 2,
   or a salt thereof;
[11] the compound according to [7], wherein the compound is:
   (1) (3S,4R)-4-amino-1-[3-(5,6-difluoro-1H-benzimidazol-2-yl)-5-(3,5-difluorophenyl)-2-(1-methyl-1H-pyrazol-4-yl)-4-pyridinyl]-3-piperidinol;
   (2) methyl 2-{4-[(3S,4R)-4-amino-3-hydroxy-1-piperidinyl]-5-(3-chloro-5-fluorophenyl)-2-methyl-3-pyridinyl}-5-fluoro-1H-benzimidazole-7-carboxylate;
   (3) methyl 2-{4-[(3S,4R)-4-amino-3-hydroxy-1-piperidinyl]-5-(2,5-difluorophenyl)-2-methyl-3-pyridinyl}-5-fluoro-1H-benzimidazole-7-carboxylate;
   (4) (3S,4R)-4-amino-1-[5-(3,5-difluorophenyl)-2-methyl-3-(5,6,7-trifluoro-1H-benzimidazol-2-yl)-4-pyridinyl]-3-piperidinol;
   (5) (3S,4R)-1-[5-(3-chloro-5-fluorophenyl)-3-(6-methoxy-1H-benzimidazol-2-yl)-2-methyl-4-pyridinyl]-4-(3-oxetanylamino)-3-piperidinol;
   (6) (3S,4R)-1-[5-(3,5-difluorophenyl)-3-(5-fluoro-6-methoxy-1H-benzimidazol-2-yl)-2-methyl-4-pyridinyl]-4-(ethylamino)-3-piperidinol;
   (7) (3S,4R)-1-[5-(3-chloro-5-fluorophenyl)-3-(5,6-difluoro-1H-benzimidazol-2-yl)-2-methyl-4-pyridinyl]-4-(3-oxetanylamino)-3-piperidinol;
   (8) (3S,4R)-1-[3-(5,6-difluoro-1H-benzimidazol-2-yl)-5-(3,5-difluorophenyl)-2-methyl-4-pyridinyl]-4-[(3,3,3-trifluoropropyl)amino]-3-piperidinol;
   (9) (3S,4R)-1-[5-(3,5-difluorophenyl)-2-methyl-3-(5,6,7-trifluoro-1H-benzimidazol-2-yl)-4-pyridinyl]-4-(ethylamino)-3-piperidinol; or
   (10) methyl 2-{4-[(3S,4R)-4-amino-3-hydroxy-1-piperidinyl]-5-(3,5-difluorophenyl)-2-(4-morpholinyl)-3-pyridinyl}-5-fluoro-1H-benzimidazole-7-carboxylate,
   or a salt thereof;
[12] the compound according to [1], wherein R⁴ is a hydrogen atom,
   or a salt thereof;
[13] the compound according to [12], wherein the compound represented by the general formula (I) is a compound represented by the general formula (1-2): 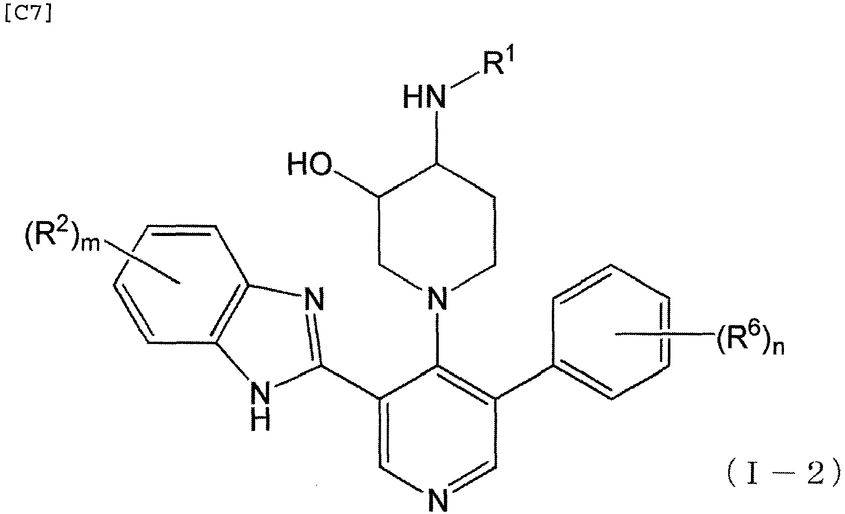 wherein all symbols have the same meanings as described in [1],
   or a salt thereof;
[14] the compound according to [12] or [13], wherein R² is a C1-4 alkoxy, a halogen or a C1-4 alkoxycarbonyl; and R⁶ is a C1-4 alkoxy or a halogen,
   or a salt thereof;
[15] the compound according to any of [12] to [14], wherein R⁶ is a halogen; and n is 2,
   or a salt thereof;
[16] the compound according to [12], wherein the compound is:
   (1) (3S,4R)-4-amino-1-[3-(5,6-difluoro-1H-benzimidazol-2-yl)-5-(3,5-difluorophenyl)-4-pyridinyl]-3-piperidinol;
   (2) (3S,4R)-4-amino-1-[3-(1H-benzimidazol-2-yl)-5-(3,5-difluorophenyl)-4-pyridinyl]-3-piperidinol;
   (3) (3S,4R)-4-amino-1-[3-(2,5-difluorophenyl)-5-(6-methoxy-1H-benzimidazol-2-yl)-4-pyridinyl]-3-piperidinol; or
   (4) methyl 2-{4-[(3S,4R)-4-amino-3-hydroxy-1-piperidinyl]-5-(2,5-difluorophenyl)-3-pyridinyl}-5-fluoro-1H-benzimidazole-7-carboxylate,
   or a salt thereof;
[17] the compound according to [1] or a salt thereof, wherein the compound represented by the general formula (I) is a compound represented by the general formula (1-3): 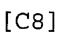 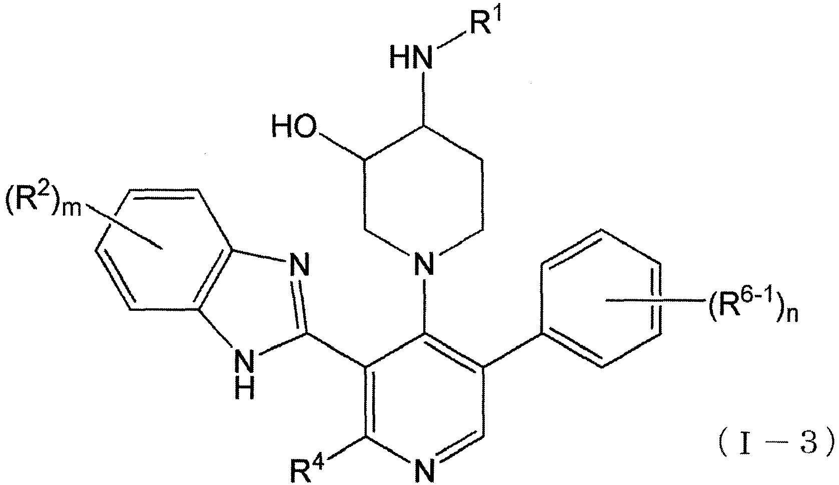 wherein R⁶⁻¹ represents a halogen and other symbols have the same meanings as described in [1];
[18] the compound according to [17] or a salt thereof, wherein the compound represented by the general formula (1-3) is a compound represented by the general formula (1-4): 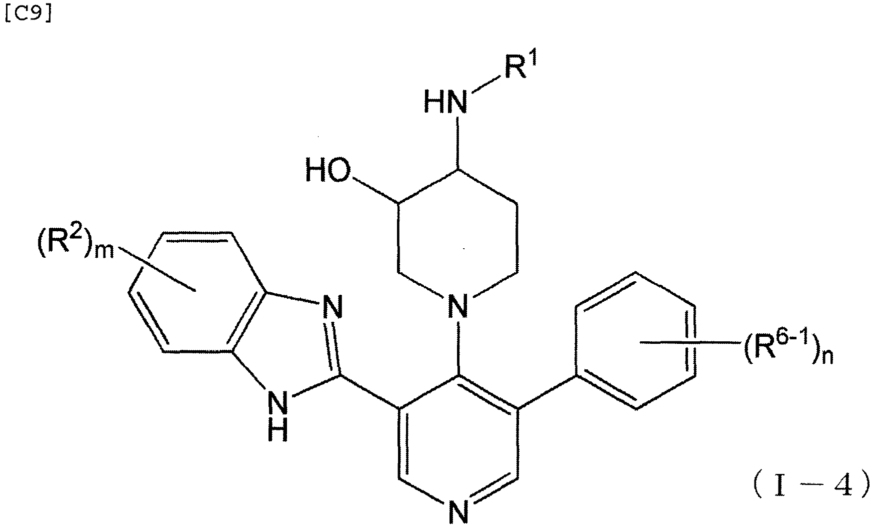 wherein all symbols have the same meanings as described in [1] ;
[19] the compound according to [17] or [18] or a salt thereof, wherein the compound represented by the general formula (1-4) is a compound represented by the general formula (1-5) : 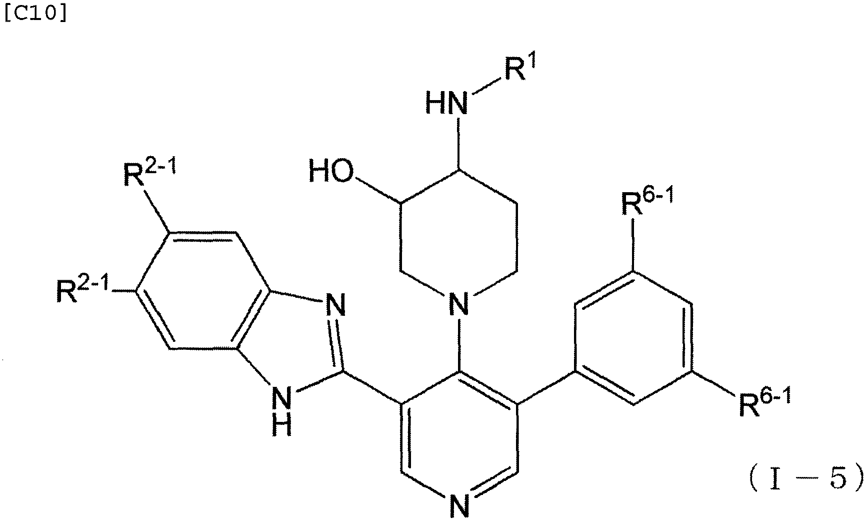 wherein R²⁻¹ represents a halogen and other symbols have the same meanings as described in [1];
[20] a pharmaceutical composition containing the compound represented by the general formula (I) according to [1] or a salt thereof, and a pharmaceutically acceptable carrier;
[21] the pharmaceutical composition according to [20], which is a somatostatin receptor agonist;
[22] the pharmaceutical composition according to [20] or [21], which is a prophylactic and/or therapeutic agent for a somatostatin-related disease;
[23] the pharmaceutical composition according to any of [20] to [22], wherein the somatostatin-related disease is acromegaly, or a gastrointestinal symptom accompanying gastrointestinal obstruction;
[24] a prophylactic and/or therapeutic agent for a somatostatin-related disease, containing the compound represented by the general formula (I) according to [1] or a salt thereof as an active component, wherein the prophylactic and/or therapeutic agent is administered together with at least one drug selected from the group consisting of pegvisomant, bromocriptine and cabergoline;
[25] a prophylactic and/or therapeutic agent for a somatostatin-related disease, containing the compound represented by the general formula (I) according to [1] or a salt thereof as an active component, wherein the prophylactic and/or therapeutic agent is administered together with at least one drug selected from the group consisting of prochlorperazine, levomepromazine, risperidone, metoclopramide, domperidone, diphenhydramine, chlorpheniramine, dimenhydrinate, promethazine, diprophylline, famotidine, cimetidine, scopolamine, tropisetron, granisetron, ondansetron, azasetron, ramosetron, indisetron, palonosetron, cisapride, mosapride, dexamethasone, betamethasone, prednisolone, aprepitant, olanzapine, quetiapine, perospirone, methylnaltrexone and morphine;
[26] a method for prophylaxis and/or therapy of a somatostatin-related disease, including administering to a mammal an effective amount of the compound represented by the general formula (I) according to [1] or a salt thereof;
[27] the compound represented by the general formula (I) according to [1] or a salt thereof for prophylaxis and/or therapy of a somatostatin-related disease;
[28] use of the compound represented by the general formula (I) according to [1] or a salt thereof for producing a prophylactic and/or therapeutic agent for a somatostatin-related disease;
[29] a prophylactic and/or therapeutic agent for a somatostatin-related disease containing the compound represented by the general formula (I) according to [1] or a salt thereof as an active component, wherein the prophylactic and/or therapeutic agent is used to be administered in combination with at least one drug selected from the group consisting of pegvisomant, bromocriptine and cabergoline;
[30] a prophylactic and/or therapeutic agent for a somatostatin-related disease containing at least one drug selected from the group consisting of pegvisomant, bromocriptine and cabergoline as an active component, wherein the prophylactic and/or therapeutic agent is used to be administered in combination with the compound represented by the general formula (I) according to [1] or a salt thereof;
[31] a prophylactic and/or therapeutic agent for a somatostatin-related disease containing at least one drug selected from the group consisting of pegvisomant, bromocriptine and cabergoline and the compound represented by the general formula (I) according to [1] or a salt thereof;
[32] a pharmaceutical composition containing at least one drug selected from the group consisting of pegvisomant, bromocriptine and cabergoline and the compound represented by the general formula (I) according to [1] or a salt thereof;
[33] a prophylactic and/or therapeutic agent for a somatostatin-related disease containing the compound represented by the general formula (I) according to [1] or a salt thereof as an active component, wherein the prophylactic and/or therapeutic agent is used to be administered in combination with at least one drug selected from the group consisting of prochlorperazine, levomepromazine, risperidone, metoclopramide, domperidone, diphenhydramine, chlorpheniramine, dimenhydrinate, promethazine, diprophylline, famotidine, cimetidine, scopolamine, tropisetron, granisetron, ondansetron, azasetron, ramosetron, indisetron, palonosetron, cisapride, mosapride, dexamethasone, betamethasone, prednisolone, aprepitant, olanzapine, quetiapine, perospirone, methylnaltrexone and morphine;
[34] a prophylactic and/or therapeutic agent for a somatostatin-related disease containing at least one drug selected from the group consisting of prochlorperazine, levomepromazine, risperidone, metoclopramide, domperidone, diphenhydramine, chlorpheniramine, dimenhydrinate, promethazine, diprophylline, famotidine, cimetidine, scopolamine, tropisetron, granisetron, ondansetron, azasetron, ramosetron, indisetron, palonosetron, cisapride, mosapride, dexamethasone, betamethasone, prednisolone, aprepitant, olanzapine, quetiapine, perospirone, methylnaltrexone and morphine as an active component, wherein the prophylactic and/or therapeutic agent is used to be administered in combination with the compound represented by the general formula (I) according to [1] or a salt thereof;
[35] a prophylactic and/or therapeutic agent for a somatostatin-related disease containing at least one drug selected from the group consisting of prochlorperazine, levomepromazine, risperidone, metoclopramide, domperidone, diphenhydramine, chlorpheniramine, dimenhydrinate, promethazine, diprophylline, famotidine, cimetidine, scopolamine, tropisetron, granisetron, ondansetron, azasetron, ramosetron, indisetron, palonosetron, cisapride, mosapride, dexamethasone, betamethasone, prednisolone, aprepitant, olanzapine, quetiapine, perospirone, methylnaltrexone and morphine and the compound represented by the general formula (I) according to [1] or a salt thereof;
[36] a pharmaceutical composition containing at least one drug selected from the group consisting of prochlorperazine, levomepromazine, risperidone, metoclopramide, domperidone, diphenhydramine, chlorpheniramine, dimenhydrinate, promethazine, diprophylline, famotidine, cimetidine, scopolamine, tropisetron, granisetron, ondansetron, azasetron, ramosetron, indisetron, palonosetron, cisapride, mosapride, dexamethasone, betamethasone, prednisolone, aprepitant, olanzapine, quetiapine, perospirone, methylnaltrexone and morphine and the compound represented by the general formula (I) according to [1] or a salt thereof;
[37] a method for prophylaxis and/or therapy of a somatostatin-related disease, including separately or simultaneously administering, to a patient in need of prophylaxis and/or therapy of a somatostatin-related disease, an effective amount of at least one drug selected from the group consisting of pegvisomant, bromocriptine and cabergoline and an effective amount of the compound represented by the general formula (I) according to [1] or a salt thereof;
[38] a method for prophylaxis and/or therapy of a somatostatin-related disease, including administering, to a patient in need of prophylaxis and/or therapy of a somatostatin-related disease, an effective amount of at least one drug selected from the group consisting of pegvisomant, bromocriptine and cabergoline, wherein the method further includes administering an effective amount of the compound represented by the general formula (I) according to [1] or a salt thereof;
[39] a method for prophylaxis and/or therapy of a somatostatin-related disease, including administering, to a patient in need of prophylaxis and/or therapy of a somatostatin-related disease, an effective amount of the compound represented by the general formula (I) according to [1] or a salt thereof, wherein the method further includes administering an effective amount of at least one drug selected from the group consisting of pegvisomant, bromocriptine and cabergoline;
[40] a method for prophylaxis and/or therapy of a somatostatin-related disease, including separately or simultaneously administering, to a patient in need of prophylaxis and/or therapy of a somatostatin-related disease, an effective amount of at least one drug selected from the group consisting of prochlorperazine, levomepromazine, risperidone, metoclopramide, domperidone, diphenhydramine, chlorpheniramine, dimenhydrinate, promethazine, diprophylline, famotidine, cimetidine, scopolamine, tropisetron, granisetron, ondansetron, azasetron, ramosetron, indisetron, palonosetron, cisapride, mosapride, dexamethasone, betamethasone, prednisolone, aprepitant, olanzapine, quetiapine, perospirone, methylnaltrexone and morphine and an effective amount of the compound represented by the general formula (I) according to [1] or a salt thereof;
[41] a method for prophylaxis and/or therapy of a somatostatin-related disease, including administering, to a patient in need of prophylaxis and/or therapy of a somatostatin-related disease, an effective amount of at least one drug selected from the group consisting of prochlorperazine, levomepromazine, risperidone, metoclopramide, domperidone, diphenhydramine, chlorpheniramine, dimenhydrinate, promethazine, diprophylline, famotidine, cimetidine, scopolamine, tropisetron, granisetron, ondansetron, azasetron, ramosetron, indisetron, palonosetron, cisapride, mosapride, dexamethasone, betamethasone, prednisolone, aprepitant, olanzapine, quetiapine, perospirone, methylnaltrexone and morphine, wherein the method further includes administering an effective amount of the compound represented by the general formula (I) according to [1] or a salt thereof;
[42] a method for prophylaxis and/or therapy of a somatostatin-related disease, including administering, to a patient in need of prophylaxis and/or therapy of a somatostatin-related disease, an effective amount of the compound represented by the general formula (I) according to [1] or a salt thereof, wherein the method further includes administering an effective amount of at least one drug selected from the group consisting of prochlorperazine, levomepromazine, risperidone, metoclopramide, domperidone, diphenhydramine, chlorpheniramine, dimenhydrinate, promethazine, diprophylline, famotidine, cimetidine, scopolamine, tropisetron, granisetron, ondansetron, azasetron, ramosetron, indisetron, palonosetron, cisapride, mosapride, dexamethasone, betamethasone, prednisolone, aprepitant, olanzapine, quetiapine, perospirone, methylnaltrexone and morphine;
[43] the compound represented by the general formula (I) according to [1] or a salt thereof used for prophylaxis and/or therapy of a somatostatin-related disease, which is administered in combination with at least one drug selected from the group consisting of pegvisomant, bromocriptine and cabergoline;
[44] at least one drug selected from the group consisting of pegvisomant, bromocriptine and cabergoline used for prophylaxis and/or therapy of a somatostatin-related disease, which is administered in combination with the compound represented by the general formula (I) according to [1] or a salt thereof;
[45] the compound represented by the general formula (I) according to [1] or a salt thereof used for prophylaxis and/or therapy of a somatostatin-related disease, which is administered in combination with at least one drug selected from the group consisting of prochlorperazine, levomepromazine, risperidone, metoclopramide, domperidone, diphenhydramine, chlorpheniramine, dimenhydrinate, promethazine, diprophylline, famotidine, cimetidine, scopolamine, tropisetron, granisetron, ondansetron, azasetron, ramosetron, indisetron, palonosetron, cisapride, mosapride, dexamethasone, betamethasone, prednisolone, aprepitant, olanzapine, quetiapine, perospirone, methylnaltrexone and morphine; and
[46] at least one drug selected from the group consisting of prochlorperazine, levomepromazine, risperidone, metoclopramide, domperidone, diphenhydramine, chlorpheniramine, dimenhydrinate, promethazine, diprophylline, famotidine, cimetidine, scopolamine, tropisetron, granisetron, ondansetron, azasetron, ramosetron, indisetron, palonosetron, cisapride, mosapride, dexamethasone, betamethasone, prednisolone, aprepitant, olanzapine, quetiapine, perospirone, methylnaltrexone and morphine used for prophylaxis and/or therapy of a somatostatin-related disease, which is administered in combination with the compound represented by the general formula (I) according to [1] or a salt thereof.

### [Advantageous Effects of Invention]

The compound represented by the general formula (I) or a salt thereof (hereinafter collectively referred to as the present compound) as disclosed herein is a low-molecular compound having strong agonistic activity particularly for, among somatostatin receptors, somatostatin receptor subtype 2 (SSTR2), and can be orally administered. Therefore, the present compound does not require intramuscular injection that is required for administration of existing peptide medicaments typically including octreotide acetate and lanreotide acetate, and can be easily administered and can alleviate pain accompanied by the therapy of patients. The present compound also has a sufficiently weak hERG inhibitory activity and/or phospholipidosis action relative to the SSTR2 agonistic activity, and thus can suppress or alleviate side effects resulting from the activity/action. The present compound can thus be safely used for patients with somatostatin-related diseases in need of administration thereof, particularly for patients with acromegaly and gastrointestinal obstruction.

### [Description of Embodiments]

Examples of "halogen" as used herein include fluorine, chlorine, bromine and iodine atoms.

The "C1-4 alkyl" as used herein includes methyl, ethyl, propyl, isopropyl, butyl, sec-butyl, tert-butyl and isobutyl groups.

The "C1-4 alkoxy" as used herein includes methoxy, ethoxy, propoxy, isopropoxy, butoxy, sec-butoxy, tert-butoxy and isobutoxy groups.

The "C1-4 alkoxycarbonyl" as used herein includes methoxycarbonyl, ethoxycarbonyl, propoxycarbonyl, isopropoxycarbonyl, butoxycarbonyl, sec-butoxycarbonyl, tert-butoxycarbonyl and isobutoxycarbonyl groups.

Examples of "C5-6 monocyclic carbocycle" as used herein include cyclopentane, cyclohexane, cyclopentene, cyclohexene, cyclopentadiene, cyclohexadiene and benzene rings.

Examples of "5- to 6-membered monocyclic heterocycle" as used herein include "5- to 6-membered monocyclic heterocycles containing 1 to 4 nitrogen atoms, 1 to 2 oxygen atoms and/or 1 sulfur atom" and the like. Examples of the "5- to 6-membered monocyclic heterocycles containing 1 to 4 nitrogen atoms, 1 to 2 oxygen atoms and/or 1 sulfur atom" include pyrrole, imidazole, triazole, tetrazole, pyrazole, pyridine, pyrazine, pyrimidine, pyridazine, furan, pyran, thiophene, thiopyran, oxazole, isoxazole, thiazole, isothiazole, furazan, oxadiazole, oxazine, oxadiazine, thiadiazole, thiazine, thiadiazine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrofuran, tetrahydrofuran, dihydropyran, tetrahydropyran, dihydrothiophene, tetrahydrothiophene, dihydrothiopyran, tetrahydrothiopyran, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrothiadiazole, tetrahydrothiadiazole (thiadiazolidine), dihydrothiazine, tetrahydrothiazine, dihydrothiadiazine, tetrahydrothiadiazine, morpholine, thiomorpholine, oxathiane, dioxolane, dioxane and dioxole rings and the like.

In the present invention, unless particularly stated, the symbol:

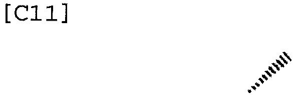

indicates that the bond projects below the plane of the paper (i.e. α-configuration), and the symbol: 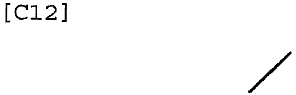 indicates that the bond is the α-configuration, β-configuration or the mixture of these configurations at arbitrary proportions, as apparent to a person skilled in the art.

In the present invention, R¹ is preferably, for example, 3-oxetanyl, 3,3,3-trifluoropropyl or ethyl. R¹ is also preferably a hydrogen atom.

In the present invention, R² is preferably, for example, a C1-4 alkoxy, a halogen or a C1-4 alkoxycarbonyl, more preferably, for example, a C1-4 alkoxy or a halogen and particularly preferably, for example, fluorine.

In the present invention, R³ is preferably, for example, a hydrogen atom.

In the present invention, R⁴ is preferably a hydrogen atom or a C1-4 alkyl, more preferably, for example, a hydrogen atom or methyl and particularly preferably, for example, methyl. R⁴ is also particularly preferably a hydrogen atom.

In the present invention, R⁵ is preferably, for example, a hydrogen atom.

In the present invention, ring1 is preferably, for example, a C5-6 monocyclic carbocycle or a 5- to 6-membered monocyclic heterocycle and more preferably, for example, a benzene ring.

In the present invention, R⁶ is preferably, for example, a C1-4 alkoxy or a halogen and more preferably, for example, methoxy, fluorine or chlorine.

In the present invention, R²⁻¹ is preferably, for example, fluorine.

In the present invention, R⁶⁻¹ is preferably, for example, fluorine.

In the present invention, m is preferably, for example, 2.

In the present invention, n is preferably, for example, 2.

In the present invention, examples of the general formula (I) preferably include the general formula (IB): 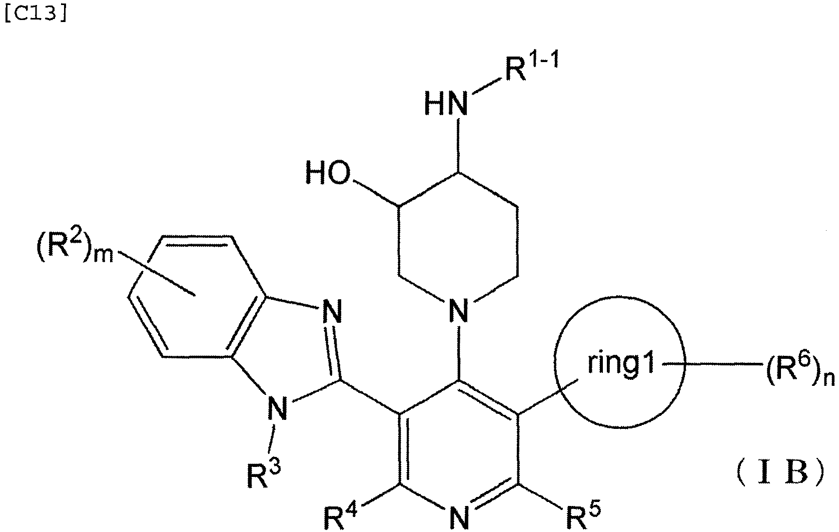 wherein R¹⁻¹ represents 3-oxetanyl, 3,3,3-trifluoropropyl or ethyl and other symbols have the same meanings as above,
the general formula (IB-1): 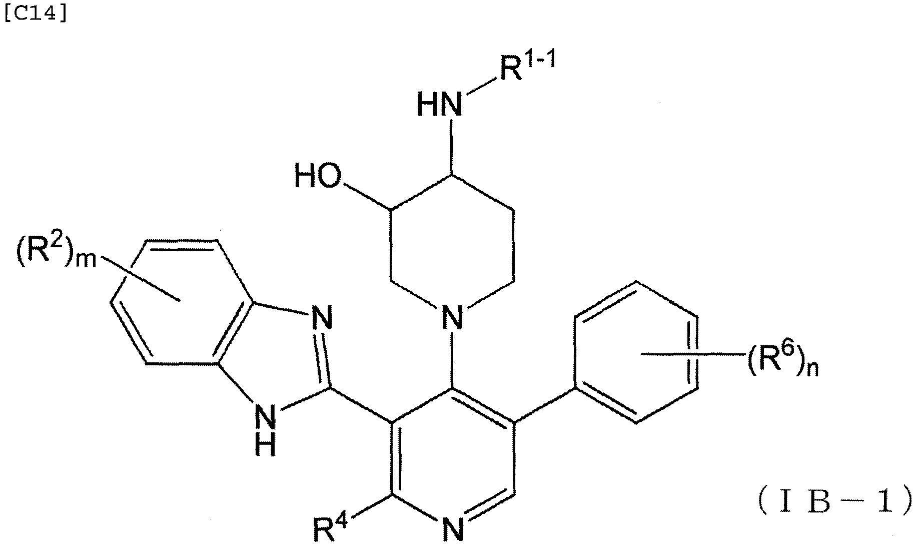 wherein all symbols have the same meanings as above,
the general formula (I-1):

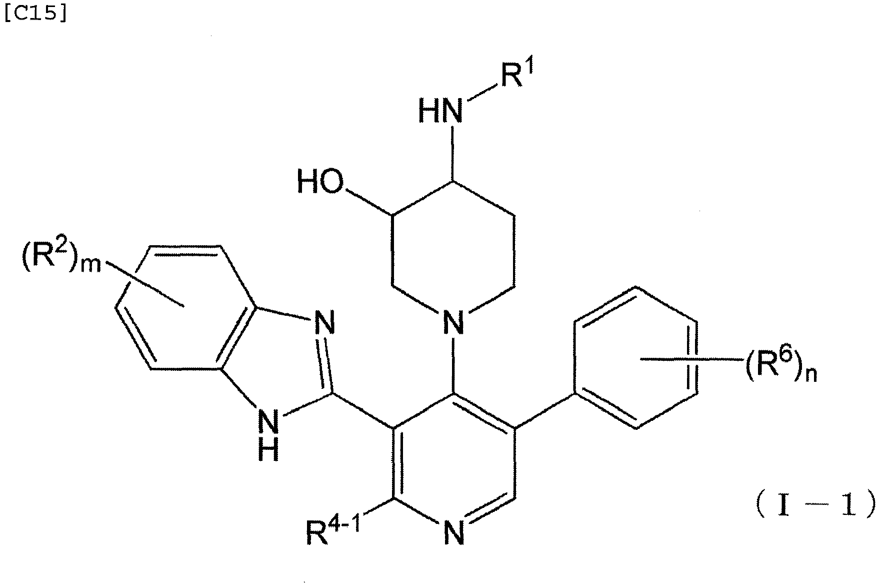

wherein R⁴⁻¹ represents a C1-4 alkyl, a C5-6 monocyclic carbocycle which may be substituted with 1 to 3 R⁷ groups or a 5- to 6-membered monocyclic heterocycle which may be substituted with 1 to 3 R⁸ groups and other symbols have the same meanings as above,
the general formula (1-2): 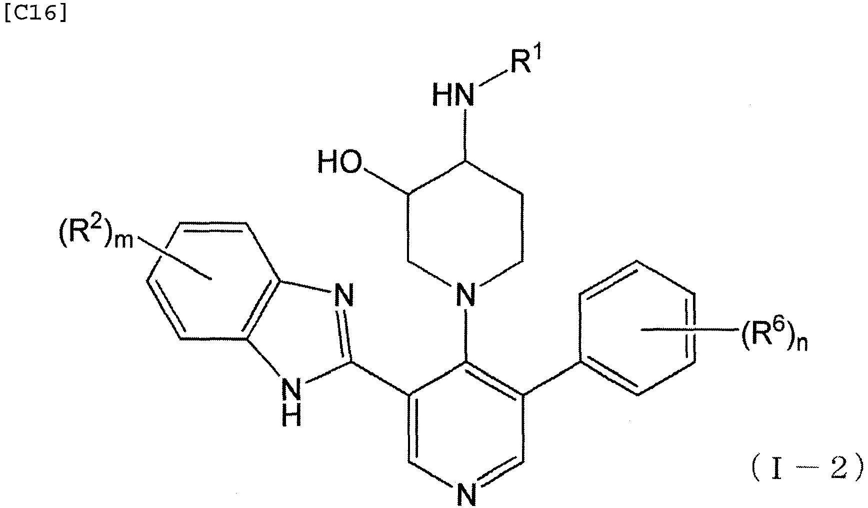 wherein all symbols have the same meanings as above,
the general formula (1-3): 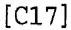 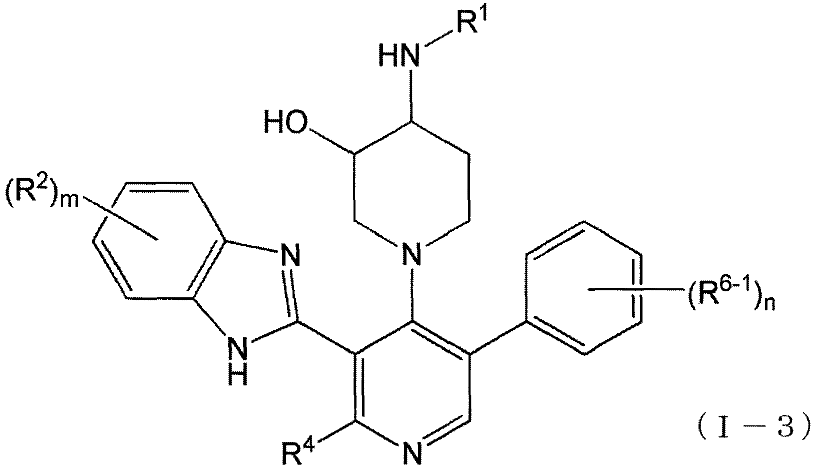 wherein R⁶⁻¹ represents a halogen and other symbols have the same meanings as above,
the general formula (1-4): 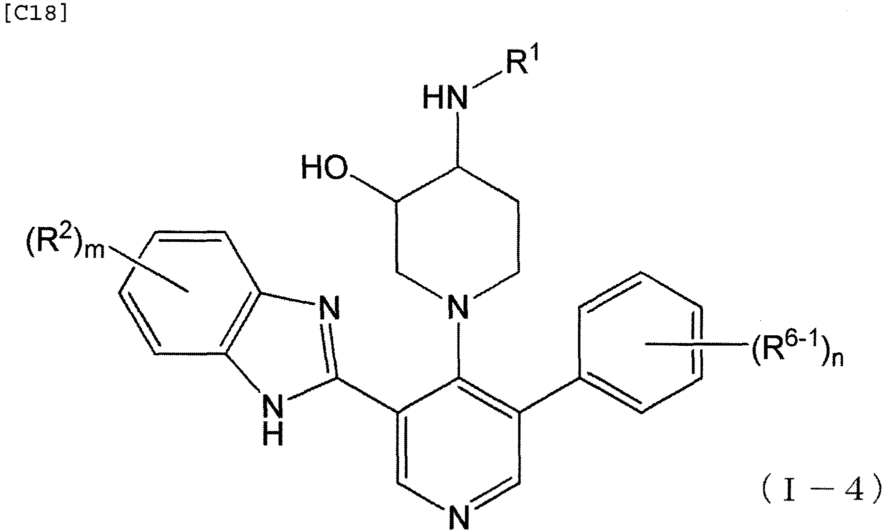 wherein all symbols have the same meanings as above,
the general formula (1-5): 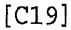 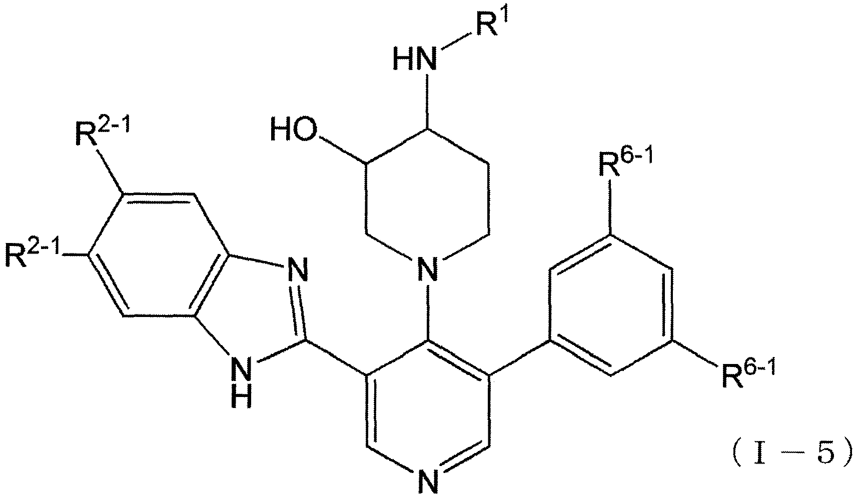 wherein R²⁻¹ represents a halogen and other symbols have the same meanings as above. More preferably, examples include the general formula (I-A): 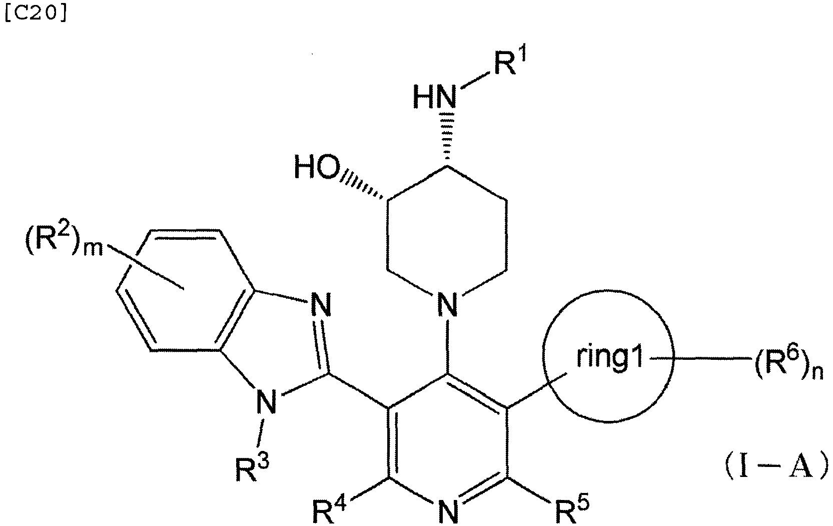 wherein all symbols have the same meanings as above,
the general formula (IB-A): 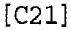 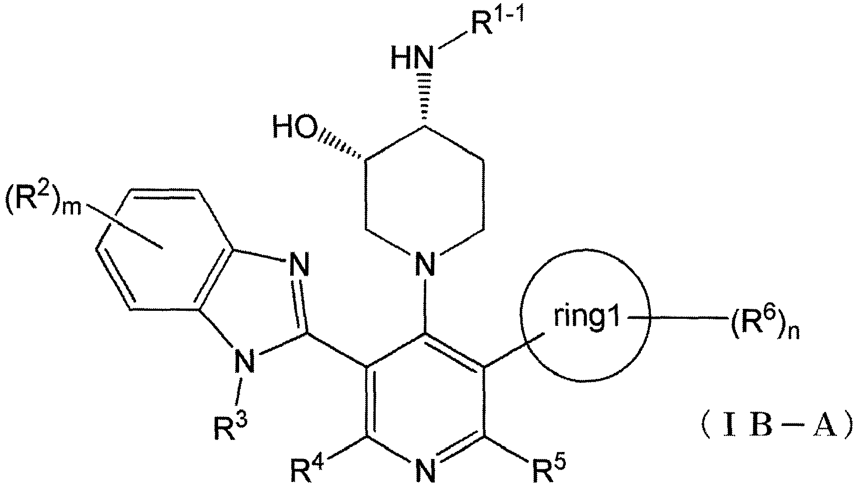 wherein all symbols have the same meanings as above,
the general formula (IB-1-1): 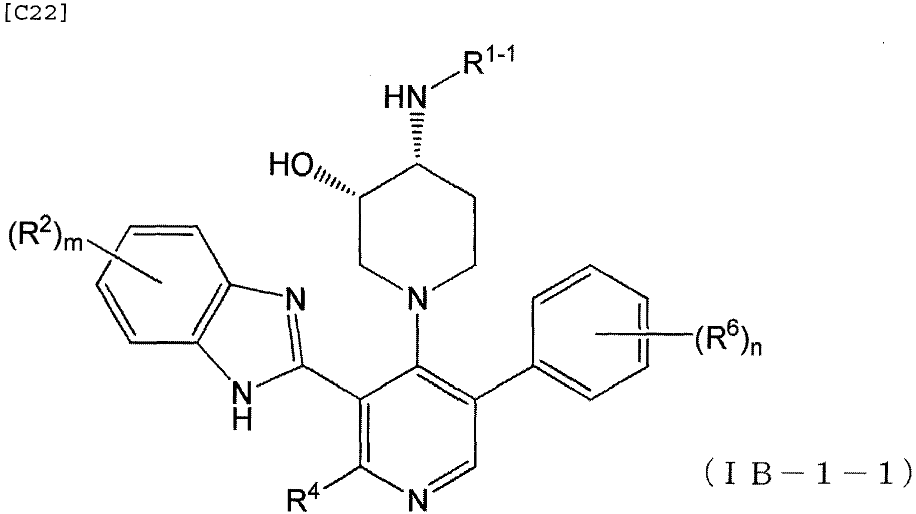 wherein all symbols have the same meanings as above,
the general formula (I-1-1): 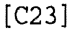 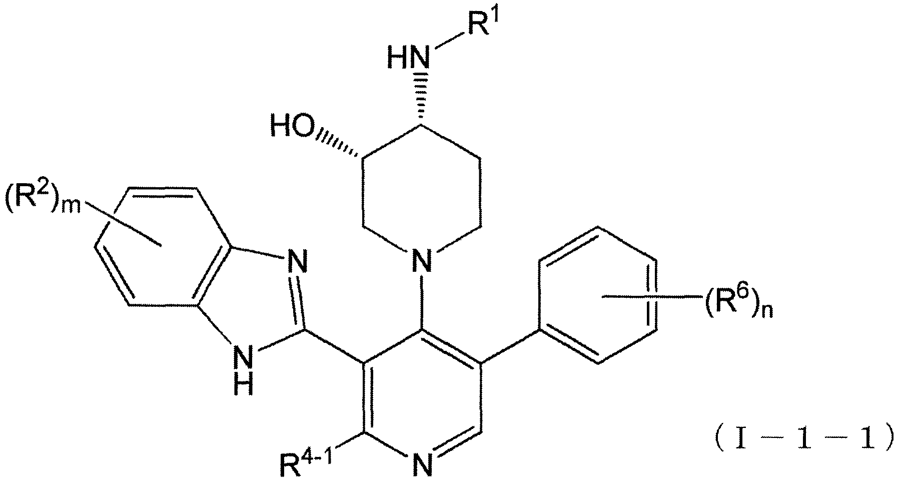 wherein all symbols have the same meanings as above,
the general formula (1-2-1): 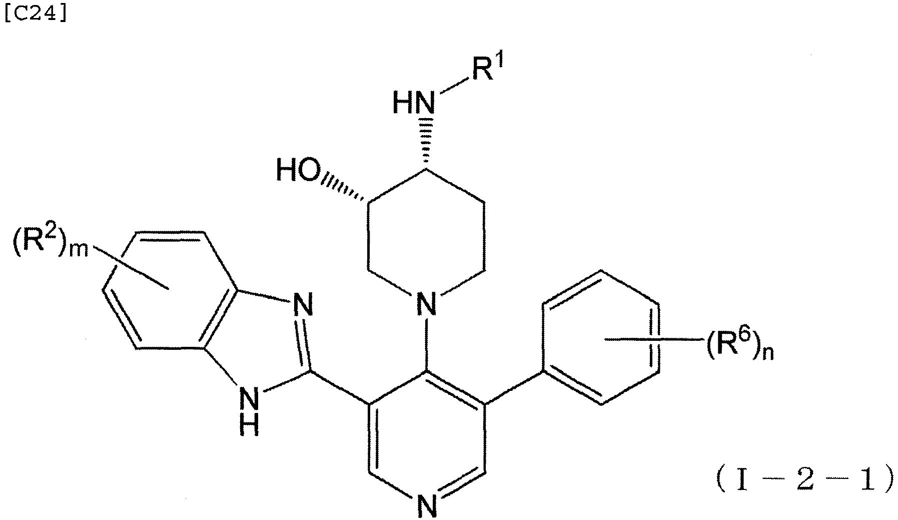 wherein all symbols have the same meanings as above,
the general formula (1-3-1): 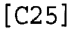 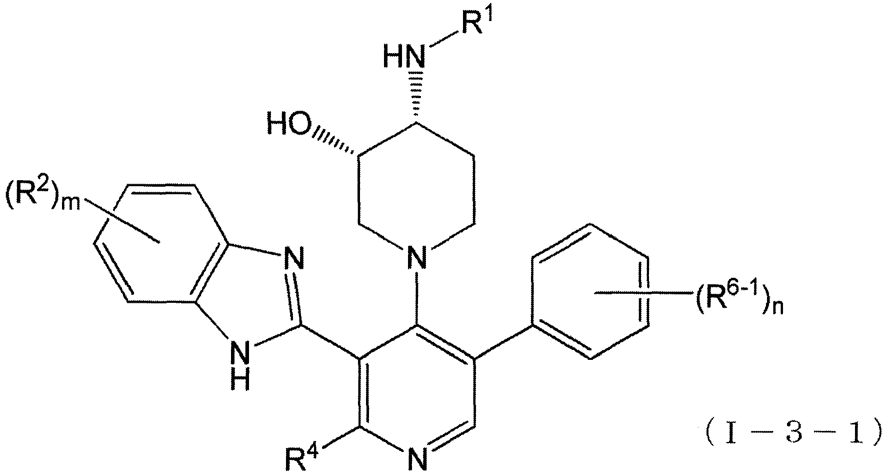 wherein all symbols have the same meanings as above,
the general formula (1-4-1): 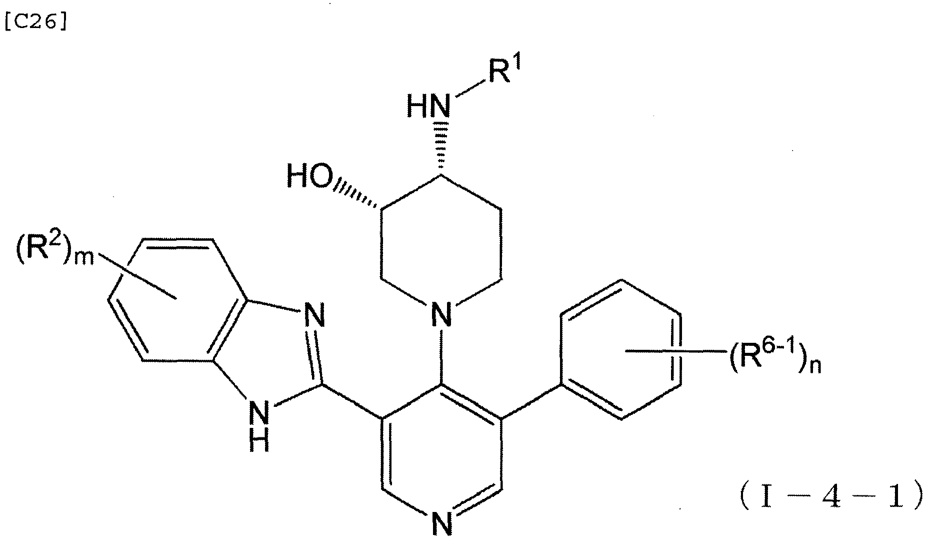 wherein all symbols have the same meanings as above, and
the general formula (1-5-1): 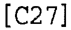 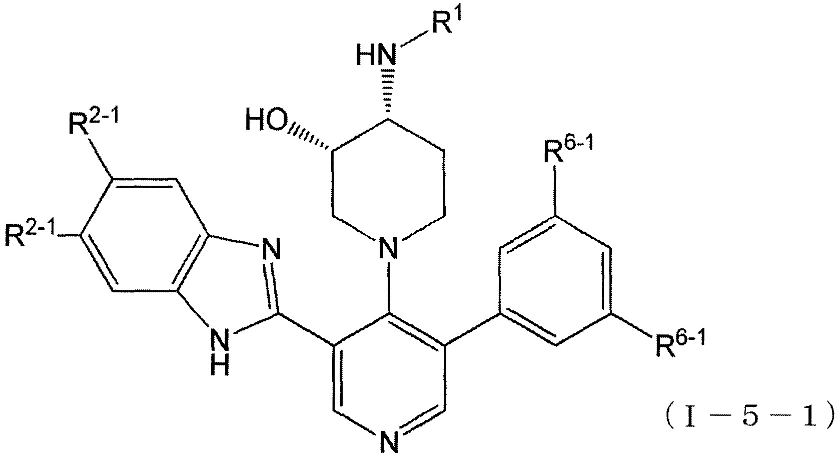 wherein all symbols have the same meanings as above.

In the present invention, the compound is preferably, for example:
(1) (3S,4R)-4-amino-1-[3-(5,6-difluoro-1H-benzimidazol-2-yl)-5-(3,5-difluorophenyl)-2-(1-methyl-1H-pyrazol-4-yl)-4-pyridinyl]-3-piperidinol;
(2) methyl 2-{4-[(3S,4R)-4-amino-3-hydroxy-1-piperidinyl]-5-(3-chloro-5-fluorophenyl)-2-methyl-3-pyridinyl}-5-fluoro-1H-benzimidazole-7-carboxylate;
(3) methyl 2-{4-[(3S,4R)-4-amino-3-hydroxy-1-piperidinyl]-5-(2,5-difluorophenyl)-2-methyl-3-pyridinyl}-5-fluoro-1H-benzimidazole-7-carboxylate;
(4) (3S,4R)-4-amino-1-[5-(3,5-difluorophenyl)-2-methyl-3-(5,6,7-trifluoro-1H-benzimidazol-2-yl)-4-pyridinyl]-3-piperidinol;
(5) (3S,4R)-1-[5-(3-chloro-5-fluorophenyl)-3-(6-methoxy-1H-benzimidazol-2-yl)-2-methyl-4-pyridinyl]-4-(3-oxetanylamino)-3-piperidinol;
(6) (3S,4R)-1-[5-(3,5-difluorophenyl)-3-(5-fluoro-6-methoxy-1H-benzimidazol-2-yl)-2-methyl-4-pyridinyl]-4-(ethylamino)-3-piperidinol;
(7) (3S,4R)-1-[5-(3-chloro-5-fluorophenyl)-3-(5,6-difluoro-1H-benzimidazol-2-yl)-2-methyl-4-pyridinyl]-4-(3-oxetanylamino)-3-piperidinol;
(8) (3S,4R)-1-[3-(5,6-difluoro-1H-benzimidazol-2-yl)-5-(3,5-difluorophenyl)-2-methyl-4-pyridinyl]-4-[(3,3,3-trifluoropropyl)amino]-3-piperidinol;
(9) (3S,4R)-1-[5-(3,5-difluorophenyl)-2-methyl-3-(5,6,7-trifluoro-1H-benzimidazol-2-yl)-4-pyridinyl]-4-(ethylamino)-3-piperidinol; or
(10) methyl 2-{4-[(3S,4R)-4-amino-3-hydroxy-1-piperidinyl]-5-(3,5-difluorophenyl)-2-(4-morpholinyl)-3-pyridinyl}-5-fluoro-1H-benzimidazole-7-carboxylate;
or a salt thereof.

The compound is more preferably, for example:
(1) methyl 2-{4-[(3S,4R)-4-amino-3-hydroxy-1-piperidinyl]-5-(3-chloro-5-fluorophenyl)-2-methyl-3-pyridinyl}-5-fluoro-1H-benzimidazole-7-carboxylate;
(2) (3S,4R)-1-[5-(3,5-difluorophenyl)-3-(5-fluoro-6-methoxy-1H-benzimidazol-2-yl)-2-methyl-4-pyridinyl]-4-(ethylamino)-3-piperidinol;
(3) (3S,4R)-1-[5-(3-chloro-5-fluorophenyl)-3-(5,6-difluoro-1H-benzimidazol-2-yl)-2-methyl-4-pyridinyl]-4-(3-oxetanylamino)-3-piperidinol;
(4) (3S,4R)-1-[3-(5,6-difluoro-1H-benzimidazol-2-yl)-5-(3,5-difluorophenyl)-2-methyl-4-pyridinyl]-4-[(3,3,3-trifluoropropyl)amino]-3-piperidinol; or
(5) (3S,4R)-1-[5-(3,5-difluorophenyl)-2-methyl-3-(5,6,7-trifluoro-1H-benzimidazol-2-yl)-4-pyridinyl]-4-(ethylamino)-3-piperidinol;
or a salt thereof.

The compound particularly preferably, for example: methyl 2-{4-[(3S,4R)-4-amino-3-hydroxy-1-piperidinyl]-5-(3-chloro-5-fluorophenyl)-2-methyl-3-pyridinyl}-5-fluoro-1H-benzimidazole-7-carboxylate or a salt thereof.

(3S,4R)-1-[5-(3,5-difluorophenyl)-3-(5-fluoro-6-methoxy-1H-benzimidazol-2-yl)-2-methyl-4-pyridinyl]-4-(ethylamino)-3-piperidinol or a salt thereof is also particularly preferable.

(3S,4R)-1-[5-(3-chloro-5-fluorophenyl)-3-(5,6-difluoro-1H-benzimidazol-2-yl)-2-methyl-4-pyridinyl]-4-(3-oxetanylamino)-3-piperidinol or a salt thereof is also particularly preferable.

(3S,4R)-1-[3-(5,6-difluoro-1H-benzimidazol-2-yl)-5-(3,5-difluorophenyl)-2-methyl-4-pyridinyl]-4-[(3,3,3-trifluoropropyl)amino]-3-piperidinol or a salt thereof is also particularly preferable.

(3S,4R)-1-[5-(3,5-difluorophenyl)-2-methyl-3-(5,6,7-trifluoro-1H-benzimidazol-2-yl)-4-pyridinyl]-4-(ethylamino)-3-piperidinol or a salt thereof is also particularly preferable.
(1) (3S,4R)-4-amino-1-[3-(5,6-difluoro-1H-benzimidazol-2-yl)-5-(3,5-difluorophenyl)-4-pyridinyl]-3-piperidinol;
(2) (3S,4R)-4-amino-1-[3-(1H-benzimidazol-2-yl)-5-(3,5-difluorophenyl)-4-pyridinyl]-3-piperidinol;
(3) (3S,4R)-4-amino-1-[3-(2,5-difluorophenyl)-5-(6-methoxy-1H-benzimidazol-2-yl)-4-pyridinyl]-3-piperidinol;
(4) methyl 2-{4-[(3S,4R)-4-amino-3-hydroxy-1-piperidinyl]-5-(2,5-difluorophenyl)-3-pyridinyl}-5-fluoro-1H-benzimidazole-7-carboxylate;
(5) (3S,4R)-1-[3-(5,6-difluoro-1H-benzimidazol-2-yl)-5-(3,5-difluorophenyl)-4-pyridinyl]-4-(3-oxetanylamino)-3-piperidinol;
(6) (3S,4R)-1-[3-(3-chloro-5-fluorophenyl)-5-(5,6-difluoro-1H-benzimidazol-2-yl)-4-pyridinyl]-4-[(3,3,3-trifluoropropyl)amino]-3-piperidinol;
(7) (3S,4R)-1-[3-(5,6-difluoro-1H-benzimidazol-2-yl)-5-(2,5-difluorophenyl)-4-pyridinyl]-4-(ethylamino)-3-piperidinol;
(8) (3S,4R)-1-[3-(5,6-difluoro-1H-benzimidazol-2-yl)-5-(3-fluoro-5-methoxyphenyl)-4-pyridinyl]-4-[(3,3,3-trifluoropropyl)amino]-3-piperidinol; or
(9) (3S,4R)-1-[3-(3,5-difluorophenyl)-5-(6-fluoro-5-methoxy-1H-benzimidazol-2-yl)-4-pyridinyl]-4-[(3,3,3-trifluoropropyl)amino]-3-piperidinol;
or a salt thereof is also preferable.

The compound is more preferably, for example:
(1) (3S,4R)-4-amino-1-[3-(5,6-difluoro-1H-benzimidazol-2-yl)-5-(3,5-difluorophenyl)-4-pyridinyl]-3-piperidinol;
(2) methyl 2-{4-[(3S,4R)-4-amino-3-hydroxy-1-piperidinyl]-5-(2,5-difluorophenyl)-3-pyridinyl}-5-fluoro-1H-benzimidazole-7-carboxylate;
(3) (3S,4R)-1-[3-(5,6-difluoro-1H-benzimidazol-2-yl)-5-(3,5-difluorophenyl)-4-pyridinyl]-4-(3-oxetanylamino)-3-piperidinol;
(4) (3S,4R)-1-[3-(5,6-difluoro-1H-benzimidazol-2-yl)-5-(2,5-difluorophenyl)-4-pyridinyl]-4-(ethylamino)-3-piperidinol; or
(5) (3S,4R)-1-[3-(3,5-difluorophenyl)-5-(6-fluoro-5-methoxy-1H-benzimidazol-2-yl)-4-pyridinyl]-4-[(3,3,3-trifluoropropyl)amino]-3-piperidinol;
or a salt thereof.

The compound is particularly preferably, for example: (3S,4R)-4-amino-1-[3-(5,6-difluoro-1H-benzimidazol-2-yl)-5-(3,5-difluorophenyl)-4-pyridinyl]-3-piperidinol or a salt thereof.

Methyl 2-{4-[(3S,4R)-4-amino-3-hydroxy-1-piperidinyl]-5-(2,5-difluorophenyl)-3-pyridinyl}-5-fluoro-1H-benzimidazole-7-carboxylate or a salt thereof is also particularly preferable.

(3S,4R)-1-[3-(5,6-difluoro-1H-benzimidazol-2-yl)-5-(3,5-difluorophenyl)-4-pyridinyl]-4-(3-oxetanylamino)-3-piperidinol or a salt thereof is also particularly preferable.

(3S,4R)-1-[3-(5,6-difluoro-1H-benzimidazol-2-yl)-5-(2,5-difluorophenyl)-4-pyridinyl]-4-(ethylamino)-3-piperidinol or a salt thereof is also particularly preferable.

(3S,4R)-1-[3-(3,5-difluorophenyl)-5-(6-fluoro-5-methoxy-1H-benzimidazol-2-yl)-4-pyridinyl]-4-[(3,3,3-trifluoropropyl)amino]-3-piperidinol or a salt thereof is also particularly preferable.

### [Isomers]

The present invention encompasses all isomers unless otherwise particularly stated. For example, alkyl groups, alkoxy groups and the like include linear and branched groups. Moreover, the present invention encompasses isomers for double bonds, rings and condensed rings (E-forms, Z-forms, cis forms and trans forms), isomers due to asymmetrical carbon atoms (R and S forms, α and β configurations, enantiomers and diastereomers), optically active substances having optical rotating activity (D, L, d and 1 forms), polar substances which can be separated by chromatography (high polarity substances and low polarity substances), equilibrium compounds, rotamers, mixtures thereof at arbitrary proportions and racemic mixtures. The present invention also encompasses tautomers.

### [Salt and solvate]

A salt of the compound represented by the general formula (I) disclosed herein encompasses all pharmacologically acceptable salts. The pharmacologically acceptable salt is preferably a water-soluble salt with low toxicity. Examples of appropriate salts include acid addition salts (such as inorganic acid salt [examples: hydrochloride, hydrobromide, hydroiodide, sulphate, phosphate, nitrate and the like], organic acid salts [examples: acetate, trifluoroacetate, lactate, tartrate, oxalate, fumarate, maleate, benzoate, citrate, methanesulphonate, ethanesulphonate, benzenesulphonate, toluenesulphonate, isethionate, glucuronate, gluconate and the like], salts with acidic natural amino acids [examples: aspartate, glutamate and the like] and the like) and the like.

A salt also encompasses quaternary ammonium salts. The quaternary ammonium salt represents a compound represented by the general formula (I) in which a nitrogen atom thereof is quaternised with an R⁰ group. The R⁰ group as used herein represents, for example, a C1-8 alkyl group which may be substituted with a phenyl group.

The compound represented by the general formula (I) can be converted to the salt, N-oxide and solvate according to well-known methods.

The N-oxide of the compound represented by the general formula (I) represents the compound represented by the general formula (I) in which a nitrogen atom is oxidized. The N-oxide may form salts such as acid addition salts as described above.

The compound represented by the general formula (I), a salt thereof or an N-oxide thereof may form a solvate with, for example, water or an alcoholic solvent (such as ethanol). The solvate preferably has low toxicity and is water soluble.

The compound represented by the general formula (I) and a salt thereof may be in the form of without forming a solvate or may be in the form of a solvate with a pharmaceutically acceptable solvent such as water and ethanol. The solvate is preferably a hydrate. The compound represented by the general formula (I) or a salt thereof can be converted to the solvate according to well-known methods.

The compound represented by the general formula (I) and a salt thereof may form a co-crystal with an appropriate co-crystal former. The co-crystal is preferably pharmaceutically acceptable as formed with a pharmaceutically acceptable co-crystal former. A co-crystal is defined to be a crystal typically formed of 2 or more molecules by intermolecular interaction that is not ionic bonding. The co-crystal may be a complex of a neutral molecule and a salt. Co-crystals may be prepared according to well-known methods such as melt crystallization, recrystallization from a solvent or physical grinding of components together. Appropriate co-crystal formers include those disclosed in WO 2006/007448.

In the present invention, all the recitations on the present compound encompass the compound represented by the general formula (I), a salt thereof, a solvate (such as hydrate) thereof, an N-oxide thereof or a co-crystal thereof, or a solvate (such as hydrate), N-oxide or co-crystal of a salt of the compound represented by the general formula (I).

Namely, in the present invention, the compound represented by the general formula (I) or a salt thereof encompasses a solvate (such as hydrate), N-oxide or co-crystal of the compound represented by the general formula (I) or a solvate (such as hydrate), N-oxide or co-crystal of a salt of the compound represented by the general formula (I).

### [Prodrug]

The prodrug of the compound represented by the general formula (I) refers to a compound which is converted *in vivo* to the compound represented by the general formula (I) by the reaction with enzymes, gastric acid and the like. Examples of the prodrug of the compound represented by the general formula (I) include, when the compound represented by the general formula (I) has an amino group, compounds in which the amino group is acylated, alkylated or phosphorylated (e.g. compounds represented by the general formula (I) in which the amino group thereof is converted to eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, acetoxymethyl, tert-butyl or the like); when the compound represented by the general formula (I) has a hydroxy group, compounds in which the hydroxy group is acylated, alkylated, phosphorylated or converted to borate (e.g. compounds represented by the general formula (I) in which the hydroxy group thereof is converted to acetyl, palmitoyl, propanoyl, pivaloyl, succinyl, fumaryl, alanyl, dimethylaminomethylcarbonyl or the like) and the like. The prodrug of the compound represented by the general formula (I) may be the one which is converted to the compound represented by the general formula (I) under the physiological condition such as those disclosed in "Iyakuhin no Kaihatsu", vol. 7 "Bunshi Sekkei", p. 163-198, 1990, Hirokawa Shoten Co. The prodrug of the compound represented by the general formula (I) can be produced by the methods well known *per se.* The prodrug of the compound represented by the general formula (I) may form, similarly to the compound represented by the general formula (I), for example, salts such as acid addition salts, or may form solvates with water or an alcoholic solvent (such as ethanol).

### [Labelled compound]

In the present invention, the compound represented by the general formula (I), or a salt thereof encompasses a so-called labelled compound in which some or all atoms constituting the compound is substituted with an isotope thereof. The labelled compound may be produced according to the methods well known *per se.* Examples of isotopes which may be used for labelling suitably include, but are not limited to, ²H, ³H, ¹³C, ¹⁴C, ¹⁵N, ¹⁶N, ¹⁷O, ¹⁸O, ³⁵S, ³⁶Cl, ⁷⁷Br, ¹²⁵I and the like.

### [Production method]

### [Method for producing compound of the present invention]

The compound represented by the general formula (I) or a salt thereof may be produced by well-known methods, for example, methods described in the following [A1] to [A5], methods equivalent to these methods, methods described in Examples, methods equivalent to those described in Examples, or methods described in Comprehensive Organic Transformations: A Guide to Functional Group Preparations, 2nd Edition (Richard C. Larock, John Wiley & Sons Inc., 1999), methods adapted from the foregoing or methods combining the foregoing without limitation. In the production methods described hereinbelow, raw material compounds may be those forming salts. Examples of the salts include those mentioned above as salts of the compound represented by the general formula (I).

Among the present compounds represented by the general formula (I), the compound wherein R¹ is a hydrogen atom, namely the compound represented by the general formula (IA): 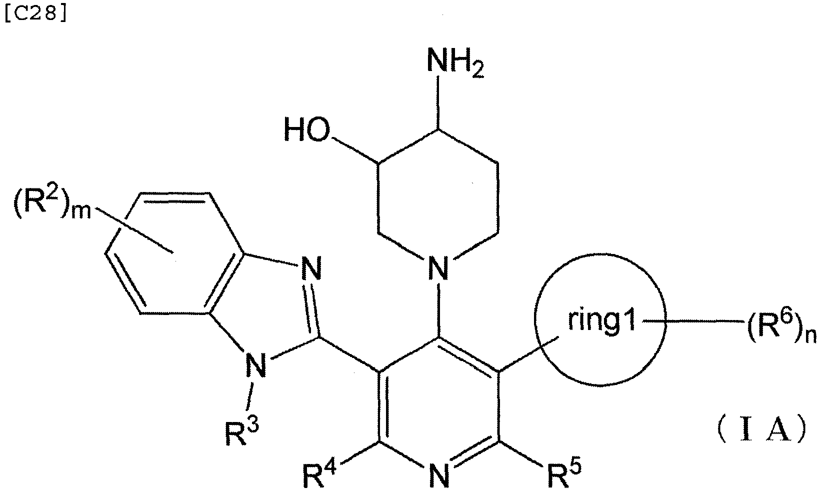 wherein all symbols have the same meanings as above, can be produced by subjecting a compound represented by the general formula (II): 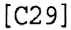 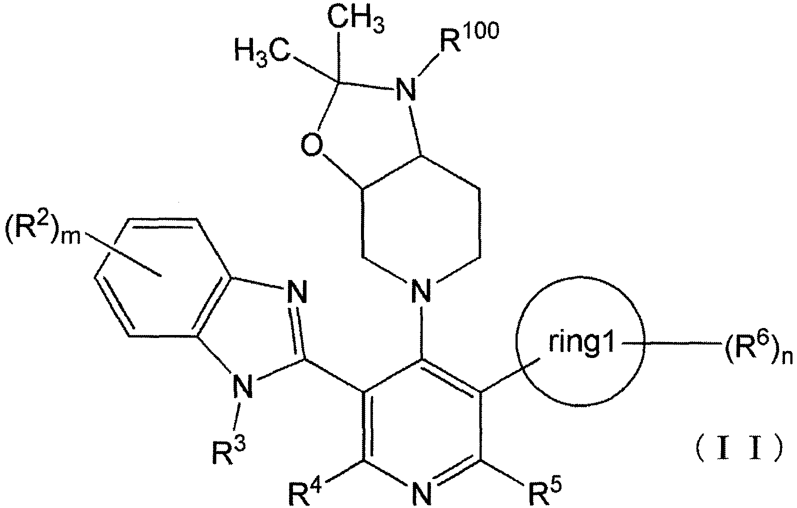 wherein R¹⁰⁰ represents a group deprotected under an acidic condition and other symbols have the same meanings as above, to a deprotection reaction under an acidic condition. Upon the reaction, a protection reaction and/or deprotection reaction of a functional group may be optionally carried out.

A deprotection reaction under an acidic condition may be carried out by, for example, in water of an organic solvent (examples: dichloromethane, chloroform, 1,4-dioxane, ethyl acetate, anisole or mixed solvents thereof) and in an organic acid (examples: acetic acid, trifluoroacetic acid, methanesulphonic acid and the like), an inorganic acid (examples: hydrochloric acid, sulphuric acid and the like) or a mixture thereof (examples: hydrogen bromide/acetic acid and the like) at a temperature of 0°C to 100°C.

Examples of the groups deprotected under an acidic condition include a t-butoxycarbonyl (Boc) group, a 2,2,2,-trichloroethoxycarbonyl group, a 2-trimethylsilylethoxycarbonyl group, an allyloxycarbonyl (Alloc) group, a vinyloxycarbonyl group, a benzyloxycarbonyl (Cbz) group, a 1-methyl-1-(4-biphenyl)ethoxycarbonyl (Bpoc) group, a formyl group, a benzoyl group, a p-methoxybenzyl group, a benzyloxymethyl (BOM) group, a 2-(trimethylsilyl)ethoxymethyl (SEM) group, a triphenylmethyl group, a p-toluenesulphonyl group and the like.

The group deprotected under an acidic condition is not particularly limited as long as the group can be easily and selectively eliminated and may be those other than described above. For example, the groups disclosed in Protective Groups in Organic Synthesis (T. W. Greene, John Wiley & Sons Inc, 1999) may be used.

Being easily understood by a person skilled in the art, a desired present compound can be easily produced by using the deprotection reactions according to need.

If necessary, the reaction may be followed by a well-known method for converting the compound to a desired salt.

Among the present compounds represented by the general formula (I), the compound wherein R¹ is 3-oxetanyl, 3,3,3-trifluoropropyl or ethyl, namely the compound represented by the general formula (IB): 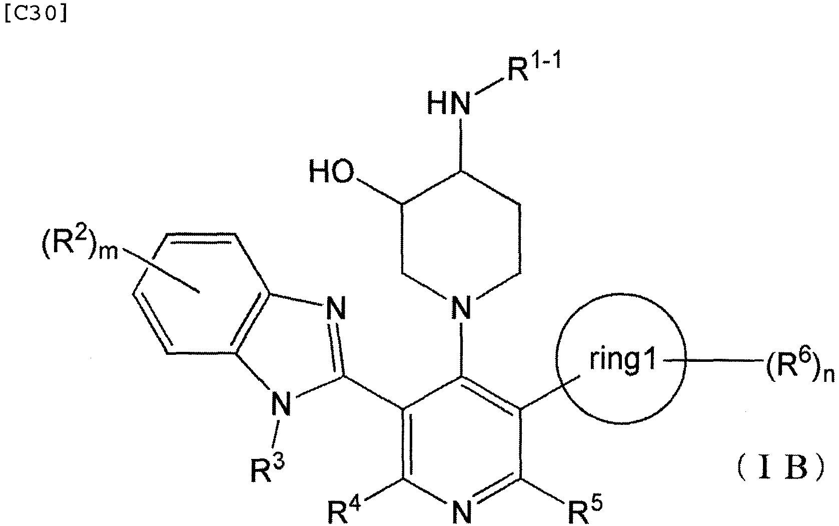 wherein R¹⁻¹ represents 3-oxetanyl, 3,3,3-trifluoropropyl or ethyl and other symbols have the same meanings as above, can be produced by subjecting the compound represented by the general formula (IA) and 3-oxetanone, 3,3,3-trifluoropropanal or acetaldehyde to a reductive amination reaction. Upon amination, a protection reaction and/or deprotection reaction of a functional group may be carried out.

The reductive amination reaction is well known. An imine produced by the reaction may be isolated before reduction, or an imine is produced in the reaction system and may be reduced without isolation (in one-pot). The imine production reaction is well known and can be carried out, for example, in an organic solvent (examples: methanol, ethanol, dichloromethane, chloroform, dichloroethane, benzene, toluene or mixed solvents thereof) in the presence or absence of a dehydrating agent (examples: anhydrous magnesium sulphate, Molecular Sieve (product name) and the like) and in the presence or absence of an acid (examples: hydrochloric acid, acetic acid and the like) at 20°C to reflux temperature. The reduction reaction of an imine is also well known and can be carried out, for example, in an organic solvent (examples: tetrahydrofuran, diethyl ether, dichloroethane, dichloromethane, dimethylformamide, acetic acid, methanol, ethanol or mixtures thereof), in the presence of a reducing agent (examples: sodium triacetoxyborohydride, sodium cyanoborohydride, sodium borohydride, zinc borohydride, diisobutylaluminium hydride, 2-picoline-borane complex and the like) at a temperature of 0°C to 40°C, or in a solvent (examples: ether solvents (examples: tetrahydrofuran, dioxane, dimethoxyethane, diethyl ether and the like), alcoholic solvents (examples: methanol, ethanol and the like), benzene solvents (examples: benzene, toluene and the like), nitrile solvents (examples: acetonitrile and the like), amide solvents (examples: dimethylformamide and the like), water, ethyl acetate, acetic acid or mixed solvents thereof), in the presence of a catalyst (examples: palladium-carbon, palladium black, palladium hydroxide, platinum oxide, Raney nickel and the like) under normal or increased pressure in a hydrogen atmosphere at a temperature of 0°C to 200°C. The reductive amination reaction carried out without isolation of an imine is well known, and can be carried out, for example, in an organic solvent (examples: dichloroethane, dichloromethane, dimethylformamide, acetic acid or mixtures thereof) in the presence of a reducing agent (examples: sodium triacetoxyborohydride, sodium cyanoborohydride, sodium borohydride, 2-picoline-borane complex and the like) at a temperature of 0°C to 40°C.

Alternatively, the reaction can be carried out by, for example, in an organic solvent (examples: dichloroethane, dichloromethane or mixed solvents thereof) in the presence of a tertiary amine (examples: triethylamine, diisopropylethylamine and the like), using a Lewis acid (examples: titanium tetrachloride and the like) at 0°C to 40°C and then in the presence of a reducing agent (examples: sodium triacetoxyborohydride, sodium cyanoborohydride, 2-picoline-borane complex and the like) at a temperature of 0°C to 40°C.

If necessary, the reaction may be further followed by a well-known method for converting the compound to a desired salt.

The compound represented by the general formula (II) can be produced according to the method illustrated in the following reaction scheme 1. In the reaction scheme 1, X¹ represents bromine, chlorine, an iodine, mesylate or triflate and other symbols have the same meanings as above.

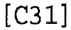

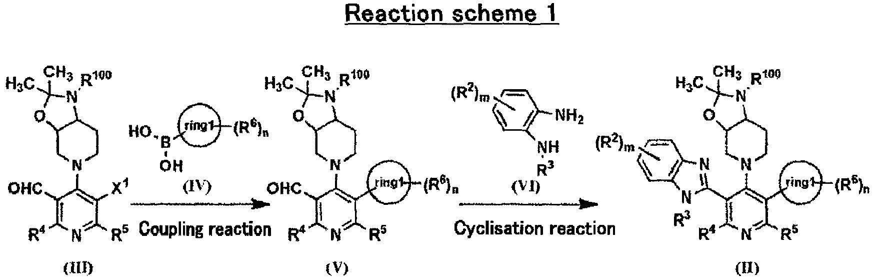

The compound represented by the general formula (V) can be produced by subjecting the compound represented by the general formula (III) and the compound represented by the general formula (IV) to a coupling reaction.

The coupling reaction is well known and can be carried out by, for example, reacting in an organic solvent (examples: benzene, toluene, dimethylformamide, 1,4-dioxane, tetrahydrofuran, methanol, acetonitrile, dimethoxyethane, acetone or mixed solvents thereof), with a base (examples: sodium ethylate, sodium hydroxide, potassium hydroxide, triethylamine, sodium carbonate, sodium hydrogen carbonate, potassium carbonate, caesium carbonate, thallium carbonate, tripotassium phosphate, caesium fluoride, barium hydroxide, tetrabutylammonium fluoride and the like) or aqueous solutions thereof or mixtures thereof in the presence of a catalyst (examples: bis(di-tert-butyl(4-dimethylaminophenyl)phosphine)dichloropalladium(II) ((A-taPhos)₂PdCl₂), tetrakis(triphenylphosphine)palladium (Pd(PPh₃)₄), bis(triphenylphosphine)palladium dichloride (PdCl₂(PPh₃)₂), palladium acetate (Pd(OAc)₂), palladium black, 1,1'-bis(diphenylphosphinoferrocene)dichloropalladium (PdCl2(dppf)2), diallylpalladium dichloride (PdCl₂(allyl)₂), iodophenyl bis(triphenylphosphine)palladium (PhPdI(PPh₃)₂) and the like) at room temperature to 150°C.

The compound represented by the general formula (II) can be produced by subjecting the compound represented by the general formula (V) and the compound represented by the general formula (VI) to a cyclisation reaction.

The cyclisation reaction is well known and can be carried out by, for example, reacting in an organic solvent (examples: ethanol, N,N-dimethylformamide, N,N-dimethylacetamide, dimethyl sulfoxide or mixed solvents thereof) in the presence of an acid (examples: acetic acid, sodium hydrogen sulfite and the like) or sodium dithionite at room temperature to reflux temperature.

Among the compounds represented by the general formula (III), the compound wherein R⁴ is a C1-4 alkyl, a C5-6 monocyclic carbocycle which may be substituted with 1 to 3 R⁷ groups or a 5- to 6-membered monocyclic heterocycle which may be substituted with 1 to 3 R⁸ groups, namely the compound represented by the general formula (III-1) can be produced according to the method illustrated in the following reaction scheme 2. In the reaction scheme 2, X² represents bromine or iodine, X³ represents chlorine, R⁴⁻¹ represents a Cl-4 alkyl, a C5-6 monocyclic carbocycle which may be substituted with 1 to 3 R⁷ groups or a 5- to 6-membered monocyclic heterocycle which may be substituted with 1 to 3 R⁸ groups and other symbols have the same meanings as above.

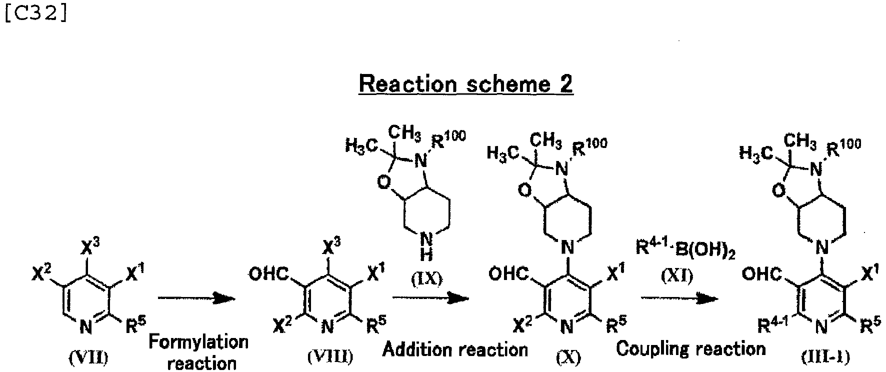

The compound represented by the general formula (VIII) can be produced by subjecting the compound represented by the general formula (VII) to a formylation reaction.

The formylation reaction is well known and can be carried out by, for example, adding, in an organic solvent (examples: tetrahydrofuran, diethyl ether or mixed solvents thereof) in the presence of a base (examples: lithium diisopropylamide, n-butyllithium and the like), a formylating agent (examples: N,N-dimethylformamide, methyl formate, ethyl formate and the like) and reacting at -78°C to room temperature.

The compound represented by the general formula (X) can be produced by subjecting the compound represented by the general formula (VIII) and the compound represented by the general formula (IX) to an addition reaction.

The addition reaction is well known and can be carried out by, for example, reacting in an organic solvent (examples: N,N-dimethylacetamide, N,N-dimethylformamide, tetrahydrofuran, acetonitrile, 2-propanol, dimethyl sulfoxide or mixed solvents thereof) in the presence of a base (examples: triethylamine, N,N-diisopropylethylamine, sodium hydrogen carbonate, potassium carbonate, tripotassium phosphate and the like) at room temperature to 120°C.

The compound represented by the general formula (III-1) can be produced by subjecting the compound represented by the general formula (X) and the compound represented by the general formula (XI) to a coupling reaction. The compound represented by the general formula (IX) is a well-known compound, or can be produced according to well-known methods, for example, according to the disclosure in Japanese Patent Application Publication No. 2009-155283 or a modification thereof.

The coupling reaction is well known and can be carried out under similar conditions as the method for producing the compound represented by the general formula (V).

Among the compounds represented by the general formula (III), the compound wherein R⁴ is a hydrogen atom, namely the compound represented by the general formula (III-2) can be produced according to the method illustrated in the following reaction scheme 3. In the reaction scheme 3, all symbols have the same meanings as above.

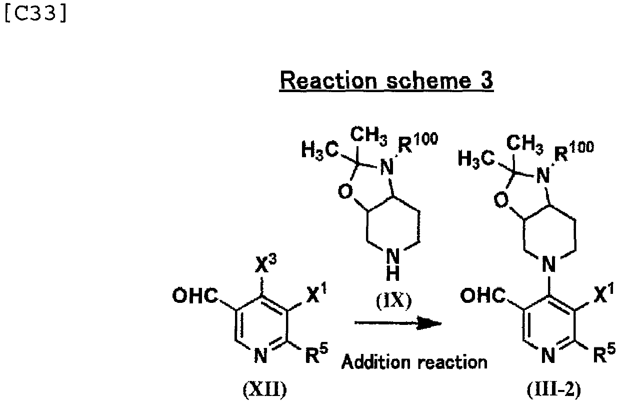

The compound represented by the general formula (III-2) can be produced by subjecting the compound represented by the general formula (XII) and the compound represented by the general formula (IX) to an addition reaction.

The addition reaction is well known and can be carried out under the similar conditions as the method for producing the compound represented by the general formula (X).

Among the present compounds, optically active compounds may be produced with an optically active starting material or reagent, or produced by resolving a racemic intermediate and leading the resolved product to the present compound or by resolving a racemic present compound.

The method for resolution is well known and examples thereof include a method in which a salt or a chelate is formed with a different optically active compound followed by recrystallisation and isolation of a desired compound or a method in which separation is directly carried out with a chiral column and the like.

The starting compounds, i.e. the compounds represented by the general formulae (III), (IV), (VI), (VII), (IX), (XI) and (XII), are well known *per se,* or can be readily produced by methods well known *per se* or a combination of methods described in, for example, Comprehensive Organic Transformations: A Guide to Functional Group Preparations, 2nd Edition (Richard C. Larock, John Wiley & Sons Inc., 1999) and the like.

In the reactions exemplified herein, any heating means such as water bath, oil bath, sand bath and microwave may be used.

In the reactions exemplified herein, a solid phase-supported reagent supported on a polymer (such as polystyrene, polyacrylamide, polypropylene and polyethylene glycol) may be used, if appropriate.

The products from the reactions exemplified herein may be purified by a conventional purification means, for example, distillation under normal or reduced pressure, chromatography (such as high performance liquid chromatography, thin layer chromatography or column chromatography) using silica gel, an ion exchange resin, an scavenger resin or magnesium silicate, or by washing or recrystallization. Purification may be carried out after each reaction step or after a series of reactions.

### [Toxicity]

The present compound has low toxicity and thus can be safely used as a medicament.

### [Application to medicaments]

The present compound has somatostatin receptor agonistic activity, and thus can be used as an agent for prophylaxis and/or therapy of somatostatin-related diseases (diseases in which somatostatin *per se* or a hormone modulated by somatostatin is involved) in mammals, particularly in humans.

Examples of such diseases include hormonal diseases (examples: acromegaly, gigantism, pituitary gigantism, Cushing's disease, Graves' disease, hyperthyroidism and the like), ateliosis (examples: skeletal dysplasia, Noonan syndrome, obesity, ateliosis accompanying obesity, uterine hypoplasia, kidney failure accompanying ateliosis, syndrome X and the like), cancer or adenoma (examples: leukaemia, chondrosarcoma, melanoma, lipoma, meningioma, neuroblastoma, pituitary adenoma, headache accompanying pituitary adenoma, growth hormone-secreting adenoma, growth hormone-releasing factor-secreting adenoma, gonadotropin-secreting adenoma, prolactinoma, thyrotropinoma, VIPoma, ACTH-secreting adenoma, thyroid cancer, medullary thyroid cancer, lung cancer, breast cancer, liver cancer, gastrointestinal and pancreatic neuroendocrine adenoma, gastrinoma, carcinoid syndrome, colorectal cancer, pancreatic cancer, islet cell carcinoma, insulin-secreting carcinoma, glucagonoma, prostate cancer, cancerous cachexia, colorectal haemangioma and the like), gastrointestinal diseases (examples: gastrointestinal symptoms accompanying gastrointestinal obstruction, gastroesophageal reflux disease, gastroduodenal reflux disease, excess gastric acid secretion, peptic ulcer, Zollinger-Ellison syndrome, protein-losing gasteroenteropathy, dumping syndrome, short bowel syndrome, inflammatory bowel disease, Crohn's disease, irritable bowel syndrome, irritable colon syndrome, enterocutaneous fistula, functional dyspepsia, nausea, vomiting, bloating and the like), diarrhea (examples: watery diarrhoea syndrome, chronic secondary diarrhoea, chemotherapy-induced diarrhoea, intractable diarrhoea accompanying acquired immune deficiency syndrome, diarrhoea accompanying irritable bowel syndrome, diarrhoea after surgery and the like), vascular diseases (examples: proliferative retinopathy, macular degeneration, age-related macular degeneration, gastrointestinal bleeding, bleeding accompanying gastroduodenal ulcer, bleeding oesophageal varices, bleeding varices of cirrhosis patients, portal hypertension, bleeding vascular graft, restenosis, scarring, psoriasis, systemic sclerosis (scleroderma), chronic rejection of allografts, hypotension, atherosclerosis, post-PTCA restenosis, hypertrophic cardiomyopathy, arteriosclerosis, cardiac valvulopathy, myocardial infarction and the like), fibrosis (examples: skin fibrosis, central nerve system fibrosis, nasal fibrosis, pulmonary fibrosis, hepatic fibrosis, kidney fibrosis, chemotherapy-induced fibrosis and the like), diabetes and diabetic complications (examples: diabetes, insulin-dependent diabetes, diabetic retinopathy, diabetic nephropathy, diabetic neuropathy, dawn phenomenon, insulin resistance, hyperinsulinemia, hyperlipidaemia and the like), inflammatory diseases (examples: arthritis, rheumatoid arthritis, psoriasis, localized inflammation, sunburn, eczema and the like), central nervous system diseases (examples: dementia, Alzheimer's disease, epilepsy and the like), respiratory diseases (examples: sleep apnoea syndrome and the like), pancreatic diseases (examples: pancreatitis, acute pancreatitis, chronic pancreatitis, pancreatic cutaneous fistula, pancreatic pseudocyst, ascites, pancreatic fistula, symptoms related to pancreatic surgery and the like), hepatic diseases (examples: hepatic cyst and the like), renal diseases (examples: hepatorenal syndrome, renal cyst, nephropathy and the like), ovarian diseases (examples: polycystic ovary syndrome and the like), bone and joint diseases (examples: osteoporosis, osteoarthritis and the like), pain, headache and the like. The present compound may also be used for, after introducing a radioactive substance (examples: ¹²³I, ¹²⁵I, ¹¹¹In and the like) to the compound directly or via an appropriate spacer, imaging of tumours having somatostatin receptors. The present compound may be used for, after introducing an antitumor drug to the compound directly or via an appropriate spacer, targeting tumours having somatostatin receptors.

Among others, the present compound is useful for prophylaxis and/or therapy of acromegaly, gigantism, pituitary gigantism, pituitary adenoma, headache accompanying pituitary adenoma, growth hormone-secreting adenoma, gastrointestinal and pancreatic neuroendocrine adenoma, gastrinoma, carcinoid syndrome, insulin-secreting carcinoma, glucagonoma, gastrointestinal symptoms accompanying gastrointestinal obstruction, renal cyst, hepatic cyst, bleeding oesophageal varices, portal hypertension, diabetic retinopathy, dementia, Alzheimer's disease, pain and headache. The present compound is particularly suitable for prophylaxis and/or therapy of acromegaly and gastrointestinal symptoms accompanying gastrointestinal obstruction.

Examples of the gastrointestinal symptom accompanying gastrointestinal obstruction include gastrointestinal symptoms accompanying gastrointestinal obstruction (malignant gastrointestinal obstruction in advanced and recurrent cancer patients) in palliative care of advanced and recurrent cancer patients, which may be ameliorated by the present compound.

Examples of polycystic kidney include autosomal dominant polycystic kidney and autosomal recessive polycystic kidney.

Examples of test methods other than those described in Examples for evaluating the pharmacological activity of the present compounds include an evaluation system of suppression of gastric acid secretion using rats. For example, suppression of gastric acid secretion by the present compounds may be evaluated by using the following method.

### (Evaluation of suppression of gastric acid secretion using rats)

Rats (7-week old male Crl:CD(SD) IGS rats (Charles River Laboratories Japan, Inc.)) are fasted overnight and deprived of water over 2 hours before the evaluation. An indwelling needle is placed in the tail of rats anesthetised with isoflurane. After they awake, rats are continuously and intravenously administered with a medium (saline (Otsuka Normal Saline, Otsuka Pharmaceutical Factory, Inc.)) or a test compound dissolved in the medium through the indwelling needle. One hour after initiation of administration, the abdomens of rats are opened under isoflurane anaesthesia and the gastric pylorus is ligated with a thread. After closing the abdomen, rats are awoken. At 5 hours after initiation of administration (4 hours after pyloric ligation), the abdomen of rats are opened again under isoflurane anaesthesia, the gastric cardia is clamped with forceps and the rats are sacrificed by bleeding. The gastric content is centrifuged at 500 × g for 15 minutes, the supernatant is recovered as the gastric juice and the amount of the gastric juice per body weight (mL/100 g-BW) is determined. The acid concentration (mmol/mL) of the gastric juice is determined by back titration using a COM-1600ST automatic titrator (Hitachi High-Technologies Corporation (Hiranuma Sangyo Co., Ltd.)). The product of the amount of the gastric juice and the acid concentration is determined as the amount of gastric acid secretion (mmol/100 g-BW), and then the percentage (%) of suppression of gastric acid secretion is determined according to the equation: {[percentage of suppression of gastric acid secretion (%)] = ([the amount of gastric acid secretion of the group administered with the medium] - [the amount of gastric acid secretion of the group administered with a test compound]) / [the amount of gastric acid secretion of the group administered with the medium] × 100}. The inventors of the present invention have found that according to this evaluation system, octreotide, for example, exhibits the percentage of suppression of gastric acid secretion of 39% at the administration rate of 1 µg/kg/h.

Other than the diseases listed above, the present compounds may also be used for prophylaxis and/or therapy of various pathological conditions in which somatostatin is involved, for example, diseases described in Life Sciences, 1987, Vol. 40, p. 419-437; and The European Journal of Medicine, 1993, Vol. 2, p. 97-105.

Upon using the present compound for pharmaceutical purposes, the present compound may be used not only as a single drug but also as a combined drug with an additional active component, for example those listed hereinbelow, for the purposes of, for example, (1) supplementing and/or enhancement of the effect thereof for prophylaxis, therapy and/or amelioration of symptoms, (2) improvement of the kinetics and absorption, reduction of the dosage thereof and/or (3) alleviation of side-effects thereof.

When the present compound is used for prophylaxis and/or therapy of acromegaly, examples of the drugs which may be used in combination with the present compound include somatostatin analogues, somatostatin receptor agonists, growth hormone receptor antagonists, dopamine receptor agonists and the like.

Patients with acromegaly are often associated with diseases related to lifestyle such as diabetes, hypertension, hyperlipidaemia and obesity and various other diseases. Therefore, the present compound may be used in combination with, for example, therapeutic agents for diabetes (examples: insulin-sensitising agents, insulin secretion promoting agents (examples: sulphonylurea and the like), biguanide, insulin, α-glucosidase inhibitors, β3 adrenaline receptor agonists, dipeptidyl peptidase IV inhibitors, amylin agonists, phosphotyrosine phosphatase inhibitors, glycogenesis inhibitors, SGLT (sodium-glucose co-transporter) inhibitors and other therapeutic agents for diabetes), therapeutic agents for diabetes complications (examples: aldose reductase inhibitors, glycation inhibitors, protein kinase C inhibitors, neurotrophic factors, neurotrophic factor increasing agents, nerve regeneration promoting agents and other therapeutic agents for diabetes complications), therapeutic agents for hypertension (examples: angiotensin-converting enzyme inhibitors, calcium antagonists, potassium channel openers, angiotensin II antagonists), therapeutic agents for hyperlipidaemia (examples: HMG-CoA reductase inhibitors, fibrate compounds, squalene synthase inhibitors, antioxidants and the like), anti-obesity agents (examples: pancreatic lipase inhibitors, central acting anti-obesity agents, peptidic appetite suppressants, cholecystokinin agonists and other anti-obesity agents), therapeutic agents for arthritis, anti-anxiety agents, antidepressants, therapeutic agents for osteoporosis, anti-epilepsy drugs, chemotherapeutic agents, immunotherapeutic agents, anti-PD-1 antibodies, antithrombotic agents, therapeutic agents for dementia, therapeutic agents for erectile dysfunction, therapeutic agents for urinary incontinence/frequent urination, therapeutic agents for dysuria, nonsteroidal antiinflammatory agents, local anaesthetics, vitamins and the like. The present compound may also be used in combination with hormones which promote secretion of other growth hormones (examples: GHRHs), GH, IGF-1, cytokines and agents for enhancing effects of cytokines.

When the present compound is used for prophylaxis and/or therapy of a gastrointestinal symptom accompanying gastrointestinal obstruction, examples of the drugs which may be used in combination with the present compound include somatostatin analogues, somatostatin receptor agonists, dopamine D2 receptor antagonists, histamine H1 receptor antagonists, concomitant drugs of histamine H1 receptor antagonists and PDE inhibitors, histamine H2 receptor antagonists, anticholinergic agents, serotonin 5HT3 receptor antagonists, serotonin 5HT4 receptor antagonists, corticosteroid, NK1 receptor antagonists, atypical antipsychotics (MARTAs), opioid, opioid antagonists and the like. The present compound may alternatively be used in combination with, for example, prochlorperazine, levomepromazine and the like.

Examples of the somatostatin analogues include octreotide, lanreotide, pasireotide and the like.

Examples of the somatostatin receptor agonists include compounds disclosed in WO 2002/091125, compounds disclosed in WO 2003/042234, compounds disclosed in WO 2003/045926, compounds disclosed in WO 2008/051272, compounds disclosed in WO 2004/046107, compounds disclosed in WO 2017/003723, compounds disclosed in WO 2017/003724 and the like.

Examples of growth hormone receptor antagonists include pegvisomant and the like.

Examples of dopamine receptor agonists include bromocriptine, cabergoline and the like.

Examples of insulin-sensitising agents include balaglitazone, netoglitazone, pioglitazone, rivoglitazone, rosiglitazone, farglitazar, muraglitazar, naveglitazar, ragaglitazar, tesaglitazar, reglixane, BM-13.1258, FK-614, KRP-297, LM-4156, LY-510929, MBX-102, MX-6054, R-119702, T-131, THR-0921, compounds disclosed in WO 2001/038325, compounds disclosed in WO 1999/058510 (such as (E)-4-[4-(5-methyl-2-phenyl-4-oxazolylmethoxy)benzyloxyimino]-4-phenylbutyric acid) and the like.

Examples of sulphonylurea include acetohexamide, chlorpropamide, glibenclamide, gliclazide, glimepiride, glipizide, glybuzole, glyclopyramide, mitiglinide, nateglinide, repaglinide, senaglinide, tolazamide, tolbutamide, JTT-608 and the like.

Examples of biguanide include buformin, fenformin, metformin and the like.

Examples of insulin include animal-derived insulin extracted from bovine or porcine pancreas, semi-synthetic human insulin synthesised from insulin extracted from porcine pancreas, human insulin synthesised by genetic engineering manners using *Escherichia coli* or yeasts, insulin-zinc containing 0.45 to 0.9 (w/w)% of zinc, protamine zinc-insulin produced from zinc chloride, protamine sulphate and insulin and the like. Insulin may be a fragment or derivative (examples: INS-1 and the like) thereof and may be an oral insulin formulation. Insulin includes various type such as ultra-rapid-acting, rapid-acting, biphasic, intermediate-acting and long acting insulins, all of which may be used by appropriately selecting the type according to the pathological conditions of patients.

Examples of α-glucosidase inhibitors include acarbose, emiglitate, miglitol, voglibose and the like.

Examples of β3 adrenaline receptor agonists include AJ-9677, AZ40140 and the like.

Examples of dipeptidyl peptidase IV inhibitors include sitagliptin, alogliptin, vildagliptin, linagliptin, anagliptin, saxagliptin, teneligliptin, bisegliptin, carmegliptin, evogliptin, omarigliptin, denagliptin, dutogliptin, gemigliptin, gosogliptin, melogliptin, NVP-DPP-728, PT-100, P32/98, TS-021, TA-6666, KRP-104, DSP-7238, SYR-472 (trelagliptin), TAK-100 and the like.

Examples of amylin agonists include pramlintide and the like.

Examples of phosphotyrosine phosphatase inhibitors include sodium vanadate and the like.

Examples of glycogenesis inhibitors include glycogen phosphorylase inhibitors, glucose-6-phosphatase inhibitors, glucagon antagonists and the like.

Examples of SGLT (sodium-glucose co-transporter) inhibitors include ipragliflozin, luseogliflozin, tofogliflozin, canagliflozin, dapagliflozin and the like.

Examples of therapeutic agents for diabetes other than those mentioned above include bromocriptine, leptin, BAY-27-9955, GLP-1 receptor agonists (examples: GLP-1, GLP-1MR, liraglutide, AC-2993 (exendin-4), BIM-51077, Aib(8,35)hGLP-1(7,37)NH₂, CJC-1131, exenatide and the like), GPR40 agonists (examples: TAK-875 and the like), GPR119 agonists, 11β-hydroxysteroid dehydrogenase inhibitors (examples: BVT-3498 and the like), adiponectin or agonists thereof, IKK inhibitors (examples: AS-2868 and the like), leptin-sensitising agents, somatostatin receptor agonists (examples: compounds disclosed in WO 2001/025228, WO 2003/042204, WO 1998/044921, WO 1998/045285, WO 1999/022735 and the like), glucokinase activating agents (examples: RO-28-1675 and the like) and the like.

Examples of aldose reductase inhibitors include tolrestat, epalrestat, imirestat, zenarestat, fidarestat, zopolrestat, minalrestat, ranirestat, CT-112 and the like.

Examples of glycation inhibitors include pimagedine, ALT-946, ALT766, EXO-226 and the like.

Examples of protein kinase C inhibitors include ruboxistaurin mesylate and the like.

Examples of neurotrophic factors include NGF, NT-3, BDNF and the like.

Examples of neurotrophic factor increasing agents include neurotrophin production/secretion-promoting agents disclosed in WO 2001/014372 (examples: 4-(4-chlorophenyl)-2-(2-methyl-1-imidazolyl)-5-[3-(2-methylphenoxy)propyl]oxazole and the like).

Examples of nerve regeneration promoting agents include Y-128, VX-853, prosaptide and the like.

Examples of other therapeutic agents for diabetic complications other than those mentioned above include alprostadil, tiapride, cilostazol, mexiletine, ethyl icosapentate, memantine, pimagedline, AGE inhibitors (examples: ALT-946, alagebrium, pyridorin, pyridoxamine and the like), reactive oxygen eliminating agents (examples: thioctic acid and the like), somatostatin receptor agonists (examples: BIM-23190), apoptosis signal regulating kinase-1 (ASK-1) inhibitors and the like.

Examples of angiotensin-converting enzyme inhibitors include captopril, enalapril, alacepril, delapril, lisinopril, imidapril, benazepril, cilazapril, temocapril, trandolapril and the like.

Examples of calcium antagonists include manidipine, nifedipine, amlodipine, efonidipine, nicardipine and the like.

Examples of potassium channel openers include levcromakalim, AL0671, NIP-121 and the like.

Examples of angiotensin II antagonists include losartan, candesartan cilexetil, eprosartan, valsartan, irbesartan, olmesartan medoxomil, E4177, 1-[[2'-(2,5-dihydro-5-oxo-4H-1,2,4-oxadiazol-3-yl)biphenyl-4-yl]methyl]-2-ethoxy-1H-benzimidazole-7-carboxylic acid and the like.

Examples of HMG-CoA reductase inhibitors include pravastatin, simvastatin, atorvastatin, fluvastatin, pitavastatin, rosuvastatin and the like.

Examples of fibrate compounds include bezafibrate, clinofibrate, clofibrate, simfibrate, fenofibrate and the like.

Examples of squalene synthase inhibitors include compounds disclosed in WO 1997/010224 (examples: N-[[(3R,5S)-1-(3-acetoxy-2,2-dimethylpropyl)-7-chloro-5-(2,3-dimethoxyphenyl)-2-oxo-1,2,3,5-tetrahydro-4,1-benzooxazepin-3-yl]acetyl]piperidine-4-acetic acid and the like) and the like.

Examples of antioxidants include lipoic acid, probucol and the like.

Examples of pancreatic lipase inhibitors include orlistat, cetilistat and the like.

Examples of central-acting anti-obesity agents include mazindol, dexfenfluramine, fluoxetine, sibutramine, fenfluramine, phentermine, amfepramone, dexamfetamine, phenylpropanolamine, clobenzorex and the like.

Examples of peptidic apetite suppressants include leptin, CNTFs (ciliary neurotrophic factors) and the like.

Examples of cholecystokinin agonists include lintitript, FPL-15849 and the like.

Examples of anti-obesity agents other than those mentioned above include lipstatin, MCH receptor antagonists (examples: SB-568849, SNAP-7941, compounds disclosed in WO 2001/082925, compounds disclosed in WO 2001/087834 and the like), neuropeptide Y antagonists (examples: CP-422935 and the like), cannabinoid receptor antagonists (examples: SR-141716, rimonabant and the like), ghrelin antagonists, 11β-hydroxysteroid dehydrogenase inhibitors (examples: BVT-3498 and the like), β3 agonists (examples: AJ-9677, AZ40140 and the like), antifeedants (examples: P-57 and the like) and the like.

Examples of therapeutic agents for arthritis include ibuprofen and the like.

Examples of anti-anxiety agents include chlordiazepoxide, diazepam, oxazolam, medazepam, cloxazolam, bromazepam, lorazepam, alprazolam, fludiazepam and the like.

Examples of antidepressants include fluoxetine, fluvoxamine, imipramine, paroxetine, sertraline and the like.

Examples of therapeutic agents for osteoporosis include alfacalcidol, calcitriol, elcatonin, calcitonin salmon, estriol, ipriflavone, risedronate disodium, pamidronate disodium, alendronate sodium hydrate, incadronate disodium and the like.

Examples of anti-epilepsy drugs include gabapentin, trileptal, keppra, zonegran, pregabalin, harkoseride, carbamazepine and the like.

Examples of chemotherapeutic agents include alkylating agents (examples: cyclophosphamide, ifosfamide and the like), anti-metabolites (examples: methotrexate, 5-fluorouracil, 5-fluorouracil derivatives (examples: doxifluridine and the like) and the like), anti-cancer antibiotics (examples: mitomycin, doxorubicin and the like), plant-derived anti-cancer agents (examples: vincristine, vindesine, paclitaxel and the like), cisplatin, carboplatin, etoposide and the like.

Examples of immunotherapeutic agents include components of microorganisms or bacteria (examples: muramyl dipeptide derivatives, picibanil and the like), polysaccharides having immunostimulating activity (examples: lentinan, sizofiran, Krestin® and the like), cytokines obtained by genetic engineering procedures (examples: interferons, interleukins (IL) (examples: IL-1, IL-2, IL-12 and the like) and the like), colony-stimulating factors (examples: granulocyte colony-stimulating factors, erythropoietin (EPO) and the like) and the like.

Examples of anti-PD-1 antibodies include nivolumab, pembrolizumab and the like.

Examples of antithrombotic agents include heparin (examples: dalteparin, heparin and the like), warfarin (examples: warfarin and the like), antithrombins (examples: argatroban and the like), thrombolytic agents (examples: urokinase, tisokinase, alteplase, nateplase, monteplase, pamiteplase and the like), platelet aggregation inhibitors (examples: ticlopidine, cilostazol, ethyl icosapentate, beraprost, sarpogrelate and the like) and the like.

Examples of the therapeutic agents for dementia include donepezil, galanthamine, rivastigmine, tacrine and the like.

Examples of therapeutic agents for erectile dysfunction include apomorphine, sildenafil and the like.

Examples of therapeutic agents for urinary incontinent/frequent urination include flavoxate, imidafenacin, oxybutynin, propiverine and the like.

Examples for therapeutic agents for dysuria include acetylcholine esterase inhibitors (examples: distigmine and the like) and the like.

Examples of nonsteroidal antiinflammatory agents include acetaminophen, aspirin, indomethacin and the like.

Examples of local anaesthetics include capsaicin, lidocaine and the like.

Examples of vitamins include vitamin B1, vitamin B12 and the like.

Examples of dopamine D2 receptor antagonists include prochlorperazine, levomepromazine, risperidone, metoclopramide, domperidone and the like.

Examples of histamine H1 receptor antagonists include diphenhydramine, chlorpheniramine, dimenhydrinate, promethazine and the like.

Examples of concomitant drugs of histamine H1 receptor antagonists with PDE inhibitors include diphenhydramine/diprophylline concomitant drugs and the like.

Examples of histamine H2 receptor antagonists include famotidine, cimetidine and the like.

Examples of anticholinergic agents include scopolamine and the like.

Examples of serotonin 5HT3 receptor antagonists include tropisetron, granisetron, ondansetron, azasetron, ramosetron, indisetron, palonosetron and the like.

Examples of serotonin 5HT4 receptor antagonists include cisapride, mosapride and the like.

Examples of corticosteroid include dexamethasone, betamethasone, prednisolone and the like.

Examples of NK1 receptor antagonists include aprepitant, fosaprepitant and the like.

Examples of atypical antipsychotics (MARTAs) include olanzapine, quetiapine, perospirone and the like.

Examples of opioid include morphine and the like.

Examples of opioid antagonists include methylnaltrexone and the like.

The combined drug of the present compound and an additional drug may be administered in the form of a concomitant drug containing both components in one formulation, or separate formulations may be administered by the same or different routes of administration. It is not necessary that separate formulations are administered simultaneously and separate formulations may be administered sequentially with a time difference. When the formulations are sequentially administered, the order or administration is not particularly limited and may be appropriately adjusted so that desired efficacy of drugs can be obtained.

The dosage of the additional drug which is used in combination with the present compound may be appropriately increased or decreased according to the clinical dosage thereof or a similar drug. The ratio between the present compound and the additional drug may be appropriately adjusted by considering the age and weight of the subject, the administration method, the time of administration, the target disease and condition and the like. Generally, 1 part by weight of the present compound may be combined with the additional drug in an amount ranging from 0.01 to 100 parts by weight. A plurality of the additional drug may be used. The additional drug may be, in addition to those mentioned above, a drug having the same mechanism as those mentioned above. Such an additional drug includes not only the one which has been discovered by now but also the one which will be discovered in future.

The dosage of the present compound may vary according to the age, weight, condition, therapeutic effect, administration method, treatment period and the like. The present compound may be orally administered to an adult once to several times daily at the amount of 0.1 mg to 300 mg per administration, parenterally administered to an adult once to several times daily at the amount of 0.1 mg to 150 mg per administration or intravenously and continuously administered over 1 hour to 24 hours daily.

As described above, the dosage may vary according to various conditions, and thus the amount less than the dosage described above may be sufficient in some cases and the amount exceeding the above dosage may be required in other cases.

When the present compound is used for prophylaxis and/or therapy of the above diseases as a single drug or a combined drug with the additional drug, the present substance which is an active component is generally formulated with a pharmaceutically acceptable carrier such as various additives or solvents and the obtained formulation is administered systemically or locally and orally or parenterally. The pharmaceutically acceptable carrier as used herein means a substance other than an active component that is generally used for medicinal formulations. The pharmaceutically acceptable carrier preferably does not exhibit pharmacological activity, is harmless and does not prevent the therapeutic effect of the active component at the dosage of the formulation. The pharmaceutically acceptable carrier may also be used in order to increase the usefulness of the active component and the formulation, to facilitate production of the formulation, to stabilize the quality or to improve the usability. Specifically, the substances described in "Iyakuhin Tenkabutsu Jiten", 2000, Yakuji Nippo Ltd. (Ed. IPEC Japan) may be appropriately selected according to the need.

Examples of the dosage form include oral administration formulations (examples: tablets, capsules, granules, powders, oral liquids, syrups, oral jelly formulations and the like), oral cavity formulations (examples: tablets for the oral cavity, spray formulations for the oral cavity, semi-solid formulations for the oral cavity, oral rinse and the like), formulations for injection (examples: injections and the like), formulations for dialysis (examples: agents for dialysis and the like), formulations for inhalation (examples: agents for inhalation and the like), ophthalmic formulations (examples: ophthalmic solutions, ophthalmic ointments and the like), otological formulations (examples: ear drops and the like), nasologic formulations (examples: nasal drops and the like), rectal formulations (examples: suppositories, semi-solid formulations for rectal administration, enema formulations and the like), vaginal formulations (examples: vaginal tablets, vaginal suppositories and the like), skin formulations (examples: topical solid formulations, topical liquids, spray formulations, ointments, creams, gels, plasters and pressure sensitive adhesives and the like) and the like.

### [Oral administration formulations]

Examples of an oral administration formulation include tablets, capsules, granules, powders, oral liquids, syrups, oral jelly formulations and the like. The oral administration formulation may be classified into rapidly disintegrating formulations for which the release of an active component from the formulations is not particularly controlled and release-controlled formulations for which the release is controlled according to the purposes by adjusting the dosage design and production method, such as enteric formulations and sustained release formulations. The enteric formulations refer to a formulation which is designed to release an active component mainly in the small intestine rather than in the stomach with the purpose of prevention of decomposition of the active component in the stomach or reduction of stimulation of the stomach by the active component. The enteric formulation may be generally produced by providing a coating of an acid-insoluble enteric base. The sustained release formulations refer to a formulation for which the release rate, release time and release site of an active component from the formulation is controlled with the purpose of reduction in the frequency of administration or reduction of side effects. The sustained release formulation may be generally produced by using an appropriate agent for sustained release. Among the oral administration formulations, capsules, granules, tablets may be provided with an appropriate coating film of a saccharide, sugar alcohol, polymer compound and the like with the purpose of easy ingestion or prevention of decomposition of an active component.

### (1) Tablets

Tablets are an orally administered solid formulation having a certain shape. Examples thereof include those generally referred to as tablets such as plain tablets, film-coated tablets, sugar-coated tablets, multilayered tablets and dry-coated tablets as well as orally disintegrating tablets, chewable tablets, effervescent tablets, dispersible tablets, soluble tablets and the like. Plain tablets may be generally produced according to the following procedure (a), (b) or (c):
(a) An active component is mixed with an additive such as a vehicle, a binding agent and a disintegrating agent to obtain a homogeneous mixture which is granulated by an appropriate method using water or a solution containing a binding agent, mixed with a lubricant and the like, compressed and moulded;
(b) An active component is mixed with an additive such as a vehicle, a binding agent and a disintegrating agent to obtain a homogeneous mixture which is then directly compressed and moulded, or granules prepared with an additive are mixed with an active component, a lubricant and the like to obtain a homogeneous mixture which is then compressed and moulded;
(c) An active component is mixed with an additive such as a vehicle and a binding agent to obtain a homogeneous mixture which is then wetted and kneaded with a solvent, moulded in a certain mould and dried by an appropriate method. Film-coated tablets may be generally produced by providing appropriate thin coating films of a polymer and the like to plain tablets. Sugar-coated tablets may be generally produced by providing coating films containing a saccharide or sugar alcohol to plain tablets. Multilayerd tablets may be produced by stacking layers of powder granules having different compositions and compressing and moulding the product according to an appropriate method. Dry-coated tablets may be produced by coating inner core tablets with outer layers having different compositions. Tablets may be formed as enteric tablets or sustained release tablets according to appropriate well-known methods. Orally disintegrating tablets, chewable tablets, effervescent tablets, dispersible tablets and soluble tablets are the tablets to which unique functions are imparted by appropriately selecting additives, and may be produced according to the production procedures described above for the tablets. Orally disintegrating tablets refer to a tablet ingested by rapid dissolution or disintegration in the oral cavity; chewable tablets refer to a tablet ingested by chewing; effervescent tablets refer to a tablet which is dissolved or dispersed in water with rapid effervescence; dispersible tablets refer to a tablet which is ingested after dispersion in water; and the soluble tablets refer to a tablet which is ingested after dissolution in water. The effervescent tablets may be produced by using an additive which is an appropriate acidic substance, carbonate salt, hydrogen carbonate salt and the like.

### (2) Capsules

Capsules are a formulation containing a capsule shell filled with an active component or an active component coated with a capsule base. Examples thereof include hard capsules, soft capsules and the like. Hard capsules may be produced by mixing an active component with an additive such as a vehicle to obtain a homogeneous mixture, or obtaining granules or moulded substance by an appropriate method, which is then directly, or after appropriately being moulded, added to a capsule shell. Soft capsules may be produced by capsulating and moulding a mixture of an active component and an additive into a certain shape with an appropriate capsule base such as gelatine having an increased plasticity by addition of glycerol, D-sorbitol or the like. Capsules may be formed as enteric capsules or sustained release capsules according to appropriate well-known methods. An capsule base may be added with a colorant, a preservative or the like.

### (3) Granules

Granules are a granulated formulation. Examples thereof include those generally referred to as granules as well as effervescent granules. Granules may be generally produced according to the following procedure (a), (b) or (c):
(a) A powder active component is mixed with an additive such as a vehicle, a binding agent or a disintegrating agent to obtain a homogeneous mixture which is then granulated by an appropriate method;
(b) A granulated active component is mixed with an additive such as a vehicle to obtain a homogeneous mixture;
(c) A granulated active component is mixed with an additive such as a vehicle to obtain a homogeneous mixture which is then granulated by an appropriate method. Granules may be optionally provided with a film or may be formed as enteric granules or sustained release granules using appropriate well-known methods. Effervescent granules may be produced by using an additive which is an appropriate acidic substance, carbonate salt, hydrogen carbonate salt and the like. The effervescent granules refer to a granule which is dissolved or dispersed in water with rapid effervescence. The granules may also be formed as fine granules by controlling the particle size.

### (4) Powders

Powders are powdery formulations and may be generally produced by mixing an active component with an additive such as a vehicle to obtain a homogeneous mixture.

### (5) Oral liquids

Oral liquids are a formulation in the form of solution or flowable and viscous gel. Examples thereof include those generally referred to as oral liquids as well as elixirs, suspensions, emulsions, lemonades and the like. Oral liquids may be generally produced by mixing an active component with an additive and purified water to homogeneously dissolve, emulsify or suspend the active component and optionally filtering the product. Elixirs refer to a clear oral liquid containing ethanol having sweet taste and aroma. Elixirs may be generally produced by dissolving a solid active component or an infusion thereof in ethanol, purified water, a flavouring agent and sucrose, an additional saccharide or a sweetening agent and obtaining a clear liquid by filtration or other methods. Suspensions refer to an oral liquid in which an active component is finely and homogeneously suspended. Suspensions may be generally produced by suspending a solid active component in a suspending agent or an additional additive and purified water or oil and homogenising the whole product according to an appropriate method. Emulsions refer to an oral liquid in which an active component is finely and homogeneously emulsified. Emulsions may be generally produced by adding an emulsifying agent and purified water to a liquid active component and emulsifying and homogenising the whole product according to an appropriate method. Lemonades refer to a clear oral liquid having sweet taste and sour taste.

### (6) Syrups

Syrups are a viscous liquid or solid formulation containing a saccharide or a sweetening agent. Examples thereof include agents for syrups. Syrups may be generally produced by dissolving, mixing, suspending or emulsifying an active component in a solution of sucrose, other saccharides or a sweetening agent or solely a syrup and optionally boiling the product followed by filtering while heating. Formulations for syrups refer to a granular or powdery formulation to which water is added to provide syrups and may be sometimes referred to as dry syrups. Formulations for syrups may be generally produced according to the production procedures described above for the granules or powders by using a saccharide or a sweetening agent as an additive.

### (7) Oral jelly formulations

Oral jelly formulations are a shaped gel formulation without flowability. Oral jelly formulations may be generally produced by mixing an active component with an additive and a polymer gel base, allowing formation of gel and shaping into a certain shape according to appropriate methods.

### [Oral cavity formulations]

### (1) Tablets for the oral cavity

Tablets for the oral cavity are a formulation having a certain shape which is administered to the oral cavity. Examples thereof include troches, sublingual tablets, buccal tablets, adhering tablets, chewing gum tablets and the like. Tablets for the oral cavity may be generally produced according to the production procedures described for the tablets. Troches refer to a tablet for the oral cavity which is gradually dissolved or disintegrated in the oral cavity and is applied locally to the oral cavity or pharynx; sublingual tablets refer to a tablet for the oral cavity to be rapidly dissolved under the tongue to allow absorption of an active component through oral mucosa; buccal tablets refer to a tablet for the oral cavity to be gradually dissolved between the molars and cheeks to allow absorption of an active component through oral mucosa; adhering tablets refer to a tablet for the oral cavity which is adhered to oral mucosa; and chewing gum tablets refer to a tablet for the oral cavity to be chewed to release an active component.

### (2) Spray formulations for the oral cavity

Spray formulations for the oral cavity are a formulation to spray an active component in the form of mist, powder, foam or paste. Spray formulations for the oral cavity may be generally produced by dissolving or suspending an active component and an additive in a solvent or the like, optionally filtering thereof and packing the product into a container together with liquefied gas or compressed gas, or by preparing a solution or suspension with an active component and an additive and packing the product into a container to which a spraying pump is attached.

### (3) Semi-solid formulations for the oral cavity

Semi-solid formulations for the oral cavity are a formulation to be applied to the oral mucosa. Examples thereof include creams, gels, ointments and the like. Semi-solid formulations for the oral cavity may be generally produced by emulsifying an active component together with an additive in purified water and an oil component such as petrolatum, or by mixing an active component and an additive with a base such as a polymer gel or an oil or fat and obtaining a homogeneous mixture. Creams refer to a semi-solid formulation in the form of an oil-in-water or water-in-oil emulsion and lipophilic formulations in the form of a water-in-oil emulsion may also be referred to as oil-based creams. Creams may be generally produced by preparing an oil phase from petrolatum or a higher alcohol or a mixture thereof with an additive such as an emulsifying agent, separately preparing a water phase from purified water or a mixture thereof with an additive such as an emulsifying agent, adding an active component either to the oil phase or the water phase, heating both phases and mixing the oil phase and the water phase until homogeneity to obtain an emulsion. Gels refer to a gel formulation and examples thereof include water-based gels, oil-based gels and the like. Water-based gels may be produced by dissolving or suspending an active component in an additive such as a polymer compound and purified water and allowing crosslinking by heating and cooling or addition of a gel-forming agent. Oil-based gels may be produced by mixing an active component with a liquid oil base such as a glycol or a higher alcohol and an additive. Ointments refer to a semi-solid formulation containing an active component dissolved or dispersed in a base. Examples thereof include oil- or fat-based ointments, water-soluble ointments and the like. Oil- or fat-based ointments may be generally produced by melting an oil- or fat-based base such as an oil or fat, a wax and a hydrocarbon including paraffin by heating, dissolving or dispersing an active component therein and mixing and kneading to obtain a homogeneous mixture. Water-soluble ointments may be generally produced by melting a water-soluble base such as macrogol by heating and mixing and kneading an active component therein to obtain a homogeneous mixture.

### (4) Oral rinses

Oral rinses are a liquid formulation to be applied locally to the oral cavity or pharynx and may include solid formulations which are dissolved upon use. Oral rinses may be generally produced by homogeneously dissolving an active component in a solvent and an additive and optionally filtering the solution. Solid formulations which are dissolved upon use may be generally produced according to the production procedures described for the tablets and granules.

### [Formulations for injection]

### (1) Injections

Injections are an aseptic formulation in the form of solution, suspension or emulsion or solid to be dissolved or suspended upon use, which are directly administered to body tissues and organs such as under the skin, in the muscle or to a vessel. Examples thereof include those generally referred to as injections as well as lyophilised injections, powder injections, pre-filled syringes, cartridges, transfusions, implantable injections, sustained release injections and the like. Injections may be generally produced according to the following procedure (a) or (b):
(a) An active component or a mixture of an active component with an additive is dissolved, suspended or emulsified in water for injection or another aqueous solvent or a nonaqueous solvent and the product is packed into a container for injection which is then sterilised;
(b) An active component or a mixture of an active component with an additive is dissolved, suspended or emulsified in water for injection or another aqueous solvent or a nonaqueous solvent and the product is subjected to aseptic filtration or the product is homogeneously prepared in an aseptic manner and is charged into a container for injection which is then sealed. Lyophilised injections may be generally produced by dissolving an active component or an active component together with an additive such as a vehicle in water for injection, subjecting the solution to aseptic filtration, charging the solution in a container for injection followed by lyophilisation or lyophilising the solution in a container dedicated for lyophilisation followed by packing the product in a container for injection. Powder injections may be generally produced by aseptic filtration and crystallization to obtain powder which is directly or a mixture thereof with a sterilized additive is charged into a container for injection. Pre-filled syringes may be generally produced by charging an active component or a solution, suspension or emulsion of an active component and an additive into a syringe. Cartridges refer to an injection in the form of a cartridge containing a drug solution to be placed in a dedicated syringe. Cartridges containing a drug solution may be generally produced by charging an active component or a solution, suspension or emulsion of an active component and an additive into a cartridge. Transfusions refer to an injection generally of 100 mL of more which is intravenously administered. Implantable injections refer to an injection in the form of a solid or gel, which is to be applied using an implantable tool or by surgery under the skin or in the muscle in order to release an active component over a long period of time. Implantable injections may be generally produced by forming a pellet, microsphere or gel with a biodegradable polymer compound. Sustained release injections refer to an injection applied in the muscle in order to release an active component over a long period of time and may be generally produced by dissolving or suspending an active component in a vegetable oil or obtaining a microsphere suspension with a biodegradable polymer compound.

### [Formulations for dialysis]

### (1) Agents for dialysis

Agents for dialysis are a liquid formulation or a solid formulation dissolved upon use to be used for peritoneal dialysis or haemodialysis. Examples thereof include agents for peritoneal dialysis, agents for haemodialysis and the like. Agents for peritoneal dialysis refer to an aseptic agent for dialysis used for peritoneal dialysis and may be generally produced by charging a solution of an active component and an additive in a solvent at a certain volume or a mixture of an active component and an additive into a container, sealing the same and optionally sterilizing the same. Solid formulations to be dissolved upon use may be generally produced according to the production procedures described above for the tablets and granules. Agents for haemodialysis refer to an agent for dialysis used for haemodialysis and may be generally produced by charging a solution of an active component and an additive in a solvent at a certain volume or a mixture of an active component and an additive into a container. Solid formulations to be dissolved upon use may be generally produced according to the production procedures described above for the tablets and granules.

### [Formulations for inhalation]

### (1) Agents for inhalation

Agents for inhalation are a formulation applied to the bronchus or lung by inhaling aerosols of an active component. Examples thereof include powder agents for inhalation, liquid agents for inhalation, aerosols for inhalation and the like. Powder agents for inhalation refer to a formulation to be inhaled as aerosols of solid particles at a predetermined amount, and may be generally produced by preparing fine particles of an active component and optionally mixing thereof with an additive such as lactose to obtain a homogeneous mixture. Liquid agents for inhalation refer to a liquid agent for inhalation to be applied by a nebuliser and the like and may be generally produced by homogeneously dissolving or suspending an active component in a solvent, an appropriate tonicity agent, a pH-controlling agent and the like and optionally filtering the product. Aerosols for inhalation refer to a metered-dose agent for inhalation to spray a predetermined amount of active component packed in a container together with a propellant. Aerosols for inhalation may be generally produced by preparing a solution or suspension from an active component, a solvent, an appropriate dispersant, a stabilising agent and the like and charging the product in a pressure resistant container attached with a flow regulating valve together with a liquid propellant.

### [Ophthalmic formulations]

### (1) Ophthalmic solutions

Ophthalmic solutions are a liquid aseptic formulation or a solid aseptic formulation to be dissolved or suspended upon use, which is applied to ophthalmic tissue such as conjunctival sac. Ophthalmic solutions may be generally produced by charging a solution or suspension of an active component and an additive in a solvent or the like at a certain volume or a mixture of an active component and an additive in a container.

### (2) Ophthalmic ointments

Ophthalmic ointments are a semi-solid aseptic formulation to be applied to ophthalmic tissue such as conjunctival sac, and may be generally produced by charging a homogeneous mixture of a base such as petrolatum and a solution or fine powder of an active component in a container.

### [Otological formulations]

### (1) Ear drops

Ear drops are a liquid or semi-solid formulation or a solid formulation to be dissolved or suspended upon use, which is administered to the external ear or middle ear. Ear drops are generally produced by charging a solution or suspension of an active component and an additive in a solvent or like at a certain volume or a mixture of an active component and an additive in a container.

### [Nasologic formulations]

### (1) Nasal drops

Nasal drops are a formulation to be administered to the nasal cavity or nasal mucosa and examples thereof include nasal powders, nasal liquids and the like. Nasal powders refer to a fine powder nasal drop to be administered to the nasal cavity and may be generally produced by making appropriately fine powder of an active component and optionally mixing the active component with an additive to obtain a homogeneous mixture. Nasal liquids refer to a nasal drop which is liquid or solid to be dissolved or suspended upon use and is administered to the nasal cavity. Nasal liquids may be generally produced by dissolving or suspending an active component in a solvent and an additive and optionally filtering the product. An additive for nasal liquids which may be used includes a tonicity agent, a pH controlling agent and the like.

### [Rectal formulations]

### (1) Suppositories

Suppositories are a semi-solid formulation having a certain shape, which is applied in the rectum and releases an active component by melting at body temperature or gradually dissolving or dispersing in water. Suppositories may be generally produced by dissolving or homogeneously dispersing a homogeneous mixture of an active component with an additive such as a dispersant and an emulsifying agent in a base liquefied by heating and the like, charging a predetermined amount of the product in a container and solidifying/moulding the same. A base for suppositories which may be generally used includes oil- or fat-based bases and hydrophilic bases.

### (2) Semi-solid formulations for rectal administration

Semi-solid formulations for rectal administration are a formulation applied around or in the anus and examples thereof include rectal creams, rectal gels, rectal ointments and the like. Semi-solid formulations for rectal administration may be generally produced by emulsifying an active component together with an additive in purified water and an oil component such as petrolatum, or by homogeneously mixing an active component and an additive with a base which is a polymer gel or an oil or fat. Rectal creams may be generally produced by preparing an oil phase from petrolatum or a higher alcohol or a mixture thereof with an additive such as an emulsifying agent, separately preparing a water phase from purified water or a mixture thereof with an additive such as an emulsifying agent, adding an active component either to the oil phase or the water phase, heating both phases and mixing the oil phase and the water phase until homogeneity to obtain an emulsion. Rectal gels refer to a gel formulation and examples thereof include water-based gels, oil-based gels and the like. Water-based gels may be produced by dissolving or suspending an active component in an additive such as a polymer compound and purified water and allowing crosslinking by heating and cooling or addition of a gel-forming agent. Oil-based gels may be produced by mixing an active component with a liquid oil base such as a glycol or a higher alcohol and an additive. Rectal ointments refer to a semi-solid formulation containing an active component dissolved or suspended in a base and examples thereof include oil- or fat-based ointments, water-soluble ointments and the like. Oil- or fat-based ointments may be generally produced by melting an oil- or fat-based base such as an oil or fat, a wax and a hydrocarbon including paraffin by heating, dissolving or suspending an active component therein and mixing and kneading to obtain a homogeneous mixture. Water-soluble ointments may be generally produced by melting a water-soluble base such as macrogol by heating and mixing and kneading an active component therein to obtain a homogeneous mixture.

### (3) Enema formulations

Enema formulations are a liquid or viscous gel formulation to be applied through the anus. Enema formulations are generally produced by dissolving or suspending an active component in a solvent or the like at a certain volume using purified water or an appropriate aqueous solvent and charging the product in a container. An additive which may be used for enema formulations includes a dispersant, a stabilising agent, a pH controlling agent and the like.

### [Vaginal formulations]

### (1) Vaginal tablets

Vaginal tablets are a solid formulation having a certain shape, which is applied in the vagina and releases an active component by gradually dissolving or dispersing in water. Vaginal tablets may be generally produced according to the production procedures described above for the tablets.

### (2) Vaginal suppositories

Vaginal suppositories are a semi-solid formulation having a certain shape, which is applied in the vagina and releases an active component by melting at body temperature or gradually dissolving or dispersing in water. Vaginal suppositories may be generally produced according to the production procedures described above for the rectal suppositories and the like.

### [Skin formulations]

### (1) Topical solid formulations

Topical solid formulations are a solid formulation to be applied or spread on skin including the scalp or nails and examples thereof include topical powders. Topical powders refer to a topical solid powder formulation and may be generally produced by mixing an active component with an additive such as a vehicle to obtain a homogeneous mixture which is then formed into powders.

### (2) Topical liquids

Topical liquids are a liquid formulation to be applied on skin including the scalp or nails and examples thereof include liniments, lotions and the like. Topical liquids may be generally produced by dissolving, emulsifying or suspending an active component in a solvent, an additive and the like and optionally filtering the product. Liniments refer to a liquid or muddy topical liquid to be rubbed into the skin. Lotions refer to a topical liquid containing an active component dissolved, emulsified or finely dispersed in an aqueous liquid. Lotions may be generally produced by preparing a solution, suspension or emulsion of an active component, an additive and purified water to obtain a homogeneous product.

### (3) Spray formulations

Spray formulations are a formulation to spray an active component in the form of mist, powder, foam or paste on the skin and examples thereof include topical aerosols, pump spray formulations and the like. Spray formulations may be generally produced by preparing a solution or suspension of an active component, optionally filtering the product and charging the product in a container. Topical aerosols refer to a spray formulation which sprays an active component together with liquefied gas or compressed gas packed in a container. Topical aerosols may be generally produced by preparing a solution or suspension of an active component and packing the product into a pressure resistant container attached with a continuous injection valve together with a liquid propellant. An additive such as a dispersant and a stabilising agent may be optionally added to topical aerosols. Pump spray formulations refer to a spray formulation which sprays an active component in a container by means of a pump. Pump spray formulations may be generally produced by dissolving or suspending an active component and an additive and charging the product in a container to which a pump is attached.

### (4) Ointments

Ointments are a semi-solid formulation to be applied on the skin containing an active component dissolved or dispersed in a base. Examples thereof include oil- or fat-based ointments, water soluble ointments and the like. Oil- or fat-based ointments may be generally produced by melting an oil- or fat-based base such as an oil or fat, a wax and a hydrocarbon including paraffin by heating, dissolving or suspending an active component therein and mixing and kneading to obtain a homogeneous mixture. Water soluble ointments may be generally produced by melting a water-soluble base such as macrogol by heating and mixing and kneading an active component therein to obtain a homogeneous mixture.

### (5) Creams

Creams are a semi-solid formulation in the form of an oil-in-water or water-in-oil emulsion to be applied on the skin and lipophilic formulations in the form of a water-in-oil emulsion may also be referred to as oil-based creams. Creams may be generally produced by preparing an oil phase from petrolatum or a higher alcohol or a mixture thereof with an additive such as an emulsifying agent, separately preparing a water phase from purified water or a mixture thereof with an additive such as an emulsifying agent, adding an active component either to the oil phase or the water phase, heating both phases and mixing the oil phase and the water phase until homogeneity to obtain an emulsion.

### (6) Gels

Gels are a gel formulation to be applied on the skin and examples thereof include water-based gels and oil-based gels. Water-based gels may be generally produced by dissolving or suspending an active component in an additive such as a polymer compound and purified water and allowing crosslinking by heating and cooling or addition of a gel-forming agent. Oil-based gels may be produced by mixing an active component with a liquid oil base such as a glycol or a higher alcohol and an additive.

### (7) Plasters and pressure sensitive adhesives

Plasters and pressure sensitive adhesives are a formulation to be adhered on the skin and examples thereof include tapes and cataplasms. Plasters and pressure sensitive adhesives may be generally produced by homogeneously mixing an active component with a base which is a polymer compound or a mixture thereof, spreading the mixture on a support or a liner (release material) and shaping the same. Plasters and pressure sensitive adhesives may be formed as transdermal absorption formulations by using a release-controlled film. An additive such as an adhesive or an absorption-promoting agent may be optionally used for plasters and pressure sensitive adhesives. Tapes refer to a plaster and pressure sensitive adhesive containing a base that contains little water and examples thereof include plasters and the like. Tapes may be generally produced with a base which is a water insoluble natural or synthetic polymer compound such as a resin, a plastic, a rubber or the like by spreading on a fabric or spreading on or incorporating into a plastic film an active component or a homogeneous mixture of an active component and an additive and shaping the product. Tapes may also be produced by incorporating a mixture of an active component and a base or another additive into a release material made of a release-controlled film, a support and a liner (release material) and shaping the same. Cataplasms refer to a plaster and pressure sensitive adhesive containing a base which contains water and may be generally produced by homogeneously mixing an active component with a liquid substance such as purified water or glycerol or homogeneously mixing and kneading a natural or synthetic polymer compound such as a water soluble polymer or a water-absorbable polymer and purified water together with an active component, spreading the mixture on a fabric or the like and shaping the same.

Unless otherwise defined, all technical and scientific terms and abbreviations used herein have the same meanings as those commonly understood by a person skilled in the art to which the present invention belongs.

Contents of all patent literatures and non patent literatures or references explicitly cited herein may be incorporated herein as a part of the present specification.

### [Examples]

The present invention is hereinafter specifically described by way of Examples and Biological Examples which do not limit the present invention. The present compounds and compounds described in Examples are denominated by using ACD/Name (version 6.00, available from Advanced Chemistry Development Inc.) or Chemdraw Ultra (version 12.0, available from Cambridge Soft).

The references to "Hi-flash SI" and "Hi-flash NH" in brackets in the sections of medium pressure preparative liquid chromatography respectively indicate the type of the columns used (Hi-flash SI: silica gel (available from Yamazen Corporation) and Hi-flash NH: aminopropyl group-bonded silica gel (available from Yamazen Corporation)).

HPLC retention time (min) is the value measured under the following conditions:
column: YMC Triart C₁₈ (particle diameter: 1.9 × 10⁻⁶ m; column length: 30 × 2.0 mm I.D.); flow rate: 1.0 mL/min; column temperature: 40°C; mobile phase (A): 0.1% trifluoroacetic acid aqueous solution; mobile phase (B): 0.1% trifluoroacetic acid-acetonitrile solution; gradient (the ratio of the mobile phase (A):the mobile phase (B) is indicated): [0 min] 95:5; [0.1 min] 95:5; [1.2 min] 5:95; [1.4 min] 5:95; [1.41 min] 95:5; [1.5 min] 95:5; detector: UV(PDA), ELSD, MS.

The numerical values indicated in the sections of NMR are values (chemical shift values) measured on ¹H-NMR using the solvents indicated.

Biological Example 4 as described hereinbelow is an example of a test demonstrating the usefulness of the present compounds for acromegaly. However, diseases which the present compounds target are not limited to acromegaly. It has been described above that the present compounds are useful for prophylaxis and/or therapy of all diseases in which somatostatin *per se* or a hormone modulated by somatostatin is involved.

### Reference Example 1

### 2,5-Dibromo-4-chloropyridine-3-carbaldehyde

To a solution of 3,5-dibromo-4-chloropyridine (CAS number: 13626-17-0, MILESTONE, N24466) (2 g) in tetrahydrofuran (hereinafter abbreviated as THF) (28 mL), lithium diisopropylamide (1 M solution in THF, 7.74 mL) was added dropwise at -78°C over 20 minutes. After stirring at - 78°C for 5 minutes, N,N-dimethylformamide (hereinafter abbreviated as DMF) (0.688 mL) was added dropwise over 3 minutes. After stirring at -78°C for 10 minutes, acetic acid (1.69 mL) was added, the reaction solution was heated to room temperature, diluted with water and extracted with ethyl acetate. The residue obtained by concentrating the organic layer under reduced pressure was purified by medium pressure preparative liquid chromatography (Hi-flash SI) (n-hexane:ethyl acetate = 90:10 to 0:100) to obtain a titled compound (1.95 g) having the following physical properties. HPLC retention time (min): 1.01;
MS (ESI, Pos.): 300 (M+H)⁺;
¹H-NMR (CDCl₃): δ 10.1, 8.86.

### Reference Example 2

### tert-butyl (3aS,7aR)-5-(2,5-dibromo-3-formylpyridin-4-yl)-2,2-dimethylhexahydro[1,3]oxazolo[5,4-c]pyridine-1(2H)-carboxylate

To a solution of the compound (1.3 g) produced in Reference Example 1 and tert-butyl (3aS,7aR)-2,2-dimethylhexahydro[1,3]oxazolo[5,4-c]pyridine-1(2H)-carboxylate (CAS number: 1173005-74-7, the compound disclosed in Example 8 of Japanese Patent Application Publication No. 2009-155283) (1.1 g) in N,N-dimethylacetamide (hereinafter abbreviated as DMA) (13 mL), triethylamine (1.8 mL) was added and the mixture was stirred at 50°C for 2 hours. The reaction solution was cooled to room temperature, diluted with water and extracted with ethyl acetate. The residue obtained by concentrating the organic layer under reduced pressure was purified by medium pressure preparative liquid chromatography (Hi-flash SI) (n-hexane:ethyl acetate = 90:10 to 0:100) to give a titled compound (1.2 g) having the following properties.
HPLC retention time (min): 1.24;
MS (ESI, Pos.): 520 (M+H)⁺.

### Reference Example 3

### tert-butyl (3aS,7aR)-5-[5-bromo-3-formyl-2-(1-methyl-1H-pyrazol-4-yl)pyridin-4-yl]-2,2-dimethylhexahydro[1,3]oxazolo[5,4-c]pyridine-1(2H)-carboxylate

Under a nitrogen atmosphere, to a solution of the compound (100 mg) produced in Reference Example 2 in 1,4-dioxane (0.8 mL), (1-methylpyrazol-4-yl)boronic acid (24 mg), [1,1'-bis(diphenylphosphino)ferrocene]palladium(II) dichloride (31.5 mg) and 2 M tripotassium phosphate aqueous solution (32 µL) were added and the mixture was stirred at 50°C for 5 hours. The mixture was left to cool and diluted with ethyl acetate, an insoluble substance was filtered off and the filtrate was concentrated under reduced pressure. The resulting residue was purified by medium pressure preparative liquid chromatography (Hi-flash SI) (n-hexane:ethyl acetate = 90:10 to 0:100) to give a titled compound having the following properties.
HPLC retention time (min): 0.88;
MS (ESI, Pos.): 522 (M+H)⁺.

### Reference Example 4

### tert-butyl (3aS,7aR)-5-[5-(3,5-difluorophenyl)-3-formyl-2-(1-methyl-1H-pyrazol-4-yl)pyridin-4-yl]-2,2-dimethylhexahydro[1,3]oxazolo[5,4-c]pyridine-1(2H)-carboxylate

Under a nitrogen atmosphere, to a solution of the compound produced in Reference Example 3 in 1,4-dioxane (1.0 mL), 3,5-difluorophenylboronic acid (30.4 mg), [1,1'-bis(diphenylphosphino)ferrocene]palladium(II) dichloride (31.5 mg) and 2 M tripotassium phosphate aqueous solution (32 µL) were added and the mixture was stirred at 90°C for 1 hour. The mixture was left to cool and diluted with ethyl acetate, an insoluble substance was filtered off and the filtrate was concentrated under reduced pressure. The resulting residue was purified by medium pressure preparative liquid chromatography (Hi-flash SI) (n-hexane:ethyl acetate = 90:10 to 0:100) to give a titled compound (13 mg) having the following properties.
HPLC retention time (min): 0.92;
MS (ESI, Pos.): 554 (M+H)⁺.

### Reference Example 5

### tert-butyl (3aS,7aR)-5-[5-(3,5-difluorophenyl)-3-[7-(methoxycarbonyl)-1H-benzimidazol-2-yl]-2-(1-methyl-1H-pyrazol-4-yl)pyridin-4-yl]-2,2-dimethylhexahydro[1,3]oxazolo[5,4-c]pyridine-1(2H)-carboxylate

Under an oxygen atmosphere, to a solution of the compound (13 mg) produced in Reference Example 4 in ethanol (1.0 mL), methyl 2,3-diaminobenzoate (5.8 mg) and acetic acid (13.5 µL) were added and the mixture was stirred at 80°C for 13 hours. The reaction solution was left to cool and concentrated under reduced pressure. The resulting residue was purified by medium pressure preparative liquid chromatography (Hi-flash SI) (n-hexane:ethyl acetate = 90:10 to 0:100) to give a titled compound having the following properties.
HPLC retention time (min): 0.95;
MS (ESI, Pos.): 700 (M+H)⁺.

### Example 1

### Methyl 2-{4-[(3S,4R)-4-amino-3-hydroxy-1-piperidinyl]-5-(3,5-difluorophenyl)-2-(1-methyl-1H-pyrazol-4-yl)-3-pyridinyl}-1H-benzimidazole-7-carboxylate

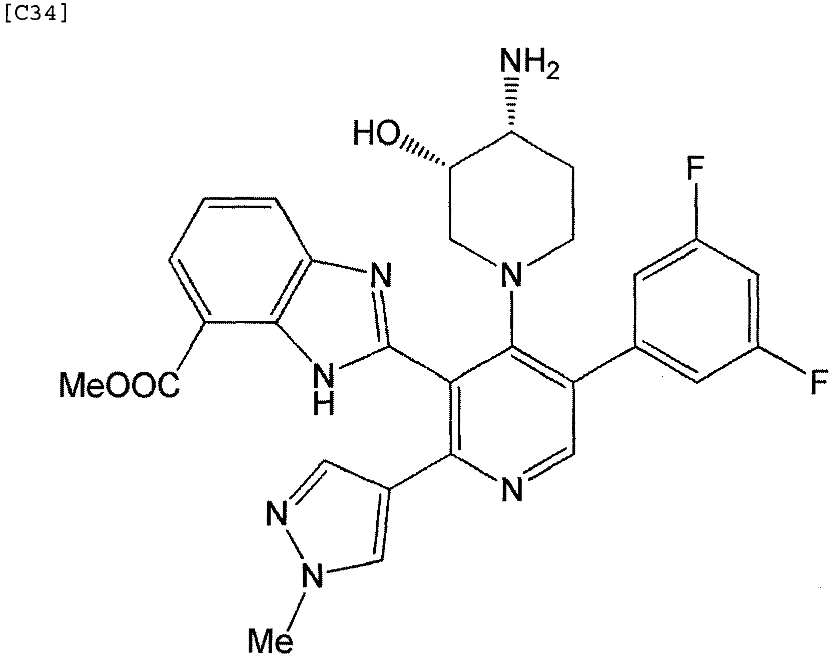

Under a nitrogen atmosphere, to a solution of the compound produced in Reference Example 5 in dichloromethane (1.0 mL), trifluoroacetic acid (87 µL) was added and the mixture was stirred at room temperature for 3 hours. The reaction solution was concentrated under reduced pressure and the resulting residue was purified by medium pressure preparative liquid chromatography (Hi-flash NH) (ethyl acetate:methanol = 100:0 to 80:20) to give the present compound (7 mg) having the following properties.
HPLC retention time (min): 0.57;
MS (ESI, Pos.): 560 (M+H)⁺;
¹H-NMR (CD₃OD): δ 8.38, 8.04, 8.02, 7.51-7.41, 7.28-7.18, 7.08-6.98, 6.85, 3.96, 3.72, 3.37-3.28, 3.21-3.09, 2.93-2.73, 2.46-2.37, 2.23-2.17, 1.09-0.79.

### Examples 1(1) to 1(4)

The present compounds having the following properties were obtained in the similar procedures as in Reference Example 3 → Reference Example 4 → Reference Example 5 → Example 1 by using a corresponding boronic acid compound instead of (1-methylpyrazol-4-yl)boronic acid, a corresponding boronic acid compound instead of 3,5-difluorophenylboronic acid and a corresponding benzene-1,2-diamine compound instead of methyl 2,3-diaminobenzoate.

### Example 1(1)

### (3S,4R)-4-amino-1-[3-(5,6-difluoro-1H-benzimidazol-2-yl)-2,5-bis(3,5-difluorophenyl)pyridin-4-yl]piperidin-3-ol HPLC retention time (min): 0.63;

MS (ESI, Pos.): 570 (M+H)⁺;
¹H-NMR (CDCl₃): δ 11.46, 8.43, 7.42, 7.20-7.09, 6.96-6.86, 6.82, 6.73-6.62, 3.64-3.42, 2.98-2.57, 2.29-2.20, 1.14-0.97, 0.81-0.63.

### Example 1(2)

### (3S,4R)-4-amino-1-[3-(5,6-difluoro-1H-benzimidazol-2-yl)-5-(3,5-difluorophenyl)-2-(1-methyl-1H-pyrazol-4-yl)-4-pyridinyl]-3-piperidinol

HPLC retention time (min): 0.58;
MS (ESI, Pos.): 538 (M+H)⁺;
¹H-NMR (CD₃OD): δ 8.36, 7.54, 7.37, 7.20, 7.09-6.97, 6.89-6.83, 3.73, 3.42-3.34, 3.18-3.04, 2.89-2.68, 2.54-2.41, 2.28-2.16, 1.12-0.98, 0.95-0.76.

### Example 1(3)

### Methyl 2-{4-[(3S,4R)-4-amino-3-hydroxy-1-piperidinyl]-5-(3,5-difluorophenyl)-2-(1-methyl-1H-pyrazol-3-yl)-3-pyridinyl}-1H-benzimidazole-7-carboxylate

HPLC retention time (min): 0.60;
MS (ESI, Pos.): 560 (M+H)⁺;
¹H-NMR (CD₃OD): δ 8.43, 7.98, 7.95, 7.46-7.33, 7.29-7.20, 7.11-6.99, 6.01, 3.98, 3.67, 3.40-3.32, 3.22-3.10, 2.95-2.78, 2.50-2.40, 2.25-2.15, 1.13-0.80.

### Example 1(4)

### Methyl 2-{4-[(3S,4R)-4-amino-3-hydroxy-1-piperidinyl]-5-(3,5-difluorophenyl)-2-(1-methyl-1H-pyrazol-5-yl)-3-pyridinyl}-1H-benzimidazole-7-carboxylate

HPLC retention time (min): 0.63;
MS (ESI, Pos.): 560 (M+H)⁺;
¹H-NMR (CD₃OD): δ 8.50, 7.98, 7.92, 7.38, 7.35-7.24, 7.14, 7.12-7.01, 5.74, 3.97, 3.93, 3.41-3.35, 3.22-3.09, 2.96-2.79, 2.50-2.40, 2.30-2.19, 1.11-0.84.

### Reference Example 6

### tert-butyl (3aS,7aR)-5-(5-bromo-3-formyl-2-methylpyridin-4-yl)-2,2-dimethylhexahydro[1,3]oxazolo[5,4-c]pyridine-1(2H)-carboxylate

To a solution of the compound (200 mg) produced in Reference Example 2 in 1,4-dioxane (1.5 mL), 2 M tripotassium phosphate aqueous solution (0.40 uL), trimethylboroxine (16.9 mg) and [1,1'-bis(diphenylphosphino)ferrocene]palladium(II) dichloride (22 mg) were added and the mixture was stirred at 120°C for 30 minutes. The reaction solution was cooled to room temperature, diluted with ethyl acetate and washed with water and a saturated sodium chloride solution. The organic layer was dried and then concentrated. The resulting residue was purified by medium pressure preparative liquid chromatography (Hi-flash SI) (n-hexane:ethyl acetate = 90:10 to 0:100) to give a titled compound (58 mg) having the following properties.
HPLC retention time (min): 0.81;
MS (ESI, POS.): 454 (M+H)⁺;
¹H-NMR (CDCl₃): δ 10.45, 8.57, 3.99-4.31, 3.15-3.58, 2.65, 2.14-2.48, 1.48-2.03, 1.48.

### Example 2

### (3S,4R)-4-amino-1-[5-(3-chloro-5-fluorophenyl)-3-(6-methoxy-1H-benzimidazol-2-yl)-2-methyl-4-pyridinyl]-3-piperidinol

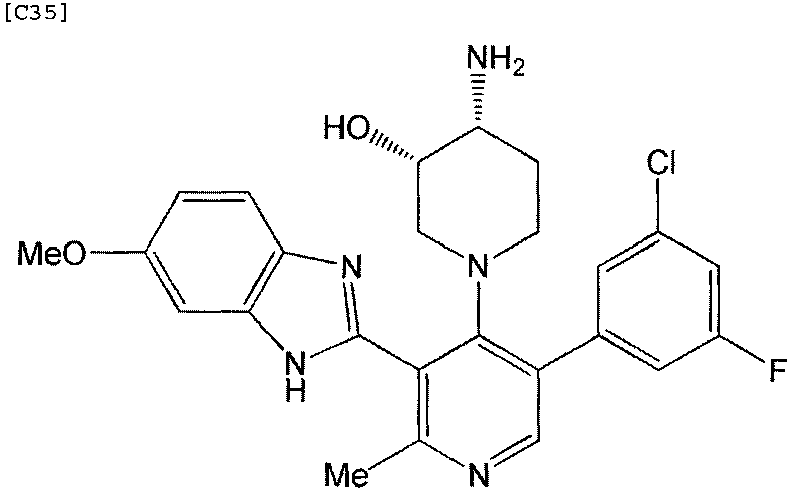

The present compounds having the following properties were obtained in the similar procedures as in Reference Example 4 → Reference Example 5 → Example 1 by using the compound produced in Reference Example 6 instead of the compound produced in Reference Example 3, 3-chloro-5-fluorophenylboronic acid instead of 3,5-difluorophenylboronic acid and 4-methoxybenzene-1,2-diamine instead of methyl 2,3-diaminobenzoate.
HPLC retention time (min):0.52;
MS (ESI, Pos.): 482 (M+H)⁺;
¹H-NMR (CD₃OD): δ 8.21, 7.58-7.42, 7.37-7.20, 7.18-7.09, 7.00-6.90, 3.87, 3.43-3.36, 3.11-2.98, 2.91-2.70, 2.50-2.39, 2.36, 2.19-2.07, 1.12-0.95.

### Examples 2(1) to 2(13)

The present compounds having the following properties were obtained in the similar procedures as in Reference Example 4 → Reference Example 5 → Example 1 by using the compound produced in Reference Example 6 instead of the compound produced in Reference Example 3, a corresponding boronic acid compound instead of 3,5-difluorophenylboronic acid and a corresponding benzene-1,2-diamine compound instead of methyl 2,3-diaminobenzoate.

### Example 2(1)

### (3S,4R)-4-amino-1-[3-(5,6-difluoro-1H-benzimidazol-2-yl)-5-(3,5-difluorophenyl)-2-methyl-4-pyridinyl]-3-piperidinol

HPLC retention time (min): 0.54;
MS (ESI, Pos.): 472 (M+H)⁺;
¹H-NMR (CD₃OD): δ 8.24, 7.61-7.54, 7.20, 7.08-6.96, 3.64-3.56, 3.17-3.04, 2.96-2.69, 2.34, 2.20-2.10, 1,34-1.18, 1.21-1.10.

### Example 2(2)

### Methyl 2-{4-[(3S,4R)-4-amino-3-hydroxy-1-piperidinyl]-5-(3,5-difluorophenyl)-2-methyl-3-pyridinyl}-5-fluoro-1H-benzimidazole-7-carboxylate

HPLC retention time (min): 0.59;
MS (ESI, Pos.): 512 (M+H)⁺;
¹H-NMR (CD₃OD): δ 8.24, 7.80-7.70, 7.25-7.18, 7.08-6.96, 4.01, 3.41-3.36, 3.16-3.02, 2.90-2.80, 2.79-2.70, 2.54-2.41, 2.32, 2.21-2.12, 1.34-1.18, 1.12-0.95.

### Example 2(3)

### Methyl 2-{4-[(3S,4R)-4-amino-3-hydroxy-1-piperidinyl]-5-(3-chloro-5-fluorophenyl)-2-methyl-3-pyridinyl}-5-fluoro-1H-benzimidazole-7-carboxylate

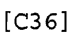

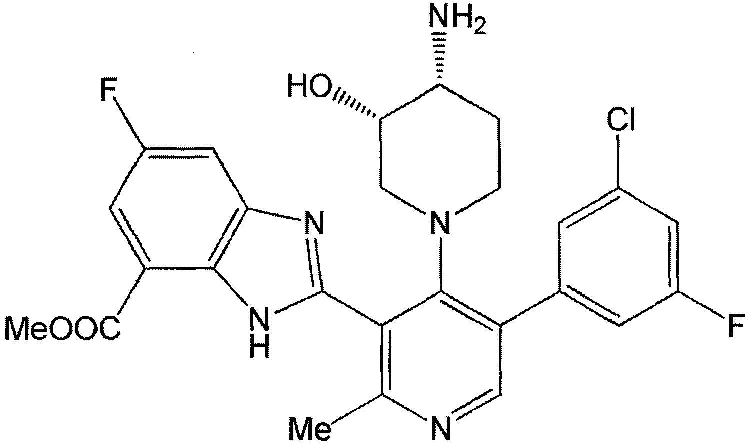

HPLC retention time (min): 0.59;
MS (ESI, Pos.): 528 (M+H)⁺;
¹H-NMR (CDCl₃): δ 11.24, 8.32, 7.79-7.70, 7.16-7.09, 6.94-6.88, 4.01, 3.46-3.42, 3.14-3.05, 2.85-2.71, 2.67-2.59, 2.50, 2.22-2.15, 1.03-0.93, 0.66-0.49.

### Example 2(4)

### (3S,4R)-4-amino-1-[5-(3-chloro-5-fluorophenyl)-3-(5,6-difluoro-1H-benzimidazol-2-yl)-2-methyl-4-pyridinyl]-3-piperidinol

HPLC retention time (min): 0.58;
MS (ESI, Pos.): 488 (M+H)⁺;
¹H-NMR (CD₃OD): δ 8.36, 7.64, 7.56-7.49, 7.43-7.37, 3.69-3.65, 3.21-2.94, 2.42, 2.37-2.29, 1.40-1.20.

### Example 2(5)

### (3S,4R)-4-amino-1-[5-(3,5-difluorophenyl)-3-(6-methoxy-1H-benzimidazol-2-yl)-2-methyl-4-pyridinyl]-3-piperidinol

HPLC retention time (min): 0.50;
MS (ESI, Pos.): 466 (M+H)⁺;
¹H-NMR (CD₃OD): δ 8.21, 7.54, 7.28-7.12, 7.10-6.92, 3.86, 3.40, 3.11-2.99, 2.88-2.75, 2.47-2.38, 2.35, 2.21-2.10, 1.10-0.90.

### Example 2(6)

### (3S,4R)-4-amino-1-[5-(3,5-difluorophenyl)-3-(5-fluoro-6-methoxy-1H-benzimidazol-2-yl)-2-methyl-4-pyridinyl]-3-piperidinol

HPLC retention time (min): 0.52;
MS (ESI, Pos.): 484 (M+H)⁺;
¹H-NMR (CD₃OD): δ 8.22, 7.40, 7.30, 7.24-7.16, 7.09-6.94, 3.94, 3.40, 3.10-2.98, 2.88-2.72, 2.50-2.40, 2.35, 2.21-2.10, 1.12-0.91.

### Example 2(7)

### Ethyl 2-{4-[(3S,4R)-4-amino-3-hydroxy-1-piperidinyl]-5-(3,5-difluorophenyl)-2-methyl-3-pyridinyl}-5-fluoro-1H-benzimidazole-7-carboxylate

HPLC retention time (min): 0.60;
MS (ESI, Pos.): 526 (M+H)⁺;
¹H-NMR (CD₃OD): δ 8.23, 7.79-7.70, 7.24-7.18, 7.05-6.95, 4.49, 3.38-3.35, 3.13-3.02, 2.88-2.72, 2.46-2.38, 2.32, 2.19-2.12, 1.43, 1.07-0.85.

### Example 2(8)

### (3S,4R)-4-amino-1-[5-(3-chloro-5-fluorophenyl)-3-(5-fluoro-6-methoxy-1H-benzimidazol-2-yl)-2-methyl-4-pyridinyl]-3-piperidinol

HPLC retention time (min): 0.55;
MS (ESI, Pos.): 500 (M+H)⁺;
¹H-NMR (CD₃OD): δ 8.21, 7.50-7.20, 3.95, 3.45-3.37, 3.12-2.95, 2.89-2.70, 2.50-2.39, 2.35, 2.18-2.07, 1.14-0.92.

### Example 2(9)

### (3S,4R)-4-amino-1-[3-(5-chloro-6-fluoro-1H-benzimidazol-2-yl)-5-(3,5-difluorophenyl)-2-methyl-4-pyridinyl]-3-piperidinol

HPLC retention time (min): 0.59;
MS (ESI, Pos.): 488 (M+H)⁺;
¹H-NMR (CD₃OD): δ 8.23, 7.82-7.71, 7.59-7.47, 7.26-7.12, 7.10-6.94, 3.47-3.38, 3.15-2.98, 2.91-2.72, 2.57-2.41, 2.36, 2.24-2.12, 1.21-0.87.

### Example 2(10)

### (3S,4R)-4-amino-1-[5-(2,5-difluorophenyl)-3-(6-methoxy-1H-benzimidazol-2-yl)-2-methyl-4-pyridinyl]-3-piperidinol

HPLC retention time (min): 0.50;
MS (ESI, Pos.): 466 (M+H)⁺;
¹H-NMR (CD₃OD): δ 8.20, 7.55, 7.34-7.17, 7.14, 6.96, 3.87, 3.43-3.36, 3.06-2.94, 2.89-2.72, 2.54-2.42, 2.38, 2.34-2.25, 1.11-0.97, 0.95-0.80.

### Example 2(11)

### (3S,4R)-4-amino-1-[3-(5,6-difluoro-1H-benzimidazol-2-yl)-5-(2,5-difluorophenyl)-2-methyl-4-pyridinyl]-3-piperidinol

HPLC retention time (min): 0.55;
MS (ESI, Pos.): 472 (M+H)⁺;
¹H-NMR (CD₃OD): δ 8.35, 7.62, 7.41-7.23, 3.72-3.55, 3.13-2.88, 2.51-2.42, 2.39, 1.38-1.25.

### Example 2(12)

### Methyl 2-{4-[(3S,4R)-4-amino-3-hydroxy-1-piperidinyl]-5-(2,5-difluorophenyl)-2-methyl-3-pyridinyl}-5-fluoro-1H-benzimidazole-7-carboxylate

Properties: amorphous;
HPLC retention time (min): 0.56;
MS (ESI, Pos.): 512 (M+H)⁺;
¹H-NMR (CD₃OD): δ 8.24, 7.78-7.70, 7.34-7.16, 4.01, 3.08-2.99, 2.86-2.73, 2.49-2.40, 2.38-2.27, 1.05-0.95, 0.84-0.69.

### Example 2(13)

### (3S,4R)-4-amino-1-[5-(3,5-difluorophenyl)-2-methyl-3-(5,6,7-trifluoro-1H-benzimidazol-2-yl)-4-pyridinyl]-3-piperidinol

HPLC retention time (min): 0.58;
MS (ESI, Pos.): 490 (M+H)⁺;
¹H-NMR (CD₃OD): δ 8.22, 7.40-7.30, 7.22-7.13, 7.07-6.97, 3.47-3.40, 3.17-3.03, 2.91-2.73, 2.60-2.50, 2.35, 2.22-2.14, 1.18-0.88.

### Example 3

### (3S,4R)-1-[5-(3-chloro-5-fluorophenyl)-3-(6-methoxy-1H-benzimidazol-2-yl)-2-methyl-4-pyridinyl]-4-(3-oxetanylamino)-3-piperidinol

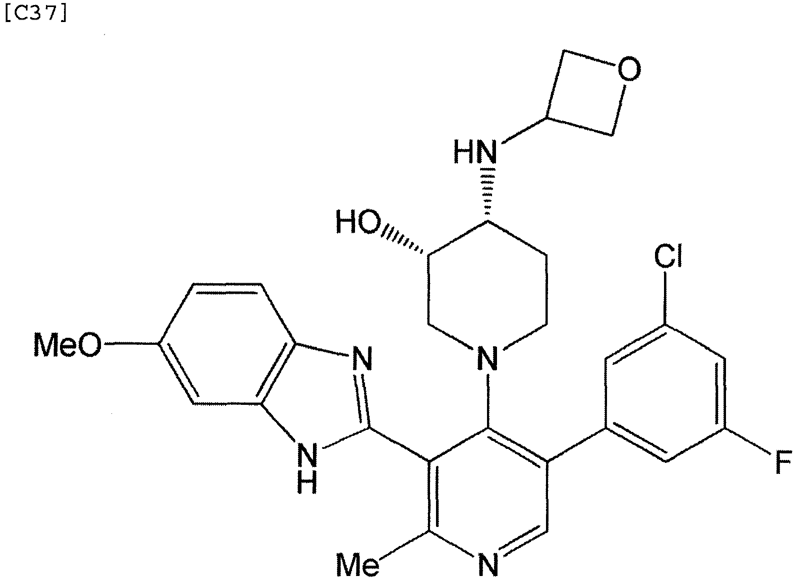

Under a nitrogen atmosphere, acetic acid (30 µL) was added to a suspension of the compound (48 mg) produced in Example 2 in methanol (1.0 mL) to which 3-oxetanone (58 µL) and 2-picoline-borane complex (19.3 mg) were added and the mixture was stirred at room temperature. After 13 hours, 3-oxetanone (39 µL) and 2-picoline-borane complex (13.9 mg) were further added and the mixture was stirred at room temperature for 13 hours. The reaction solution was added with a saturated sodium hydrogen carbonate aqueous solution and extracted with ethyl acetate and the organic layer was concentrated under reduced pressure. The resulting residue was purified by medium pressure preparative liquid chromatography (Hi-flash NH) (ethyl acetate:methanol = 100:5 to 80:20) to give the present compound (45 mg) having the following properties. Properties: amorphous;
HPLC retention time (min): 0.54;
MS (ESI, Pos.): 538 (M+H)⁺;
¹H-NMR (CD₃OD): δ 8.20, 7.65-7.50, 7.49-7.43, 7.38-7.22, 7.21-7.06, 7.00-6.91, 4.70-4.57, 4.41, 4.26, 3.97-3.82, 3.87, 3.43-3.38, 3.13-3.01, 2.93-2.69, 2.35, 2.33-2.23, 2.17-2.06, 1.08-0.94.

### Examples 3(1) to 3(16)

The present compounds having the following properties were obtained in the similar procedures as in Reference Example 4 → Reference Example 5 → Example 1 → Example 3 by using the compound produced in Reference Example 6 instead of the compound produced in Reference Example 3, a corresponding boronic acid compound instead of 3,5-difluorophenylboronic acid, a corresponding benzene-1,2-diamine compound instead of methyl 2,3-diaminobenzoate and a corresponding aldehyde compound instead of 3-oxetanone.

### Example 3(1)

### (3S,4R)-1-[3-(6-chloro-1H-benzimidazol-2-yl)-5-(3,5-difluorophenyl)-2-methyl-4-pyridinyl]-4-(ethylamino)-3-piperidinol

HPLC retention time (min): 0.60;
MS (ESI, Pos.): 498 (M+H)⁺;
¹H-NMR (CD₃OD): δ 8.23, 7.68, 7.64, 7.31, 7.23-7.18, 7.07-6.96, 3.65-3.61, 3.19-3.05, 2.97-2.73, 2.63-2.50, 2.50-2.39, 2.35, 2.34-2.26, 2.14-2.06, 1.20-1.05, 1.01, 0.97-0.90.

### Example 3(2)

### (3S,4R)-1-[5-(3,5-difluorophenyl)-3-(6-methoxy-1H-benzimidazol-2-yl)-2-methylpyridin-4-yl]-4-(ethylamino)piperidin-3-ol

HPLC retention time (min): 0.53;
MS (ESI, Pos.): 494 (M+H)⁺;
¹H-NMR (CD₃OD): δ 8.21, 7.55, 7.24-7.17, 7.15, 7.06-6.99, 6.96, 3.87, 3.65-3.61, 3.16-3.04, 2.96-2.86, 2.85-2.73, 2.62-2.50, 2.49-2.37, 2.35, 2.33-2.24, 2.14-2.06, 1.17-1.05, 1.00, 1.00-0.93.

### Example 3(3)

### (3S,4R)-1-[3-(5,6-difluoro-1H-benzimidazol-2-yl)-5-(3,5-dimethoxyphenyl)-2-methyl-4-pyridinyl]-4-(ethylamino)-3-piperidinol

HPLC retention time (min): 0.60;
MS (ESI, Pos.): 524 (M+H)⁺;
¹H-NMR (CD₃OD): δ 8.20, 7.61-7.51, 6.61-6.51, 3.83, 3.66-3.61, 3.20-3.12, 2.95-2.86, 2.82-2.71, 2.64-2.55, 2.52-2.42, 2.37, 2.36-2.27, 2.24-2.16, 1.17-1.07, 1.03, 0.95-0.86.

### Example 3(4)

### (3S,4R)-1-[5-(3,5-difluorophenyl)-3-(5-fluoro-6-methoxy-1H-benzimidazol-2-yl)-2-methyl-4-pyridinyl]-4-(ethylamino)-3-piperidinol

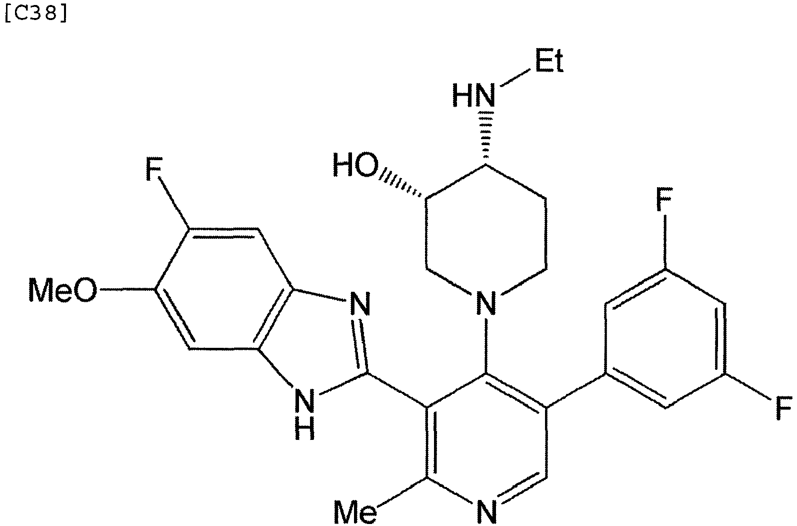

HPLC retention time (min): 0.56;
MS (ESI, Pos.): 512 (M+H)⁺;
¹H-NMR (CD₃OD): δ 8.21, 7.40, 7.30, 7.20, 7.01, 3.94, 3.66-3.60, 3.16-3.07, 2.91-2.86, 2.84-2.75, 2.61-2.37, 2.35, 2.36-2.26, 2.13-2.05, 1.18-0.84, 1.01.

### Example 3(5)

### (3S,4R)-1-[5-(3-chloro-5-fluorophenyl)-3-(5,6-difluoro-1H-benzimidazol-2-yl)-2-methyl-4-pyridinyl]-4-(3-oxetanylamino)-3-piperidinol

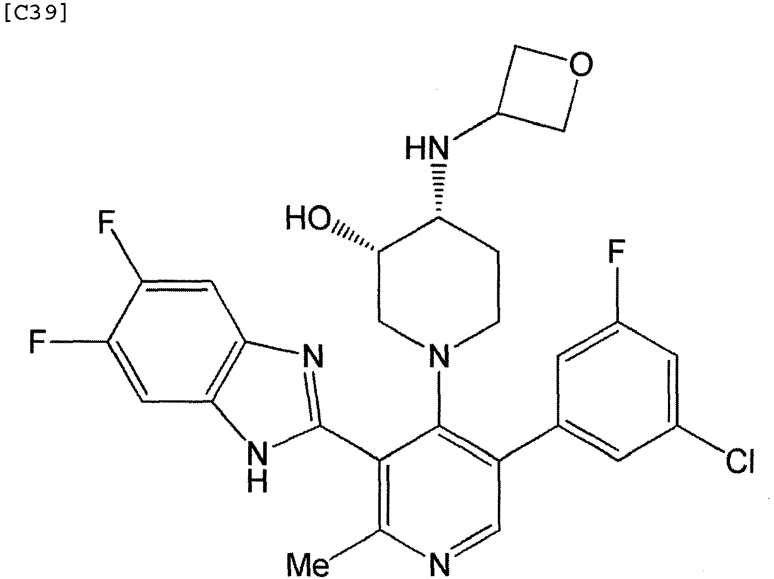

HPLC retention time (min): 0.59;
MS (ESI, Pos.): 544 (M+H)⁺;
¹H-NMR (CD₃OD): δ 8.22, 7.56, 7.45, 7.33-7.23, 4.65, 4.43, 4.31, 3.97-3.88, 3.43-3.38, 3.11-3.02, 2.90-2.68, 2.39-2.31, 2.34, 2.12-2.05, 1.04-0.97.

### Example 3(6)

### Methyl 2-{5-(3,5-dimethoxyphenyl)-4-[(3S,4R)-4-(ethylamino)-3-hydroxy-1-piperidinyl]-2-methyl-3-pyridinyl}-5-fluoro-1H-benzimidazole-7-carboxylate

HPLC retention time (min): 0.61;
MS (ESI, Pos.): 564 (M+H)⁺;
¹H-NMR (CD₃OD): δ 8.22, 7.78-7.70, 6.64-6.57, 6.56-6.50, 4.01, 3.83, 3.60-3.53, 2.98-2.90, 2.79-2.68, 2.58-2.49, 2.46-2.37, 2.32, 2.30-2.24, 2.23-2.16, 1.14-1.03, 0.99, 0.91-0.78.

### Example 3(7)

### (3S,4R)-1-[5-(3,5-dimethoxyphenyl)-3-(6-methoxy-1H-benzimidazol-2-yl)-2-methyl-4-pyridinyl]-4-(ethylamino)-3-piperidinol

HPLC retention time (min): 0.55;
MS (ESI, Pos.): 518 (M+H)⁺;
¹H-NMR (CD₃OD): δ 8.18, 7.55, 7.15, 6.95, 6.62-6.56, 6.56-6.49, 3.90, 3.83, 3.65-3.60, 3.22-3.11, 2.96-2.86, 2.82-2.69, 2.60-2.51, 2.47-2.39, 2.37, 2.31-2.22, 2.23-2.15, 1.15-1.05, 1.00, 0.94-0.84.

### Example 3(8)

### (3S,4R)-1-[5-(2,5-difluorophenyl)-3-(6-methoxy-1H-benzimidazol-2-yl)-2-methyl-4-pyridinyl]-4-(ethylamino)-3-piperidinol

HPLC retention time (min): 0.53;
MS (ESI, Pos.): 494 (M+H)⁺;
¹H-NMR (CD₃OD): δ 8.20, 7.55, 7.33-7.13, 6.96, 3.87, 3.64-3.58, 3.13-3.05, 2.95-2.76, 2.61-2.49, 2.49-2.39, 2.38, 2.34-2.19, 1.14-1.04, 0.99, 0.90-0.75.

### Example 3(9)

### (3S,4R)-1-[5-(2,5-difluorophenyl)-3-(5-fluoro-6-methoxy-1H-benzimidazol-2-yl)-2-methyl-4-pyridinyl]-4-(ethylamino)-3-piperidinol

HPLC retention time (min): 0.56;
MS (ESI, Pos.): 512 (M+H)⁺;
¹H-NMR (CD₃OD): δ 8.20, 7.41, 7.35-7.15, 3.94, 3.64-3.57, 3.12-3.03, 2.94-2.75, 2.60-2.49, 2.48-2.40, 2.38, 2.35-2.20, 1.14-1.05, 1.00, 0.88-0.72.

### Example 3(10)

### (3S,4R)-1-[3-(5,6-difluoro-1H-benzimidazol-2-yl)-5-(2,5-difluorophenyl)-2-methyl-4-pyridinyl]-4-(ethylamino)-3-piperidinol

HPLC retention time (min): 0.58;
MS (ESI, Pos.): 500 (M+H)⁺;
¹H-NMR (CD₃OD) : δ 8.21, 7.62-7.49, 7.33-7.15, 3.64-3.58, 3.11-3.01, 2.93-2.75, 2.61-2.51, 2.48-2.40, 2.40, 2.36-2.20, 1.13-1.05, 1.01, 0.88-0.70.

### Example 3(11)

### (3S,4R)-1-[5-(3-chloro-5-fluorophenyl)-3-(5-fluoro-6-methoxy-1H-benzimidazol-2-yl)-2-methyl-4-pyridinyl]-4-(3-oxetanylamino)-3-piperidinol

HPLC retention time (min): 0.57;
MS (ESI, Pos.): 556 (M+H)⁺;
¹H-NMR (CD₃OD): δ 8.21, 7.50-7.22, 4.70-4.58, 4.42, 4.28, 3.97-3.83, 3.95, 3.42-3.37, 3.12-3.00, 2.91-2.69, 2.34, 2.33-2.23, 2.13-2.05, 1.06-0.95.

### Example 3(12)

### (3S,4R)-1-[3-(5,6-difluoro-1H-benzimidazol-2-yl)-5-(3,5-difluorophenyl)-2-methyl-4-pyridinyl]-4-[(3,3,3-trifluoropropyl)amino]-3-piperidinol

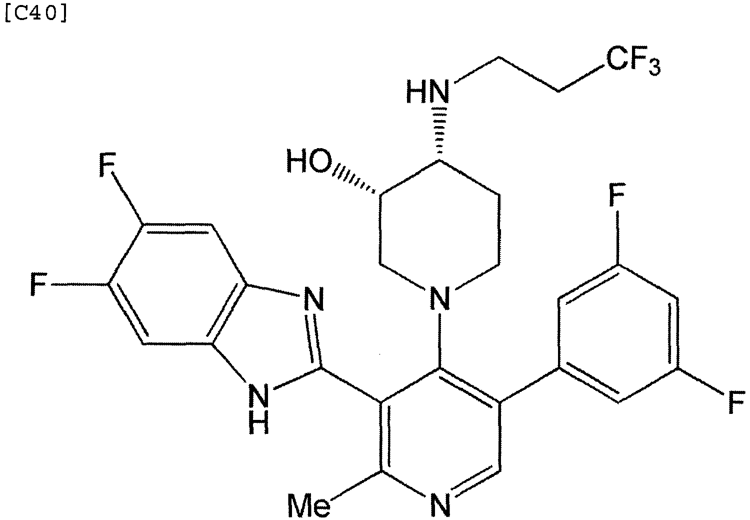

HPLC retention time (min): 0.69;
MS (ESI, Pos.): 568 (M+H)⁺;
¹H-NMR (CD₃OD): δ 8.23, 7.65-7.50, 7.22-7.14, 7.06-6.95, 3.62-3.56, 3.13-3.02, 2.92-2.55, 2.42-2.11, 2.35, 1.18-0.85.

### Example 3(13)

### (3S,4R)-1-[5-(3-chloro-5-fluorophenyl)-3-(5,6-difluoro-1H-benzimidazol-2-yl)-2-methyl-4-pyridinyl]-4-[(3,3,3-trifluoropropyl)amino]-3-piperidinol

HPLC retention time (min): 0.71;
MS (ESI, Pos.): 584 (M+H)⁺;
¹H-NMR (CD₃OD): δ 8.22, 7.63-7.43, 7.35-7.22, 3.63-3.55, 3.12-3.00, 2.93-2.57, 2.42-2.09, 2.34, 1.18-0.90.

### Example 3(14)

### (3S,4R)-1-[3-(5,6-difluoro-1H-benzimidazol-2-yl)-5-(2,5-difluorophenyl)-2-methyl-4-pyridinyl]-4-[(3,3,3-trifluoropropyl)amino]-3-piperidinol

HPLC retention time (min): 0.65;
MS (ESI, Pos.): 568 (M+H)⁺;
¹H-NMR (CD₃OD): δ 8.21, 7.62-7.50, 7.33-7.17, 3.59-3.50, 3.09-2.96, 2.90-2.55, 2.44-2.15, 2.37, 1.13-1.00, 0.92-0.77.

### Example 3(15)

### (3S,4R)-1-[5-(3,5-difluorophenyl)-2-methyl-3-(5,6,7-trifluoro-1H-benzimidazol-2-yl)-4-pyridinyl]-4-(ethylamino)-3-piperidinol

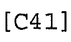

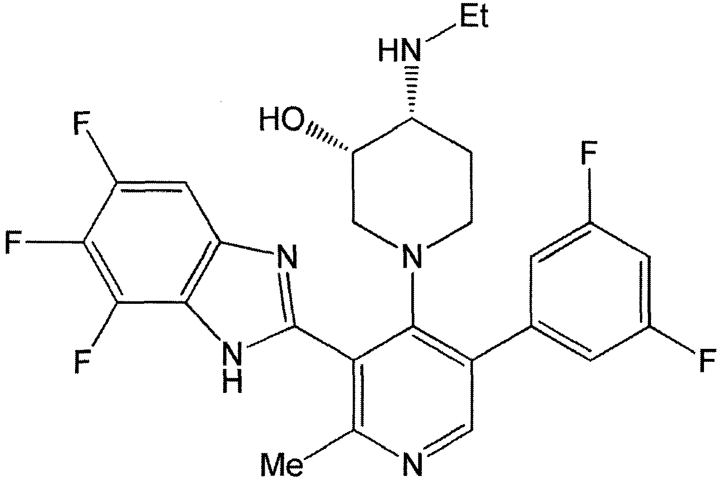

Properties: amorphous;
HPLC retention time (min): 0.62;
MS (ESI, Pos.): 518 (M+H)⁺;
¹H-NMR (CD₃OD): δ 8.20, 7.38-7.29, 7.24-7.12, 7.05-6.96, 3.70-3.60, 3.23-3.10, 2.99-2.88, 2.84-2.71, 2.70-2.42, 2.34, 2.16-2.06, 1.23-1.10, 1.06, 1.05-0.87.

### Example 3(16)

### (3S,4R)-1-[5-(3,5-difluorophenyl)-2-methyl-3-(5,6,7-trifluoro-1H-benzimidazol-2-yl)-4-pyridinyl]-4-[(3,3,3-trifluoropropyl)amino]-3-piperidinol

HPLC retention time (min): 0.69;
MS (ESI, Pos.): 586 (M+H)⁺;
¹H-NMR (CD₃OD): δ 8.23, 7.43-7.37, 7.25-7.12, 7.08-6.97, 3.63-3.54, 3.14-3.02, 2.94-2.57, 2.42-2.12, 2.36, 1.21-0.90.

### Reference Example 7

### tert-butyl (3aS,7aR)-5-(3-bromo-5-formylpyridin-4-yl)-2,2-dimethylhexahydro[1,3]oxazolo[5,4-c]pyridine-1(2H)-carboxylate

To a solution of 5-bromo-4-chloropyridine-3-carbaldehyde (CAS number: 1060802-24-5, ASTATECH, Inc. catalogue number: 66142) (3.58 g) and tert-butyl (3aS,7aR)-2,2-dimethylhexahydro[1,3]oxazolo[5,4-c]pyridine-1(2H)-carboxylate (4.99 g) in DMA (40 mL), triethylamine (4.53 mL) was added and the mixture was stirred at 100°C for 5 hours. The reaction solution was cooled to room temperature, diluted with water and extracted with ethyl acetate. The residue obtained by concentrating the organic layer under reduced pressure was purified by medium pressure preparative liquid chromatography (Hi-flash SI) (n-hexane:ethyl acetate = 90:10 to 0:100) to give a titled compound (6.08 g) having the following properties.
HPLC retention time (min): 0.84;
MS (ESI, Pos.): 440 (M+H)⁺;
¹H-NMR (CDCl₃): δ 10.30, 8.74, 8.72, 4.19-3.93, 3.83-3.72, 3.58-3.27, 2.43-2.17, 2.11-1.94, 1.74-1.43.

### Reference Example 8

### tert-butyl (3aS,7aR)-5-[3-(3,5-difluorophenyl)-5-formylpyridin-4-yl]-2,2-dimethylhexahydro[1,3]oxazolo[5,4-c]pyridine-1(2H)-carboxylate

To a solution of the compound (100 mg) produced in Reference Example 7 in 1,4-dioxane (10 mL), tripotassium phosphate (144 mg), water (3 mL), 3,5-difluorophenylboronic acid (54 mg) and bis(di-tert-butyl(4-dimethylaminophenyl)phosphine)dichloropalladium(II) (4.8 mg) were added and the mixture was stirred at 100°C for 1 hour. The reaction solution was cooled to room temperature, diluted with ethyl acetate and washed with water and a saturated sodium chloride solution. The organic layer was concentrated under reduced pressure to give a titled compound (107 mg) having the following properties.
HPLC retention time (min): 0.92;
MS (ESI, Pos.): 474 (M+H)⁺.

### Reference Example 9

### tert-butyl (3aS,7aR)-5-[3-(5,6-difluoro-1H-benzimidazol-2-yl)-5-(3,5-difluorophenyl)pyridin-4-yl]-2,2-dimethylhexahydro[1,3]oxazolo[5,4-c]pyridine-1(2H)-carboxylate

To a solution of the compound (56 mg) produced in Reference Example 8 and 1,2-diamino-4,5-difluorobenzene (19 mg) in DMA (3 mL), sodium hydrogen sulfite (25 mg) was added and the mixture was stirred at 120°C for 5 hours. The reaction solution was cooled to room temperature, diluted with water and extracted with ethyl acetate. The organic layer was concentrated under reduced pressure to give a titled compound (70 mg) having the following properties.
HPLC retention time (min): 0.95;
MS (ESI, Pos.): 598 (M+H)⁺.

### Example 4

### (3S,4R)-4-amino-1-[3-(5,6-difluoro-1H-benzimidazol-2-yl)-5-(3,5-difluorophenyl)-4-pyridinyl]-3-piperidinol

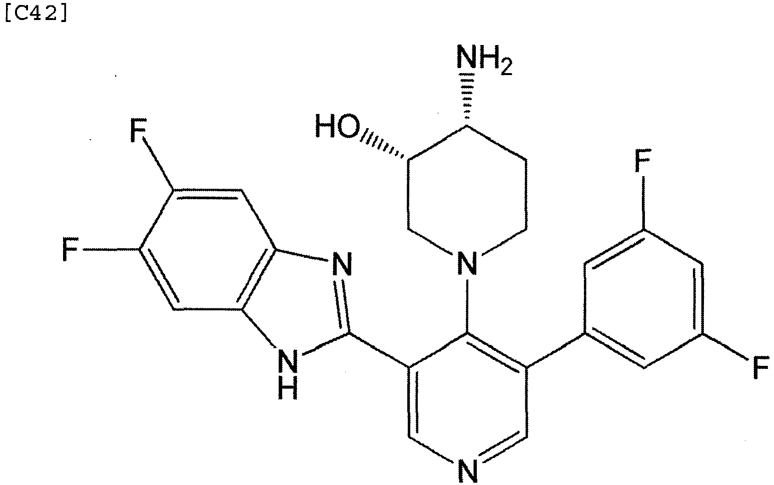

To a solution of the compound (70 mg) produced in Reference Example 9 in dichloromethane (2 mL), trifluoroacetic acid (2 mL) was added at room temperature and the mixture was stirred for 30 minutes and concentrated. The resulting residue was purified by medium pressure preparative liquid chromatography (Hi-flash NH) (ethyl acetate:methanol = 100:0 to 80:20) to give the present compound (37 mg) having the following properties.
HPLC retention time (min): 0.53;
MS (ESI, Pos.): 458 (M+H)⁺;
¹H-NMR (CD₃OD): δ 8.91, 8.35, 7.58, 7.28-6.99, 3.73-3.69, 3.18-2.94, 2.92-2.74, 2.65-2.58, 2.39-2.33, 1.60-1.28.

### Examples 4(1) to 4(16)

The present compounds having the following properties were obtained in the similar procedures as in Reference Example 8 → Reference Example 9 → Example 4 by using a corresponding boronic acid compound instead of 3,5-difluorophenylboronic acid and a corresponding benzene-1,2-diamine compound instead of 1,2-diamino-4,5-difluorobenzene.

### Example 4(1)

### (3S,4R)-4-amino-1-[3-(3,5-difluorophenyl)-5-(5,7-dimethyl-1H-benzimidazol-2-yl)-4-pyridinyl]-3-piperidinol

HPLC retention time (min): 0.52;
MS (ESI, Pos.): 450 (M+H)⁺;
¹H-NMR (CD₃OD): δ 8.85, 8.31, 7.28, 7.14-7.05, 6.95, 3.67, 3.17-3.06, 3.05-2.93, 2.88-2.80, 2.62, 2.60-2.52, 2.44, 2.38-2.31, 1.60-1.42, 1.36-1.27.

### Example 4(2)

### (3S,4R)-4-amino-1-[3-(3,5-difluorophenyl)-5-(7-methyl-1H-benzimidazol-2-yl)-4-pyridinyl]-3-piperidinol

HPLC retention time (min): 0.47;
MS (ESI, Pos.): 436 (M+H)⁺;
¹H-NMR (CD₃OD): δ 8.86, 8.33, 7.52-7.46, 7.23-7.16, 7.14-7.04, 3.70-3.64, 3.18-3.08, 3.06-2.93, 2.89-2.80, 2.65, 2.62-2.54, 2.38-2.31, 1.60-1.43, 1.36-1.26.

### Example 4(3)

### (3S,4R)-4-amino-1-[3-(5,7-dimethyl-1H-benzimidazol-2-yl)-5-phenyl-4-pyridinyl]-3-piperidinol

HPLC retention time (min): 0.50;
MS (ESI, Pos.): 414 (M+H)⁺;
¹H-NMR (CD₃OD): δ 8.89, 8.45, 7.62-7.54, 7.51, 7.27, 3.87, 3.53-3.42, 3.27-2.98, 2.68, 2.66-2.59, 2.53, 1.79-1.62, 1.42-1.32.

### Example 4(4)

### (3S,4R)-4-amino-1-[3-(1H-benzimidazol-2-yl)-5-(3,5-difluorophenyl)-4-pyridinyl]-3-piperidinol

HPLC retention time (min): 0.47;
MS (ESI, Pos.): 422 (M+H)⁺;
¹H-NMR (CD₃OD): δ 8.93, 8.36, 7.73-7.68, 7.36-7.32, 7.17-7.08, 3.71, 3.18-3.11, 3.08-2.95, 2.91-2.83, 2.64-2.54, 2.43-2.35, 1.62-1.47, 1.38-1.30.

### Example 4(5)

### 2-{4-[(3S,4R)-4-amino-3-hydroxy-1-piperidinyl]-5-(3,5-difluorophenyl)-3-pyridinyl}-1H-benzimidazole-5-carbonitrile

HPLC retention time (min): 0.55;
MS (ESI, Pos.): 447 (M+H)⁺;
¹H-NMR (CD₃OD): δ 8.95, 8.39, 8.13, 7.82, 7.62, 7.15-7.03, 3.77-3.72, 3.20-2.97, 2.93-2.85, 2.78-2.69, 2.43-2.35, 1.63-1.28.

### Example 4(6)

### (3S,4R)-4-amino-1-[3-(5,6-difluoro-1H-benzimidazol-2-yl)-5-(2,5-difluorophenyl)-4-pyridinyl]-3-piperidinol

HPLC retention time (min): 0.73;
MS (ESI, Pos.): 458 (M+H)⁺;
¹H-NMR (CD₃OD): δ 8.95, 8.33, 7.62-7.53, 7.40-7.17, 3.73-3.69, 3.12-2.87, 2.63-2.54, 2.44-2.31, 1.55-1.28.

### Example 4(7)

### (3S,4R)-4-amino-1-[3-(5,6-difluoro-1H-benzimidazol-2-yl)-5-(3-fluorophenyl)-4-pyridinyl]-3-piperidinol

HPLC retention time (min): 0.63;
MS (ESI, Pos.): 440 (M+H)⁺;
¹H-NMR (CD₃OD): δ 8.91, 8.34, 7.62-7.50, 7.27-7.17, 3.72-3.68, 3.18-2.93, 2.87-2.77, 2.70-2.60, 2.36-2.26, 1.62-1.43, 1.41-1.30.

### Example 4(8)

### (3S,4R)-4-amino-1-[3-(5,6-difluoro-1H-benzimidazol-2-yl)-5-(3,5-dimethoxyphenyl)-4-pyridinyl]-3-piperidinol

HPLC retention time (min): 0.56;
MS (ESI, Pos.): 482 (M+H)⁺;
¹H-NMR (CD₃OD): δ 8.89, 8.34, 7.59, 6.62-6.50, 3.83, 3.82-3.78, 3.20-2.86, 2.50-2.41, 1.78-1.60, 1.52-1.40.

### Example 4(9)

### (3S,4R)-4-amino-1-[3-(5-chloro-2-fluorophenyl)-5-(5,6-difluoro-1H-benzimidazol-2-yl)-4-pyridinyl]-3-piperidinol

HPLC retention time (min): 0.58;
MS (ESI, Pos.): 474 (M+H)⁺;
¹H-NMR (CD₃OD): δ 8.89, 8.41, 7.70-7.50, 7.40-7.26, 3.90-3.86, 3.28-2.95, 2.60-2.48, 1.80-1.62, 1.57-1.43.

### Example 4(10)

### (3S,4R)-4-amino-1-[3-(5,6-difluoro-1H-benzimidazol-2-yl)-5-(2,5-dimethyl-3-furyl)-4-pyridinyl]-3-piperidinol

HPLC retention time (min): 0.55;
MS (ESI, Pos.): 440 (M+H)⁺;
¹H-NMR (CD₃OD): δ 8.90, 8.22, 7.56, 6.02, 3.90-3.82, 3.14-2.75, 2.31, 2.22, 1.75-1.57, 1.52-1.39.

### Example 4(11)

### (3S,4R)-4-amino-1-[3-(3,5-difluorophenyl)-5-(5-fluoro-6-methoxy-1H-benzimidazol-2-yl)-4-pyridinyl]-3-piperidinol

HPLC retention time (min): 0.55;
MS (ESI, Pos.): 470 (M+H)⁺;
¹H-NMR (CD₃OD): δ 8.88, 8.32, 7.41, 7.31, 7.15-7.04, 3.95, 3.73-3.68, 3.17-3.06, 3.06-2.97, 2.89-2.80, 2.65-2.56, 2.39-2.31, 1.62-1.45, 1.40-1.25.

### Example 4(12)

### (3S,4R)-4-amino-1-[3-(3,5-difluorophenyl)-5-(6-methoxy-1H-benzimidazol-2-yl)-4-pyridinyl]-3-piperidinol

HPLC retention time (min): 0.56;
MS (ESI, Pos.): 452 (M+H)⁺;
¹H-NMR (CD₃OD): δ 8.85, 8.32, 7.57, 7.19-7.03, 6.95, 3.87, 3.80-3.75, 3.20-3.10, 3.07-2.95, 2.96-2.80, 2.43-2.37, 1.75-1.57, 1.46-1.34.

### Example 4(13)

### (3S,4R)-4-amino-1-[3-(5,6-difluoro-1H-benzimidazol-2-yl)-5-(2,3,5-trifluorophenyl)-4-pyridinyl]-3-piperidinol

HPLC retention time (min): 0.58;
MS (ESI, Pos.): 476 (M+H)⁺;
¹H-NMR (CD₃OD): δ 8.94, 8.36, 7.58, 7.43-7.30, 7.20-7.03, 3.78-3.72, 3.12-2.89, 2.74-2.62, 2.50-2.37, 1.57-1.28.

### Example 4(14)

### (3S,4R)-4-amino-1-[3-(5,6-difluoro-1H-benzimidazol-2-yl)-5-(3,5-difluoro-2-methoxyphenyl)-4-pyridinyl]-3-piperidinol

HPLC retention time (min): 0.57;
MS (ESI, Pos.): 488 (M+H)⁺;
¹H-NMR (CD₃OD): δ 8.95, 8.89, 8.34, 7.59, 7.27-7.15, 7.13-7.05, 6.96-6.85, 3.90, 3.87-3.76, 3.61, 3.28-2.87, 2.54-2.30, 1.75-1.37.

### Example 4(15)

### (3S,4R)-4-amino-1-[3-(2,5-difluorophenyl)-5-(6-methoxy-1H-benzimidazol-2-yl)-4-pyridinyl]-3-piperidinol

Properties: amorphous;
HPLC retention time (min): 0.57;
MS (ESI, Pos.): 452 (M+H)⁺;
¹H-NMR (CD₃OD) : δ 8.92, 8.29, 7.55, 7.39-7.13, 7.15, 6.94, 3.87, 3.72-3.67, 3.10-2.86, 2.61-2.51, 2.44-2.28, 1.60-1.42, 1.26-1.23.

### Example 4(16)

### Methyl 2-{4-[(3S,4R)-4-amino-3-hydroxy-1-piperidinyl]-5-(2,5-difluorophenyl)-3-pyridinyl}-5-fluoro-1H-benzimidazole-7-carboxylate

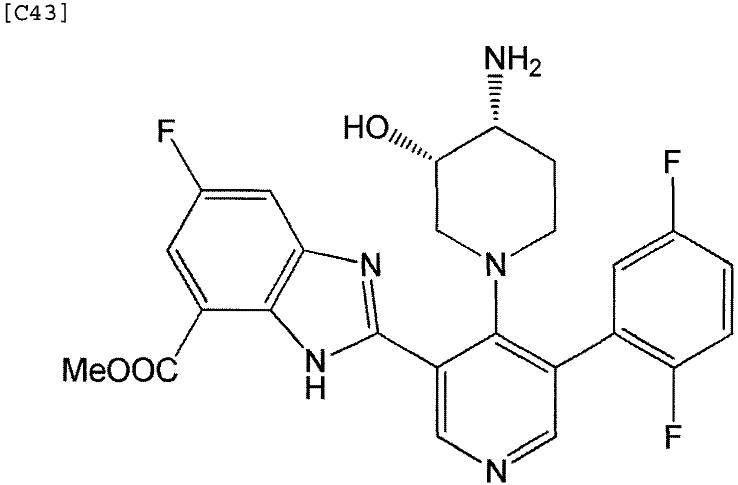

Properties: amorphous;
HPLC retention time (min): 0.59;
MS (ESI, Pos.): 498 (M+H)⁺;
¹H-NMR (DMSO-d₆): δ 8.62, 8.34, 7.89, 7.64, 7.55-7.30, 3.95. 2.98-2.07, 1.15-1.05.

### Example 5

### (3S,4R)-1-[3-(5,6-difluoro-1H-benzimidazol-2-yl)-5-(3,5-difluorophenyl)-4-pyridinyl]-4-(3-oxetanylamino)-3-piperidinol

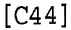

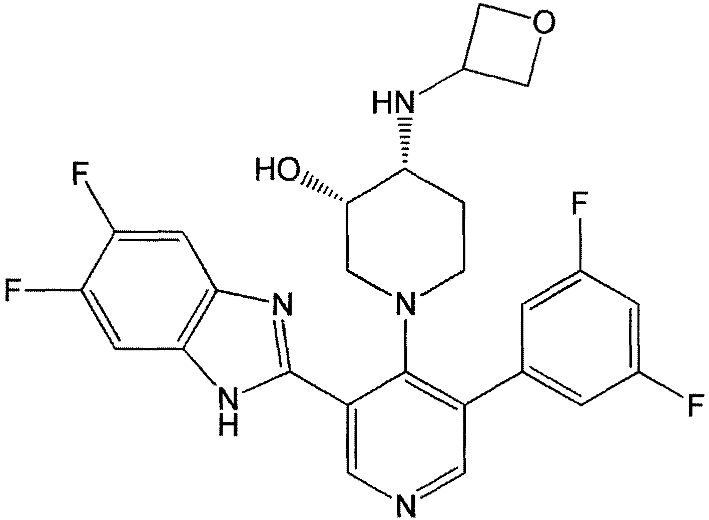

To a solution of the compound (72 mg) produced in Example 4 and 3-oxetanone (90 mg) in methanol (5 mL), 2-picoline-borane complex (34 mg) and acetic acid (0.1 mL) were added and the mixture was stirred at room temperature for 24 hours. The residue obtained by concentrating the reaction solution under reduced pressure was purified by medium pressure preparative liquid chromatography (Hi-flash NH) (ethyl acetate:methanol = 100:0 to 80:20) to give the present compound (60 mg) having the following properties. Properties: amorphous;
HPLC retention time (min): 0.59;
MS (ESI, Pos.): 514 (M+H)⁺;
¹H-NMR (CDCl₃): δ 12.55, 9.34, 8.36, 7.67-7.55, 7.29-7.16, 6.99-6.79, 4.89-4.80, 4.50-4.36, 4.08-3.97, 3.75-3.69, 3.18-2.85, 2.55-2.45, 2.37-2.29, 1.65-1.50, 1.40-1.31.

### Examples 5(1) to 5(9)

The present compounds having the following properties were obtained in the similar procedures as in Reference Example 8 → Reference Example 9 → Example 4 → Example 5 by using a corresponding boronic acid compound instead of 3,5-difluorophenylboronic acid, a corresponding benzene-1,2-diamine compound instead of 1,2-diamino-4,5-difluorobenzene and a corresponding aldehyde compound instead of 3-oxetanone.

### Example 5(1)

### (3S,4R)-1-[3-(5,6-difluoro-1H-benzimidazol-2-yl)-5-(3,5-difluorophenyl)-4-pyridinyl]-4-[(3,3,3-trifluoropropyl)amino]-3-piperidinol

HPLC retention time (min): 0.69;
MS (ESI, Pos.): 554 (M+H)⁺;
¹H-NMR (CD₃OD): δ 8.91, 8.35, 7.59, 7.17-7.03, 3.92-3.88, 3.20-2.74, 2.55-2.30, 1.58-1.39.

### Example 5(2)

### (3S,4R)-1-[3-(3-chloro-5-fluorophenyl)-5-(5,6-difluoro-1H-benzimidazol-2-yl)-4-pyridinyl]-4-(3-oxetanylamino)-3-piperidinol

HPLC retention time (min): 0.60;
MS (ESI, Pos.): 530 (M+H)⁺;
¹H-NMR (CDCl₃): δ 12.51, 9.36, 8.37, 7.67-7.58, 7.35-6.91, 4.89-4.80, 4.50-4.37, 4.07-3.98, 3.74-3.63, 3.19-2.85, 2.55-2.45, 2.36-2.28, 1.54-1.33.

### Example 5(3)

### (3S,4R)-1-[3-(3-chloro-5-fluorophenyl)-5-(5,6-difluoro-1H-benzimidazol-2-yl)-4-pyridinyl]-4-[(3,3,3-trifluoropropyl)amino]-3-piperidinol

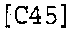

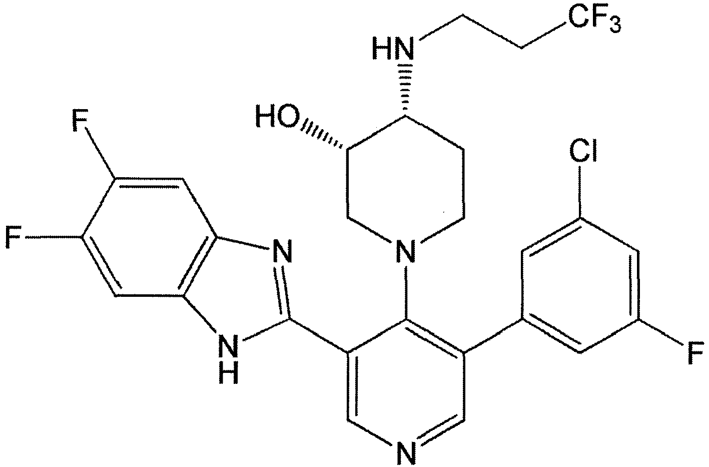

HPLC retention time (min): 0.70;
MS (ESI, Pos.): 570 (M+H)⁺;
¹H-NMR (CDCl₃): δ 12.58, 9.36, 8.37, 7.67-7.58, 7.26-7.11, 7.01-6.91, 3.86-3.81, 3.19-3.04, 2.99-2.86, 2.61-2.52, 2.44-2.24, 1.51-1.37.

### Example 5(4)

### (3S,4R)-1-[3-(5,6-difluoro-1H-benzimidazol-2-yl)-5-(2,5-difluorophenyl)-4-pyridinyl]-4-(ethylamino)-3-piperidinol

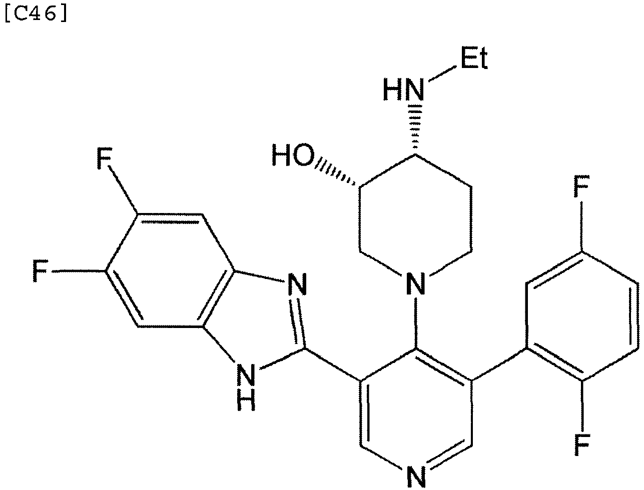

HPLC retention time (min): 0.60;
MS (ESI, Pos.): 486 (M+H)⁺;
¹H-NMR (CD₃OD): δ 8.96, 8.34, 7.66-7.58, 7.39-7.18, 3.97-3.93, 3.11-2.89, 2.76-2.30, 1.61-1.42, 1.16.

### Example 5(5)

### (3S,4R)-1-[3-(5,6-difluoro-1H-benzimidazol-2-yl)-5-(3-fluoro-5-methoxyphenyl)-4-pyridinyl]-4-[(3,3,3-trifluoropropyl)amino]-3-piperidinol

HPLC retention time (min): 0.72;
MS (ESI, Pos.): 566 (M+H)⁺;
¹H-NMR (CD₃OD): δ 8.93, 8.34, 7.64-7.54, 6.87-6.73, 3.92-3.85, 3.87, 3.21-2.96, 2.94-2.75, 2.55-2.28, 1.58-1.38.

### Example 5(6)

### (3S,4R)-1-[3-(3,5-difluorophenyl)-5-(6-fluoro-5-methoxy-1H-benzimidazol-2-yl)-4-pyridinyl]-4-[(3,3,3-trifluoropropyl)amino]-3-piperidinol

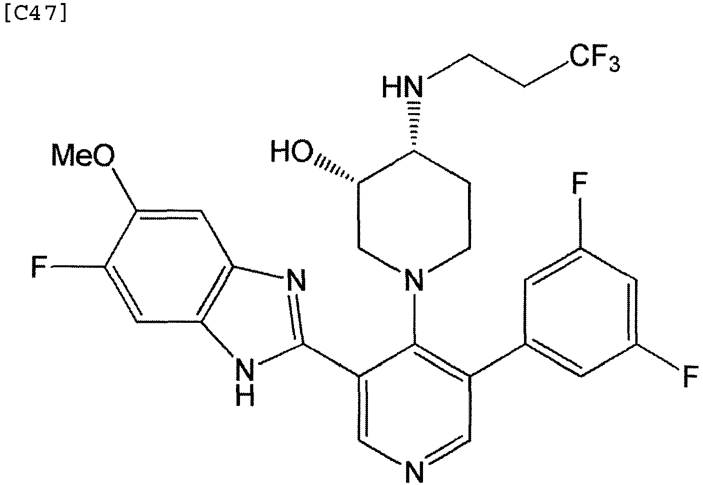

HPLC retention time (min): 0.67;
MS (ESI, Pos.): 566 (M+H)⁺;
¹H-NMR (CD₃OD): δ 8.87, 8.30, 7.41, 7.30, 7.14-7.05, 3.94, 3.99-3.82, 3.17-3.07, 3.05-2.97, 2.92-2.71, 2.57-2.45, 2.44-2.28, 1.56-1.47, 1.47-1.36.

### Example 5(7)

### (3S,4R)-1-[3-(2,5-difluorophenyl)-5-(6-methoxy-1H-benzimidazol-2-yl)-4-pyridinyl]-4-(3-oxetanylamino)-3-piperidinol

HPLC retention time (min): 0.55;
MS (ESI, Pos.): 508 (M+H)⁺;
¹H-NMR (CD₃OD): δ 8.93, 8.29, 7.60, 7.41-7.14, 6.96, 4.82-4.68, 4.54, 4.43, 4.09-3.98, 3.89, 3.72-3.67, 3.12-2.87, 2.47-2.30, 1.62-1.43, 1.33-1.22.

### Example 5(8)

### (3S,4R)-1-[3-(3,5-difluorophenyl)-5-(6-fluoro-5-methoxy-1H-benzimidazol-2-yl)-4-pyridinyl]-4-(3-oxetanylamino)-3-piperidinol

HPLC retention time (min): 0.57;
MS (ESI, Pos.): 526 (M+H)⁺;
¹H-NMR (CD₃OD): δ 8.87, 8.32-8.28, 7.45, 7.35, 7.21-7.04, 4.73, 4.56, 4.45, 4.13-4.00, 3.95, 3.81-3.64, 3.16-3.05, 3.03-2.88, 2.87-2.73, 2.50-2.39, 2.36-2.26, 1.62-1.44, 1.36-1.18.

### Example 5(9)

### (3S,4R)-1-[3-(2,5-difluorophenyl)-5-(5-fluoro-6-methoxy-1H-benzimidazol-2-yl)-4-pyridinyl]-4-(3-oxetanylamino)-3-piperidinol

HPLC retention time (min): 0.56;
MS (ESI, Pos.): 526 (M+H)⁺;
¹H-NMR (CD₃OD): δ 8.93, 8.29, 7.48, 7.41-7.15, 7.35, 4.81-4.66, 4.55, 4.44, 4.13-3.97, 3.96, 3.72-3.66, 3.12-2.84, 2.46-2.27, 1.61-1.42, 1.34-1.22.

### Reference Example 10

### tert-butyl (3aS,7aR)-5-(2-bromo-3-formylpyridin-4-yl)-2,2-dimethylhexahydro[1,3]oxazolo[5,4-c]pyridine-1(2H)-carboxylate

To a solution of 2-bromo-4-chloropyridine-3-carbaldehyde (4.5 g) (CAS number: 1289197-78-9, SIGMA ALDRICH catalogue number: 762180) and tert-butyl (3aS,7aR)-2,2-dimethylhexahydro[1,3]oxazolo[5,4-c]pyridine-1(2H)-carboxylate (5.2 g) in DMA (45 mL), triethylamine (8.5 mL) was added and the mixture was stirred at 80°C for 2 hours. The reaction solution was cooled to room temperature, diluted with water and extracted with ethyl acetate. The residue obtained by concentrating the organic layer under reduced pressure was purified by medium pressure preparative liquid chromatography (Hi-flash SI) (n-hexane:ethyl acetate = 90:10 to 0:100) to give a titled compound (7.5 g) having the following properties. HPLC retention time (min): 0.99;
MS (ESI, Pos.): 440 (M+H)⁺.

### Reference Example 11

### tert-butyl (3aS,7aR)-5-[3-formyl-2-(morpholin-4-yl)pyridin-4-yl]-2,2-dimethylhexahydro[1,3]oxazolo[5,4-c]pyridine-1(2H)-carboxylate

The compound (300 mg) produced in Reference Example 10 was dissolved in DMA (3.0 mL) to which triethylamine (0.190 mL) and morpholine (70.7 µL) were added. The reaction solution was stirred at 100°C for 1 hour, heated to 120°C and stirred for 17 hours. The reaction solution was cooled to room temperature and added with water. The organic layer was extracted three times with ethyl acetate and the combined organic layer was washed with a saturated sodium chloride solution. The organic layer was added with sodium sulphate and filtered. The residue obtained by concentrating the filtrate was purified by medium pressure preparative liquid chromatography (Hi-flash SI) (hexane-ethyl acetate = 90:10 to 0:100) to give a titled compound.

### Reference Example 12

### tert-butyl (3aS,7aR)-5-[5-bromo-3-formyl-2-(morpholin-4-yl)pyridin-4-yl]-2,2-dimethylhexahydro[1,3]oxazolo[5,4-c]pyridine-1(2H)-carboxylate

The compound produced in Reference Example 11 was dissolved in acetonitrile (1.5 mL) to which N-bromosuccinimide (94.6 mg) was added and the mixture was stirred at room temperature for 10 minutes. To the reaction solution, a 10% sodium thiosulphate aqueous solution and a saturated sodium hydrogen sulphate aqueous solution were serially added to terminate the reaction. The reaction solution was extracted twice with ethyl acetate and organic layers were combined. The organic layer was washed with a saturated sodium chloride solution and dried over sodium sulphate. The extract was filtered and then concentrated, the resulting residue was purified by silica gel column chromatography (Fuji Silysia SI50) (n-hexane:ethyl acetate = 90:10 to 0:100) to give a titled compound (192 mg) having the following properties. ¹H-NMR (CDCl₃): δ 9.66, 8.15, 4.16-4.09, 3.98, 3.96-3.72, 3.64, 3.62-3.47, 3.30, 2.38-2.05, 1.74-1.61, 1.58-1.41.

### Example 6

### Methyl 2-{4-[(3S,4R)-4-amino-3-hydroxy-1-piperidinyl]-5-(3,5-difluorophenyl)-2-(4-morpholinyl)-3-pyridinyl}-5-fluoro-1H-benzimidazole-7-carboxylate

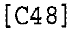

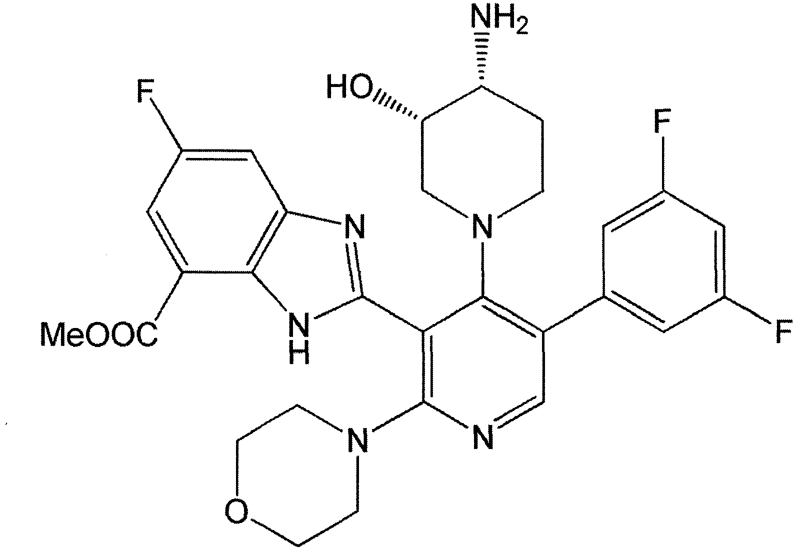

The present compound having the following properties was obtained in the similar procedures as in Reference Example 4 → Reference Example 5 → Example 1 by using the compound produced in Reference Example 12 instead of the compound produced in Reference Example 3 and methyl 2,3-diamino-5-fluorobenzoate instead of methyl 2,3-diaminobenzoate.
Properties: amorphous;
HPLC retention time (min): 0.65;
MS (ESI, Pos.): 583 (M+H)⁺;
¹H-NMR (CD₃OD): δ 8.10, 7.74, 7.71, 7.09, 6.99, 4.03, 3.47, 3.40-3.34, 3.15-2.95, 2.92-2.74, 2.46-2.37, 2.25-2.18, 1.10-0.82.

### Biological Example 1:

### Evaluation of SSTR2 agonistic activity using cells expressing human SSTR2

### [Procedures]

### (1) Isolation of human SSTR2 gene

Human brain cDNA was purchased from Ambion (catalogue No.: 7962; Lot No.: 040200121). PCR primers, hSSTR2_F1_XhoI: 5'-CACCCTCGAGGACATGGCGGATGAGCCACTCAAT-3' (SEQ ID NO: 1) and hSSTR2_R1_EcoRI: 5'-CCTTGAATTCGATACTGGTTTGGAGGTCTCCATT-3' (SEQ ID NO: 2) were designed on the basis of the sequence GenBank NM_001050.

PCR reaction (95°C for 2 min. → [98°C for 10 sec., 60°C for 30 sec. and 68°C for 90 sec.] × 30 cycles) was carried out using the human brain cDNA as a template and using KOD -plus-(TOYOBO Co., Ltd.). The amplified PCR product was subjected to 1% agarose gel electrophoresis, purified using QIAquick Gel Extraction Kit (QIAGEN) and digested with restriction enzymes *Xho*I and *Eco*RI. The digested fragments were ligated to an expression vector (pIRESneo-Myc) using the DNA Ligation Kit Ver.2 (Takara) and used for transformation of *E*. *coli* DH5a. The plasmid pIRESneo-Myc/hSSTR2 was prepared and the DNA sequence thereof was confirmed.

### (2) Culture of CHO-K1 cells

CHO-K1 (-) was cultured in Ham's F-12 medium (containing foetal bovine serum (10%), penicillin (100 U/mL) and streptomycin (0.1 mg/mL)). The transduced cells were cultured in the same medium supplemented with Geneticin (1 mg/mL).

### (3) Transduction of CHO-K1 cells

CHO-K1(-) cells were transduced with the plasmid pIRESneo-Myc/hSSTR2 using Lipofectamine 2000 (Invitrogen). After 48 hours, selection was carried out by replacing the medium with the one containing 1 mg/mL of Geneticin to establish a stable overexpressing cell line (SSTR2-CHO-K1).

### (4) Evaluation of SSTR2 agonistic activity

The human SSTR2 agonistic activity of a test compound was evaluated according to the following procedures by using a suppression activity of intracellular cyclic AMP (cAMP) production by forskolin stimulation as an index. SSTR2-CHO-K1 cells suspended in Ham's F-12 medium (containing foetal bovine serum (10%), penicillin (100 U/mL) and streptomycin (0.1 mg/mL)) supplemented with 0.25 mg/mL of Geneticin were inoculated into a 96-well plate at a density of 4.0 × 10⁴ cells/0.1 mL per well. On the next day, the medium was removed and washed twice with 0.1 mL of wash buffer [0.1% bovine serum albumin (BSA), 20 mmol/L 4-(2-hydroxyethyl)-1-piperazineethane sulphonic acid (HEPES)-containing Hank's balanced salt solution (HBSS)]. An assay buffer [500 nmol/L 3-isobutyl-1-methylxanthine (IBMX), 0.1% BSA, 20 mmol/L HEPES-containing HBSS] was added to the wells at 0.06 mL per well and the plate was incubated for 15 minutes under the conditions of 5% carbon dioxide and 37°C. Thereafter, an assay buffer containing a test compound at a concentration twice as high as the final concentration and 0.02 mmol/L of forskolin was added to wells at 0.06 mL per well and the plate was incubated for 30 minutes under the conditions of 5% carbon dioxide and 37°C. Thereafter, the Assay/Lysis buffer included in the cAMP-Screen® kit (available from Applied Biosystems) was added to wells at 0.12 mL per well and the plate was incubated for 30 minutes under the conditions of 5% carbon dioxide and 37°C. The concentration of cAMP in samples were measured by ELISA according to the instruction of the kit. The 50% effective concentration (EC₅₀) of the human SSTR2 agonistic activity was calculated, after determining the percentage (%) of suppression of cAMP production by forskolin stimulation for each sample with the percentage of 1000 nmol/L of octreotide being taken as 100%, by non-linear regression analysis with respect to the independent variable of the common logarithmic concentration of a test compound and the dependent variable of the percentage of the corresponding concentration.

### [Results]

The present compounds exhibited EC₅₀ values of 1 nmol/L or less and strong SSTR2 agonistic activity. For example, the compound produced in Example 1(2) had an EC₅₀ value of 0.032 nmol/L, the compound produced in Example 2(3) had an EC₅₀ value of 0.019 nmol/L, the compound produced in Example 2(12) had an EC₅₀ value of 0.016 nmol/L, the compound produced in Example 2(13) had an EC₅₀ value of 0.017 nmol/L, the compound produced in Example 3 had an EC₅₀ value of 0.027 nmol/L, the compound produced in Example 3(4) had an EC₅₀ value of 0.056 nmol/L, the compound produced in Example 3(5) had an EC₅₀ value of 0.023 nmol/L, the compound produced in Example 3(12) had an EC₅₀ value of 1.0 nmol/L, the compound produced in Example 3(15) had an EC₅₀ value of 0.47 nmol/L, the compound produced in Example 4 had an EC₅₀ value of 0.015 nmol/L, the compound produced in Example 4(4) had an EC₅₀ value of 0.0093 nmol/L, the compound produced in Example 4(15) had an EC₅₀ value of 0.012 nmol/L, the compound produced in Example 4(16) had an EC₅₀ value of 0.0050 nmol/L, the compound produced in Example 5 had an EC₅₀ value of 0.14 nmol/L, the compound produced in Example 5(3) had an EC₅₀ value of 0.28 nmol/L, the compound produced in Example 5(4) had an EC₅₀ value of 0.031 nmol/L, the compound produced in Example 5(5) had an EC₅₀ value of 0.42 nmol/L, the compound produced in Example 5(6) had an EC₅₀ value of 0.067 nmol/L and the compound produced in Example 6 had an EC₅₀ value of 0.081 nmol/L.

According to the present evaluation system, the compound of, for example, Example 21(27) disclosed in WO 2014/007228 had an EC₅₀ value of 0.026 nmol/L, the compound of Example 21(28) had an EC₅₀ value of 0.019 nmol/L and the compound of Example 21(29) had an EC₅₀ value of 0.038 nmol/L.

### Biological Example 2:

### Evaluation of hERG K⁺ channel inhibitory activity using cells expressing human ERG

### [Procedures]

### (1) Culture of CHO-K1 cells having human ERG gene (ether-a-go-go related gene) introduced therein

CHO-K1 cells having the human ERG gene (ether-a-go-go related gene) introduced therein were subcultured in an F-12 medium [F-12 Nutrient Mixture (HAM)] containing 10% inactivated foetal bovine serum, 100 IU/mL penicillin-100 µg/mL streptomycin and 200 µg/mL geneticin.

### (2) Evaluation of hERG K⁺ channel inhibitory activity

Patch-clamp was performed in a full-automated manner using CHO-K1 cells having the human ERG gene introduced therein and having a perforated patch formed therein with amphotericin B. For the experiment, a 384-well PatchPlate™ was used and the cell suspension and compounds were added thereto. The IKr current was measured with command voltages of holding potential of -80 mV, depolarization potential of +40 mV (2 seconds) and repolarization potential of -50 mV (2 seconds). The stimulation frequency was twice (single pulse stimulations) before and after the treatment with a drug (the concentrations of a compound for evaluation were 1, 3 and 10 µmol/L, a 5-minute incubation), and the Ikr maximum current was measured. The inhibition (%) on the CHO-K1 cells having the human ERG gene introduced therein was determined by correcting the variation in the maximum tail current before and after addition of the test substance by the variation thereof in the group treated with a medium (inhibition (%) = (1-variation in current before and after addition of a test substance / variation in current before and after addition of a medium) × 100). The 50% inhibitory concentration (IC₅₀ value) was calculated from a line connecting two points on both sides of the inhibition of 50%.

### [Results]

The present compounds exhibited IC₅₀ values of 3 (µmol/L or more, and thus the hERG inhibitory activity was sufficiently low compared to the SSTR2 agonistic activity. For example, the compound produced in Example 1(2) had an IC₅₀ value of 10 µmol/L, the compound produced in Example 2(3) had an IC₅₀ value of 5.2 µmol/L, the compound produced in Example 2(12) had an IC₅₀ value of 9.4 µmol/L, the compound produced in Example 2(13) had an IC₅₀ value of 10 µmol/L, the compound produced in Example 3 had an IC₅₀ value of 10 µmol/L, the compound produced in Example 3(4) had an IC₅₀ value of 10 µmol/L, the compound produced in Example 3(5) had an IC₅₀ value of 9.6 µmol/L, the compound produced in Example 3(12) had an IC₅₀ value of 5.1 µmol/L, the compound produced in Example 3(15) had an IC₅₀ value of 5.8 µmol/L, the compound produced in Example 4 had an IC₅₀ value of 6.9 µmol/L, the compound produced in Example 4(4) had an IC₅₀ value of 6.7 µmol/L, the compound produced in Example 4(15) had an IC₅₀ value of 9.1 µmol/L, the compound produced in Example 4(16) had an IC₅₀ value of 3.9 µmol/L, the compound produced in Example 5 had an IC₅₀ value of 7.6 µmol/L, the compound produced in Example 5(3) had an IC₅₀ value of 10 µmol/L, the compound produced in Example 5(4) had an IC₅₀ value of 8.4 µmol/L, the compound produced in Example 5(5) had an IC₅₀ value of 10 µmol/L, the compound produced in Example 5(6) had an IC₅₀ value of 10 µmol/L and the compound produced in Example 6 had an IC₅₀ value of 4.4 µmol/L.

According to the present evaluation system, the compound of, for example, Example 21(27) disclosed in WO 2014/007228 had an IC₅₀ value of 1.0 µmol/L, the compound of Example 21(28) had an IC₅₀ value of 1.6 µmol/L and the compound of Example 21(29) had an IC₅₀ value of 0.10 µmol/L.

### Biological Example 3:

### Evaluation of phospholipidosis induction ability using cultured cell line

### [Procedures]

Chinese hamster lung-derived CHL/IU cells were suspended in an MEM-E culture solution containing non-essential amino acids, sodium pyruvate and 10% foetal bovine serum and seeded in a 96-well plate. The cells cultured overnight in an incubator (5% carbon dioxide, 95% air, 37°C) were further cultured for 24 hours after replacing the culture solution with a culture solution containing 6.25, 12.5, 25, 50 or 100 µmol/L of a test substance and NBD-PE (fluorescence label N-(7-nitrobenz-2-oxa-1,3-diazol-4-yl)-1,2-dihexadecanoyl-sn-glycero-3-phosphoethanolamine triethylammonium salt) obtained from Molecular Probes. The phospholipidosis induction ability was evaluated by measuring the concentration of NBD-PE incorporated into the cells. Specifically, the cells were washed twice with PBS and then measured for the fluorescence of NBD-PE (excitation wavelength: 485 nm, fluorescence wavelength: 535 nm) incorporated in the cells on a SpectraMax plate reader available from Molecular Devices, LLC. for evaluation. After measurement of the fluorescence of NBD-PE, the cell viability was evaluated with Premix WST-1 Cell Proliferation Assay System available from TaKaRa Bio Inc. The phospholipidosis induction ability was judged to be positive when, at a dose that provided a cell viability of 50% or more, the reaction (the concentration of NBD-PE incorporated after administration of a test substance) was 25% or more of the maximum reaction of a positive control amiodarone (the concentration of NBD-PE incorporated into the cells after administration of amiodarone was regarded as 100%).

### [Results]

The present compounds at 6.25 µmol/L or more were judged to be positive in phospholipidosis induction ability and exhibited sufficiently low phospholipidosis induction ability in vitro. For example, the compound produced in Example 1(2), the compound produced in Example 3, the compound produced in Example 3(5), the compound produced in Example 3(12), the compound produced in Example 5, the compound produced in Example 5(3), the compound produced in Example 5(4), the compound produced in Example 5(5) and the compound produced in Example 5(6) were judged to be negative in phospholipidosis induction ability. For example, the compound produced in Example 2(3) at 12.5 µmol/L, the compound produced in Example 2(12) at 50 µmol/L or more, the compound produced in Example 2(13) at 25 µmol/L or more, the compound produced in Example 3(4) at 50 µmol/L or more, the compound produced in Example 3(15) at 25 µmol/L or more, the compound produced in Example 4 at 12.5 µmol/L or more, the compound produced in Example 4(4) at 25 µmol/L, the compound produced in Example 4(15) at 25 µmol/L, the compound produced in Example 4(16) at 25 µmol/L and the compound produced in Example 6 at 50 µmol/L were judged to be positive in phospholipidosis induction ability.

According to the present evaluation system, the compound of, for example, Example 21(27) disclosed in WO 2014/007228 at 3.125 µmol/L or more, the compound of Example 21(28) at 3.125 µmol/L and the compound of Example 21(29) at 1.56 µmol/L or more were judged to be positive in phospholipidosis induction ability.

### Biological Example 4:

### Evaluation of suppression of growth hormone (GH) secretion using rats

### [Procedures]

A medium (distilled water (Otsuka distilled water, Otsuka Pharmaceutical Factory, Inc.)) or a test compound dissolved in the medium was orally administered to rats (7-week old male Crl:CD(SD) IGS rats (Charles River Laboratories Japan, Inc.)) and after 7 hours and 57 minutes, the animals were administered with 50 mg/kg of pentobarbital sodium (Somnopentyl, Kyoritsu Seiyaku Corporation) via the tail vein for anaesthesia. Three minutes after administration of pentobarbital sodium, the rats were administered with 0.01 mg/kg of growth hormone-releasing hormone (GHRH, Bachem) via the tail vein to induce GH secretion. In order to measure the blood GH concentration, 0.2 mL of blood was collected through the jugular vein at 5 minutes after administration of GHRH. The collected blood was centrifuged at 4°C, 13,000 × g for 5 minutes to obtain plasma. The blood GH concentration was measured by using Rat/Mouse Growth Hormone ELISA (Millipore) following the instruction of the kit. The percentage (%) of suppression of GH secretion was determined by using the obtained blood GH concentration and the equation {[suppression (%) of GH secretion] = ([blood GH concentration of the group administered with the medium] - [blood GH concentration of the group administered with a test compound]) / [blood GH concentration of the group administered with the medium] × 100}. In the equation, the group administered with the medium represents the group of animals administered with the medium and the group administered with a test compound represents the group of animals administered with a test compound dissolved in the medium.

### [Results]

The present compounds exhibited strong suppression of GH secretion. For example, the compound produced in Example 2(3) at a dosage of 1 mg/kg had a suppression of GH secretion of 76%, the compound produced in Example 3(4) at a dosage of 1 mg/kg had a suppression of GH secretion of 71%, the compound produced in Example 3(5) at a dosage of 1 mg/kg had a suppression of GH secretion of 83%, the compound produced in Example 3(12) at a dosage of 1 mg/kg had a suppression of GH secretion of 85%, the compound produced in Example 3(15) at a dosage of 1 mg/kg had a suppression of GH secretion of 66%, the compound produced in Example 4(16) at a dosage of 1 mg/kg had a suppression of GH secretion of 67%, the compound produced in Example 5 at a dosage of 1 mg/kg had a suppression of GH secretion of 92%, the compound produced in Example 5(4) at a dosage of 1 mg/kg had a suppression of GH secretion of 90% and the compound produced in Example 5(6) at a dosage of 1 mg/kg had a suppression of GH secretion of 83%.

### Formulation Example 1:

### Tablets containing 5 mg of methyl 2-{4-[(3S,4R)-4-amino-3-hydroxy-1-piperidinyl]-5-(3-chloro-5-fluorophenyl)-2-methyl-3-pyridinyl}-5-fluoro-1H-benzimidazole-7-carboxylate

The following components can be mixed and compressed to tablets according to standard methods to obtain 10,000 tablets each containing 5 mg of the active component.
- Methyl 2-{4-[(3S,4R)-4-amino-3-hydroxy-1-piperidinyl]-5-(3-chloro-5-fluorophenyl)-2-methyl-3-pyridinyl}-5-fluoro-1H-benzimidazole-7-carboxylate: 50 g
- Carboxymethylcellulose calcium (disintegrating agent): 20 g
- Magnesium stearate (lubricant): 10 g
- Microcrystalline cellulose: 920 g

### Formulation Example 2:

### Tablets containing 5 mg of (3S,4R)-1-[3-(5,6-difluoro-1H-benzimidazol-2-yl)-5-(3,5-difluorophenyl)-4-pyridinyl]-4-(3-oxetanylamino)-3-piperidinol

The following components can be mixed and compressed to tablets according to standard methods to obtain 10,000 tablets each containing 5 mg of the active component.
- (3S,4R)-1-[3-(5,6-difluoro-1H-benzimidazol-2-yl)-5-(3,5-difluorophenyl)-4-pyridinyl]-4-(3-oxetanylamino)-3-piperidinol:50 g
- Carboxymethylcellulose calcium (disintegrating agent): 20 g
- Magnesium stearate (lubricant): 10 g
- Microcrystalline cellulose: 920 g

### Formulation Example 3:

### Injections containing 20 mg of methyl 2-{4-[(3S,4R)-4-amino-3-hydroxy-1-piperidinyl]-5-(3,5-difluorophenyl)-2-(4-morpholinyl)-3-pyridinyl}-5-fluoro-1H-benzimidazole-7-carboxylate

The following components can be mixed according to the standard method, and the solution can be then sterilised according to the standard method, divided into ampoules at 5-mL aliquot and lyophilised according to the standard method to obtain 10,000 ampoules each containing 20 mg of the active component.
- Methyl 2-{4-[(3S,4R)-4-amino-3-hydroxy-1-piperidinyl]-5-(3,5-difluorophenyl)-2-(4-morpholinyl)-3-pyridinyl}-5-fluoro-1H-benzimidazole-7-carboxylate: 200 g
- Mannitol: 20 g
- Distilled water: 50 L

### [Industrial Applicability]

The present compound has strong agonistic activity for a somatostatin receptor, particularly for somatostatin receptor subtype 2, and thus is useful as a prophylactic and/or therapeutic agent for various diseases in which somatostatin *per se* or a hormone modulated by somatostatin is involved, particularly acromegaly and gastrointestinal symptoms accompanying gastrointestinal obstruction.

## Claims

1. A compound represented by the general formula (I): 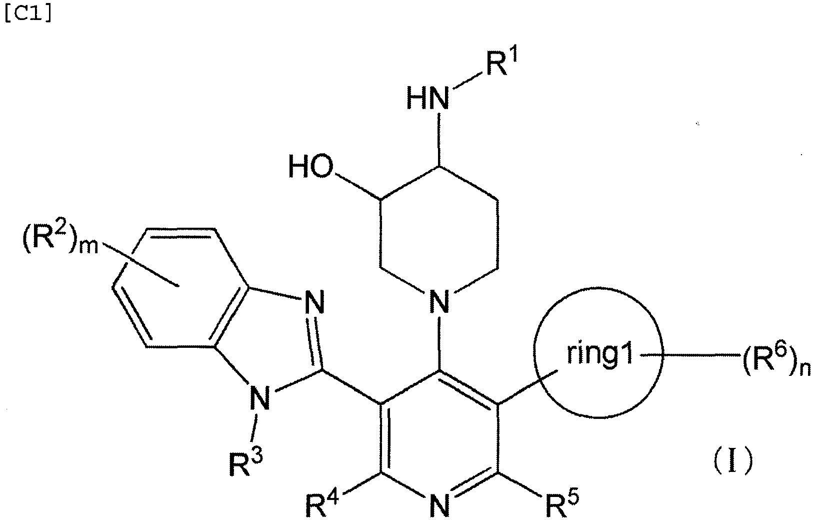 wherein, R¹ represents 3-oxetanyl, 3,3,3-trifluoropropyl, ethyl or a hydrogen atom; R² represents a Cl-4 alkyl, a C1-4 alkoxy, a halogen, nitrile or a C1-4 alkoxycarbonyl; R³ represents a C1-4 alkyl or a hydrogen atom; R⁴ represents a hydrogen atom, a C1-4 alkyl, a C5-6 monocyclic carbocycle which may be substituted with 1 to 3 R⁷ groups or a 5- to 6-membered monocyclic heterocycle which may be substituted with 1 to 3 R⁸ groups; R⁷ represents a halogen or a C1-4 alkyl; R⁸ represents a halogen or a C1-4 alkyl; when the C5-6 monocyclic carbocycle is substituted with 2 or more R⁷ groups, a plurality of R⁷ may be the same or different; when the 5- to 6-membered monocyclic heterocycle is substituted with 2 or more R⁸ groups, a plurality of R⁸ may be the same or different; R⁵ represents a C1-4 alkyl or a hydrogen atom; ring1 represents a C5-6 monocyclic carbocycle or a 5- to 6-membered monocyclic heterocycle; R⁶ represents a C1-4 alkyl, a C1-4 alkoxy or a halogen; m represents an integer of 0 to 3; n represents an integer of 0 to 3; when m is 2 or more, a plurality of R² may be the same or different; and when n is 2 or more, a plurality of R⁶ may be the same or different, or a salt thereof (excluding rac-(3R,4S)-4-amino-1-[3-(3,5-dimethoxyphenyl)-5-(4,6-dimethyl-1H-benzimidazol-2-yl)-4-pyridinyl]-3-piperidinol, rac-(3R,4S)-4-amino-1-[3-(6-fluoro-1H-benzimidazol-2-yl)-5-(3-fluoro-5-methoxyphenyl)-4-pyridinyl]-3-piperidinol and rac-(3R,4S)-4-amino-1-[3-(6-chloro-1H-benzimidazol-2-yl)-5-(3-fluoro-5-methoxyphenyl)-4-pyridinyl]-3-piperidinol).

2. The compound according to claim 1, wherein the compound represented by the general formula (I) is a compound represented by the general formula (IB): 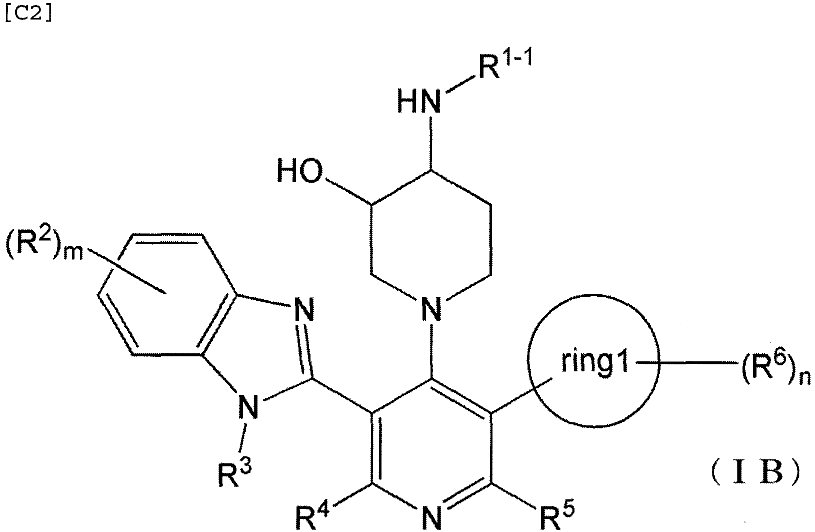 wherein R¹⁻¹ represents 3-oxetanyl, 3,3,3-trifluoropropyl or ethyl; and other symbols have the same meanings as described in claim 1,
or a salt thereof.

3. The compound according to claim 1 or 2, wherein the compound represented by the general formula (IB) is a compound represented by the general formula (IB-1): 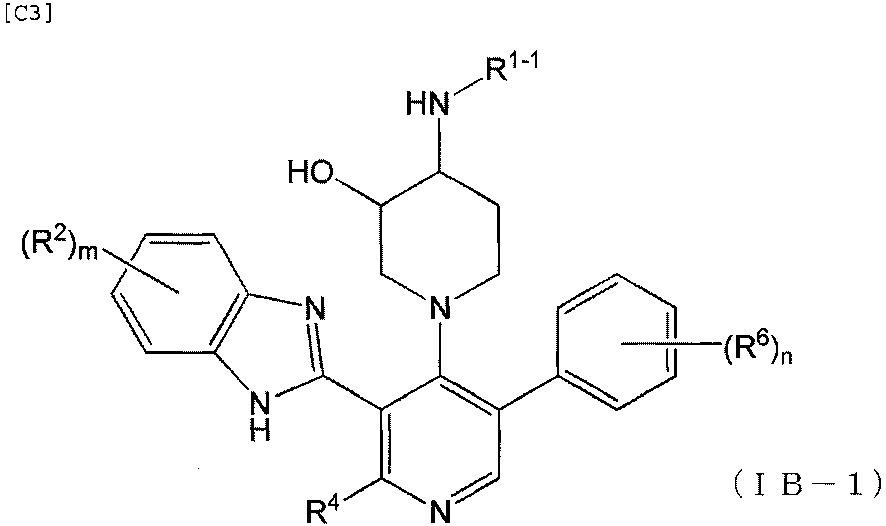 wherein all symbols have the same meanings as described in claim 1 or 2,
or a salt thereof.

4. The compound according to any one of claims 1 to 3, wherein R² is a C1-4 alkoxy, a halogen or a C1-4 alkoxycarbonyl; and R⁶ is a C1-4 alkoxy or a halogen,
or a salt thereof.

5. The compound according to any one of claims 1 to 4, wherein R⁶ is a halogen; and n is 2,
or a salt thereof.

6. The compound according to claim 2, wherein the compound is:
(1) (3S,4R)-1-[3-(5,6-difluoro-1H-benzimidazol-2-yl)-5-(3,5-difluorophenyl)-4-pyridinyl]-4-(3-oxetanylamino)-3-piperidinol;
(2) (3S,4R)-1-[3-(3-chloro-5-fluorophenyl)-5-(5,6-difluoro-1H-benzimidazol-2-yl)-4-pyridinyl]-4-[(3,3,3-trifluoropropyl)amino]-3-piperidinol;
(3) (3S,4R)-1-[3-(5,6-difluoro-1H-benzimidazol-2-yl)-5-(2,5-difluorophenyl)-4-pyridinyl]-4-(ethylamino)-3-piperidinol;
(4) (3S,4R)-1-[3-(5,6-difluoro-1H-benzimidazol-2-yl)-5-(3-fluoro-5-methoxyphenyl)-4-pyridinyl]-4-[(3,3,3-trifluoropropyl)amino]-3-piperidinol; or
(5) (3S,4R)-1-[3-(3,5-difluorophenyl)-5-(6-fluoro-5-methoxy-1H-benzimidazol-2-yl)-4-pyridinyl]-4-[(3,3,3-trifluoropropyl)amino]-3-piperidinol,
or a salt thereof.

7. The compound according to claim 1, wherein R⁴ is a C1-4 alkyl, a C5-6 monocyclic carbocycle which may be substituted with 1 to 3 R⁷ groups or a 5- to 6-membered monocyclic heterocycle which may be substituted with 1 to 3 R⁸ groups, or a salt thereof.

8. The compound according to claim 7, wherein the compound represented by the general formula (I) is a compound represented by the general formula (I-1): 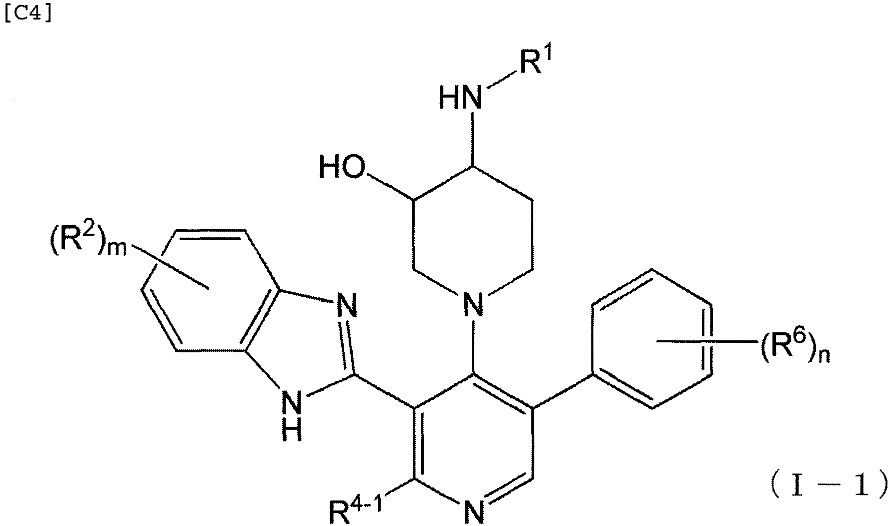 wherein R⁴⁻¹ represents a C1-4 alkyl, a C5-6 monocyclic carbocycle which may be substituted with 1 to 3 R⁷ groups or a 5- to 6-membered monocyclic heterocycle which may be substituted with 1 to 3 R⁸ groups; and other symbols have the same meanings as described in claim 1, or a salt thereof.

9. The compound according to claim 7 or 8, wherein R² is a C1-4 alkoxy, a halogen or a C1-4 alkoxycarbonyl; and R⁶ is a C1-4 alkoxy or a halogen,
or a salt thereof.

10. The compound according to any one of claims 7 to 9, wherein R⁶ is a halogen; and n is 2,
or a salt thereof.

11. The compound according to claim 7, wherein the compound is:
(1) (3S,4R)-4-amino-1-[3-(5,6-difluoro-1H-benzimidazol-2-yl)-5-(3,5-difluorophenyl)-2-(1-methyl-1H-pyrazol-4-yl)-4-pyridinyl]-3-piperidinol;
(2) methyl 2-{4-[(3S,4R)-4-amino-3-hydroxy-1-piperidinyl]-5-(3-chloro-5-fluorophenyl)-2-methyl-3-pyridinyl}-5-fluoro-1H-benzimidazole-7-carboxylate;
(3) methyl 2-{4-[(3S,4R)-4-amino-3-hydroxy-1-piperidinyl]-5-(2,5-difluorophenyl)-2-methyl-3-pyridinyl}-5-fluoro-1H-benzimidazole-7-carboxylate;
(4) (3S,4R)-4-amino-1-[5-(3,5-difluorophenyl)-2-methyl-3-(5,6,7-trifluoro-1H-benzimidazol-2-yl)-4-pyridinyl]-3-piperidinol;
(5) (3S,4R)-1-[5-(3-chloro-5-fluorophenyl)-3-(6-methoxy-1H-benzimidazol-2-yl)-2-methyl-4-pyridinyl]-4-(3-oxetanylamino)-3-piperidinol;
(6) (3S,4R)-1-[5-(3,5-difluorophenyl)-3-(5-fluoro-6-methoxy-1H-benzimidazol-2-yl)-2-methyl-4-pyridinyl]-4-(ethylamino)-3-piperidinol;
(7) (3S,4R)-1-[5-(3-chloro-5-fluorophenyl)-3-(5,6-difluoro-1H-benzimidazol-2-yl)-2-methyl-4-pyridinyl]-4-(3-oxetanylamino)-3-piperidinol;
(8) (3S,4R)-1-[3-(5,6-difluoro-1H-benzimidazol-2-yl)-5-(3,5-difluorophenyl)-2-methyl-4-pyridinyl]-4-[(3,3,3-trifluoropropyl)amino]-3-piperidinol;
(9) (3S,4R)-1-[5-(3,5-difluorophenyl)-2-methyl-3-(5,6,7-trifluoro-1H-benzimidazol-2-yl)-4-pyridinyl]-4-(ethylamino)-3-piperidinol; or
(10) methyl 2-{4-[(3S,4R)-4-amino-3-hydroxy-1-piperidinyl]-5-(3,5-difluorophenyl)-2-(4-morpholinyl)-3-pyridinyl}-5-fluoro-1H-benzimidazole-7-carboxylate,
or a salt thereof.

12. The compound according to claim 1, wherein R⁴ is a hydrogen atom,
or a salt thereof.

13. The compound according to claim 1 or 12, wherein the compound represented by the general formula (I) is a compound represented by the general formula (I-2): 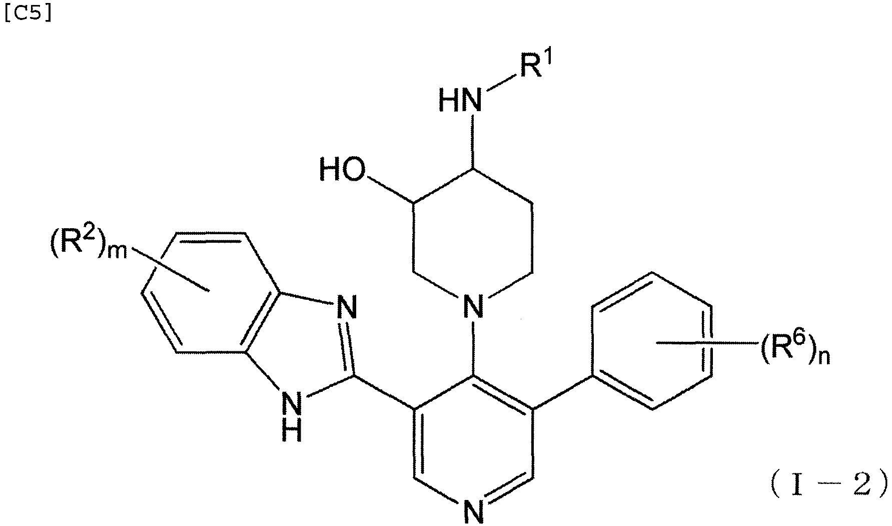 wherein all symbols have the same meanings as described in claim 1,
or a salt thereof.

14. The compound according to claim 12 or 13, wherein R² is a C1-4 alkoxy, a halogen or a C1-4 alkoxycarbonyl; and R⁶ is a C1-4 alkoxy or a halogen,
or a salt thereof.

15. The compound according to any one of claims 12 to 14, wherein R⁶ is a halogen; and n is 2,
or a salt thereof.

16. The compound according to claim 12, wherein the compound is:
(1) (3S,4R)-4-amino-1-[3-(5,6-difluoro-1H-benzimidazol-2-yl)-5-(3,5-difluorophenyl)-4-pyridinyl]-3-piperidinol;
(2) (3S,4R)-4-amino-1-[3-(1H-benzimidazol-2-yl)-5-(3,5-difluorophenyl)-4-pyridinyl]-3-piperidinol;
(3) (3S,4R)-4-amino-1-[3-(2,5-difluorophenyl)-5-(6-methoxy-1H-benzimidazol-2-yl)-4-pyridinyl]-3-piperidinol; or
(4) methyl 2-{4-[(3S,4R)-4-amino-3-hydroxy-1-piperidinyl]-5-(2,5-difluorophenyl)-3-pyridinyl}-5-fluoro-1H-benzimidazole-7-carboxylate,
or a salt thereof.

17. A pharmaceutical composition comprising the compound represented by the general formula (I) according to claim 1 or a salt thereof, and a pharmaceutically acceptable carrier.

18. The pharmaceutical composition according to claim 17, which is a somatostatin receptor agonist.

19. The pharmaceutical composition according to claim 17 or 18, which is a prophylactic and/or therapeutic agent for a somatostatin-related disease.

20. The pharmaceutical composition according to any one of claims 17 to 19, wherein the somatostatin-related disease is acromegaly, or a gastrointestinal symptom accompanying gastrointestinal obstruction.

21. A prophylactic and/or therapeutic agent for a somatostatin-related disease, comprising the compound represented by the general formula (I) according to claim 1 or a salt thereof as an active component, wherein the prophylactic and/or therapeutic agent is administered together with at least one drug selected from the group consisting of pegvisomant, bromocriptine and cabergoline.

22. A prophylactic and/or therapeutic agent for a somatostatin-related disease, comprising the compound represented by the general formula (I) according to claim 1 or a salt thereof as an active component, wherein the prophylactic and/or therapeutic agent is administered together with at least one drug selected from the group consisting of prochlorperazine, levomepromazine, risperidone, metoclopramide, domperidone, diphenhydramine, chlorpheniramine, dimenhydrinate, promethazine, diprophylline, famotidine, cimetidine, scopolamine, tropisetron, granisetron, ondansetron, azasetron, ramosetron, indisetron, palonosetron, cisapride, mosapride, dexamethasone, betamethasone, prednisolone, aprepitant, olanzapine, quetiapine, perospirone, methylnaltrexone and morphine.

23. A method for prophylaxis and/or therapy of a somatostatin-related disease, comprising administering to a mammal an effective amount of the compound represented by the general formula (I) according to claim 1 or a salt thereof.

24. The compound represented by the general formula (I) according to claim 1 or a salt thereof for prophylaxis and/or therapy of a somatostatin-related disease.

25. Use of the compound represented by the general formula (I) according to claim 1 or a salt thereof for the manufacture of a prophylactic and/or therapeutic agent for a somatostatin-related disease.
